# EUROPEAN PATENT APPLICATION

(11) **EP 4 361 166 A1**
(43) Date of publication of application: **01.05.2024**
(21) Application number: 22828523.5
(22) Date of filing: 23.06.2022
(51) Int. Cl.: C07K 5/062, A61K 49/00, C12Q 1/02, C12Q 1/37, G01N 33/68

(54) **FLUORESCENT PROBES**

(30) Priority: 24.06.2021 JP 2021105163
(71) Applicant: Tohoku Techno Arch Co., Ltd., Sendai-shi, Miyagi 980-8579 (JP); Goryo Chemical, Inc., Sapporo-shi, Hokkaido 060-0008 (JP); Senju Pharmaceutical Co., Ltd., Osaka-shi, Osaka 541-0048 (JP)
(72) Inventor: NAKAZAWA, Toru, Sendai-shi, Miyagi 980-8577 (JP); SUZUKI, Yuki, Sapporo-shi, Hokkaido 060-0008 (JP); OKA, Takayuki, Osaka-shi, Osaka 541-0048 (JP); UCHIDA, Takatoshi, Osaka-shi, Osaka 541-0048 (JP)
(74) Representative: Müller-Boré & Partner Patentanwälte PartG mbB
(86) International application number: PCT/JP2022/025205
(87) International publication number: WO 2022/270607

(57) **Abstract**

An object of the present invention is to provide a fluorescent probe that detects calpain enzyme activity with high sensitivity. In the present invention, enzymatic reactivity with a calpain is improved by bonding an amide group having α-carbon, which is bonded to an oxygen atom, to the N-terminal of a peptide chain. There is provided a fluorescent probe that detects calpain activity with higher sensitivity because the probe of the present invention is improved in reactivity with a calpain as compared to a HMRG fluorescent probe that has heretofore been reported. Specifically, the present invention relates to a compound represented by the following general formula (I) or a salt thereof.

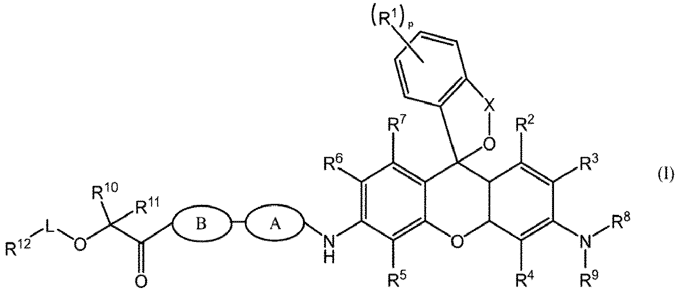

## Description

### Technical Field

The present invention relates to a fluorescent probe that detects activity of a calpain.

### Background Art

A calpain is a cysteine protease activated in a Ca²⁺-dependent manner, and is a modulator molecule that regulates the function and structure of a substrate protein through the limited cleavage of the protein. While the calpain is involved in various life phenomena, it has been reported that the pathogenic variation of its activity or its gene is associated with various diseases, such as a neurodegenerative disease, a heart or muscle disease, an ischemic disease, a cancer, and an eye disease. Further, the calpain is essential to the survival, infection, and the like of the pathogens of some infectious diseases, and it has been known that the calpain is associated with, for example, malaria, trypanosomiasis, or schistosomiasis. Accordingly, it is important to visualize the activity of the calpain in diagnosis of calpain-associated diseases and analysis of the disease mechanisms/drug discovery research.

A fluorescence imaging method serves as an effective method of detecting calpain activity in real time. In particular, a fluorescent probe based on an organic small molecule, such as coumarin (Non Patent Literature 1) or hydroxymethyl rhodamine green (HMRG) (Patent Literature 1), has heretofore been developed as means for detecting calpain activity in a live cell without depending on gene introduction.

In particular, a probe obtained by bonding a peptide to hydroxymethyl rhodamine green (HMRG) serving as a fluorescent dye has been reported as a green fluorescent probe (Patent Literature 1 and Non Patent Literature 2) . The HMRG probe is characterized by being nonfluorescent in the absence of any enzyme and being made fluorescent by a reaction between its fluorescent substrate and an enzyme.

### Citation List

### Patent Literature

[PTL 1] WO 2016/137004 A1

### Non Patent Literature

[NPL 1] THE JOURNAL OF BIOLOGICACLH (1993) 268: 23593-23600
[NPL 2] BIOCONJUGATE CHEMISTRY (2020) 31: 2241-2251

### Summary of Invention

### Technical Problem

The related-art HMRG probe as described in Patent Literature 1 has been susceptible to improvement in terms of fluorescence intensity in the presence of an enzyme. An object of the present invention is to provide a fluorescent probe that detects calpain enzyme activity with high sensitivity.

### Solution to Problem

The inventors of the present invention have found that an improvement in reactivity between a calpain and a fluorescent probe improves the sensitivity with which calpain activity is detected, more specifically, that enzymatic reactivity with the calpain is improved by bonding an amide group having α-carbon, which is bonded to an oxygen atom, to the N-terminal of a peptide chain in the basic skeleton of a calpain probe obtained by bonding the peptide to a fluorescent probe through an amide bonding.

The present invention relates to a compound represented by the following general formula (I) or a salt thereof (hereinafter sometimes referred to as "compound (I)"): 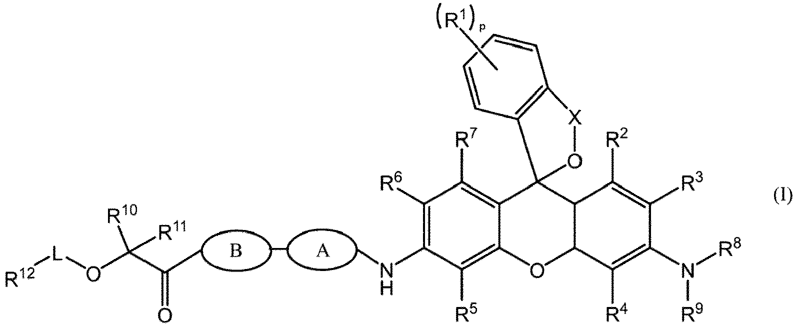 where:
A and B each independently represent an amino acid residue, and the amino acid residues may be identical to or different from each other,
   where a bond between A and NH bonded to A is an amide bond between a C-terminal of A and the NH, A and B are bonded through an amide bond, and a bond between B and a carbonyl group (C=O) bonded to B is an amide bond between an N-terminal of B and the carbonyl group (C=O);
R¹s may be identical to or different from each other, and each represent a substituent bonded to a benzene ring;
"p" represents an integer of from 0 to 4;
R², R³, R⁴, R⁵, R⁶, and R⁷ each independently represent a hydrogen atom, a hydroxyl group (OH group), a halogen atom, or a linear, branched, or cyclic alkyl group that may be substituted;
R⁸ and R⁹ each independently represent a hydrogen atom, or a linear, branched, or cyclic alkyl group that may be substituted, or R⁸ and R⁹ may be bonded to form a ring, or R³ and R⁸ may be bonded to form a ring, and/or R⁴ and R⁹ may be bonded to form a ring;
X represents a linear or branched C1 to C3 alkylene group;
R¹⁰ and R¹¹ each independently represent a hydrogen atom, a hydroxyl group (OH), an alkoxy group, or a linear, branched, or cyclic alkyl group that may be substituted, or R¹⁰ and R¹¹ may be bonded to form a ring;
R¹² represents a hydrogen atom, a linear, branched, or cyclic alkyl group that may be substituted, a linear or branched alkenyl group that may be substituted, a linear or branched alkynyl group that may be substituted, an aryl group that may be substituted, or a heterocyclic group that may be substituted, or R¹² may be bonded to R¹⁰ or R¹¹ to form a ring; and
L represents an amide bond, an ester bond, or -(linear or branched alkylene-O-)ₙ- where "n" represents an integer of from 1 to 10, or L represents a single bond.

In the compound (I), R¹ represents a substituent bonded to a benzene ring. "p" represents an integer of from 0 to 4, and may represent from 0 to 3, from 0 to 2, 0 or 1, 1, or 0. The substituent represented by R¹ may be, for example, a linear or branched alkyl group that may be substituted, a linear or branched alkoxy group that may be substituted, a halogen atom, an amino group that may be substituted, a substituted silyl group, or an acyl group, but is not limited thereto. When "p" represents 2 or more, the plurality of R¹s may be identical to or different from each other.

When R⁸ and R⁹ are bonded to form a ring, when R³ and R⁸ are bonded to form a ring, when R⁴ and R⁹ are bonded to form a ring, or when R¹⁰ and R¹¹ are bonded to form a ring, the ring may contain a heteroatom (e.g., an oxygen atom, a nitrogen atom, or a sulfur atom), and may be, for example, a cycloalkyl group or a cyclic ether. When R¹² and R¹⁰ or R¹¹ are bonded to form a ring, the ring may be a cyclic ether. In addition, those rings may each be from 3-membered to 14-membered, from 3-membered to 10-membered, from 3-membered to 8-membered, or from 3-membered to 6-membered.

Throughout this description, examples of a substituent in the alkyl group or the alkyl moiety of the other group may include a halogen atom, a hydroxyl group, an amino group, an alkylamino group, a dialkylamino group, a thiol group, an alkylthiol group, a sulfonyl group, an alkylsulfonyl group, an alkoxy group, a cyclic ether, a carboxyl group, an alkylcarbonyl group, an alkoxycarbonyl group, an alkoxycarbonylamino group, an alkylcarbonyloxy group, an alkylaminocarbonyl group, an alkylcarbonylamino group, a carbonylamino group, and a hydrazinyl group. When the alkyl group is substituted, the number of the substituents may be set to, for example, from 1 to 6, from 1 to 5, from 1 to 4, from 1 to 3, 1 or 2, 1, 2, or 3.

A substituent in the amino group may be a linear or branched alkyl group that may be substituted. When the amino group is substituted, the number of the substituents may be set to 1 or 2, 1, or 2.

A substituent of the substituted silyl group may be a linear or branched alkyl group that may be substituted, or a phenyl group that may be substituted. The number of the substituents of the silyl group may be set to, for example, from 1 to 4, from 1 to 3, 1 or 2, 1, 2, 3, or 4.

In the general formula (I), the amino acid may be a naturally-occurring amino acid or a derivative thereof as long as the amino acid is cleaved by calpain. The amino acid may be, for example, a stereoisomer of a naturally-occurring amino acid. The amino acid may be, for example, Met, Ser, Ala, Thr, Val, Tyr, Leu, Asn, Ile, Gln, Pro, Asp, Phe, Glu, Trp, Lys, Cys, Arg, Gly, His (these amino acids are represented according to three letter codes for amino acids), norleucine (Nle), or methionine sulfoxide (MetO).

Herein, the term "alkyl group" or "alkyl" moiety in the other group means a linear, branched, or cyclic saturated hydrocarbon group. The group is preferably a saturated hydrocarbon group having 1 to 6 carbon atoms, and examples thereof include a methyl group, an ethyl group, a n-propyl group, an i-propyl group, a n-butyl group, a sec-butyl group, a t-butyl group, an isobutyl group, a pentyl group, an isopentyl group, a 2,3-dimethylpropyl group, a hexyl group, a cyclopropyl group, a cyclobutyl group, a cyclopentyl group, and a cyclohexyl group. The group is more preferably a C1-5 alkyl group, and examples thereof include a methyl group, an ethyl group, a n-propyl group, an i-propyl group, a n-butyl group, a sec-butyl group, a t-butyl group, an isobutyl group, a pentyl group, an isopentyl group, or a 2,3-dimethylpropyl group. The group is still more preferably a C1-3 alkyl group, and examples thereof include a methyl group, an ethyl group, a n-propyl group, and an i-propyl group. The group is most preferably a methyl group or an ethyl group. In addition, the term "alkylene group" refers to a divalent group obtained by removing one hydrogen atom from the above-mentioned alkyl group.

The term "alkoxy group" refers to a group to be bonded to the above-mentioned alkyl group via an oxygen atom ((alkyl group)-O- group), and the alkyl group moiety is as defined in the foregoing. For example, the number of carbon atoms of the alkyl group moiety in the alkoxy group may be from 1 to 6. Examples of the alkoxy group include a methoxy group, an ethoxy group, a 1-propyloxy group, a 2-propyloxy group, a 2-methyl-1-propyloxy group, a 2-methyl-2-propyloxy group, a 2,2-dimethyl-1-propyloxy group, a 1-butyloxy group, a 2-butyloxy group, a 2-methyl-1-butyloxy group, a 3-methyl-1-butyloxy group, a 2-methyl-2-butyloxy group, a 3-methyl-2-butyloxy group, a 1-pentyloxy group, a 2-pentyloxy group, a 3-pentyloxy group, a 2-methyl-1-pentyloxy group, a 3-methyl-1-pentyloxy group, a 2-methyl-2-pentyloxy group, a 3-methyl-2-pentyloxy group, a 1-hexyloxy group, a 2-hexyloxy group, and a 3-hexyloxy group. The C1-6 alkoxy group is preferably a C1-5 alkoxy group, more preferably a methoxy group, an ethoxy group, a n-propyloxy group, an i-propyloxy group, a n-butyloxy group, a sec-butyloxy group, a t-butyloxy group, an isobutyloxy group, a pentyloxy group, an isopentyloxy group, and a 2,3-dimethylpropyloxy group.

The term "alkenyl group" refers to a monovalent group formed by removing one hydrogen atom from any appropriate carbon atom in a linear, branched, or cyclic unsaturated hydrocarbon having one or more carbon-carbon double bonds, and may have, for example, 2 to 10, 2 to 6, or 2 to 4 carbon atoms. Examples of the C2-10 alkenyl group include a vinyl group, a propenyl group, an isopropenyl group, a 1-butenyl group, a 2-butenyl group, a 3-butenyl group, a 1-pentenyl group, a 1-pentenyl group, a 3-pentenyl group, a 4-pentenyl group, a 1-methyl-1-butenyl group, a 1-methyl-2-butenyl group, a 1-methyl-3-butenyl group, a 1-methylidenebutyl group, a 2-methyl-1-butenyl group, a 2-methyl-2-butenyl group, a 2-methyl-3-butenyl group, a 2-methylidenebutyl group, a 3-methyl-1-butenyl group, a 3-methyl-2-butenyl group, a 3-methyl-3-butenyl group, a 1-ethyl-1-propenyl group, a 1-ethyl-2-propenyl group, a 1-hexenyl group, a 2-hexenyl group, a 3-hexenyl group, a 4-hexenyl group, a 5-hexenyl group, a 1-methyl-1-pentenyl group, a 1-methyl-2-pentenyl group, a 1-methyl-3-pentenyl group, a 1-methyl-4-pentenyl group, a 1-methylidenepentyl group, a 2-methyl-1-pentenyl group, a 2-methyl-2-pentenyl group, a 2-methyl-3-pentenyl group, a 2-methyl-4-pentenyl group, a 2-methylidenepentyl group, a 3-methyl-1-pentenyl group, a 3-methyl-2-pentenyl group, a 3-methyl-3-pentenyl group, a 3-methyl-4-pentenyl group, a 3-methylidenepentyl group, a 4-methyl-1-pentenyl group, a 4-methyl-2-pentenyl group, a 4-methyl-3-pentenyl group, a 4-methyl-4-pentenyl group, a 1-heptenyl group, a 2-heptenyl group, a 3-heptenyl group, a 4-heptenyl group, a 5-heptenyl group, a 6-heptenyl group, an octenyl group, a nonenyl group, and a decenyl group.

The term "alkynyl group" refers to a monovalent group formed by removing one hydrogen atom from any appropriate carbon atom in a linear or branched unsaturated hydrocarbon having one or more carbon-carbon triple bonds, and may have, for example, 2 to 6 or 2 to 4 carbon atoms. Examples of the alkynyl group may include an ethynyl group, a 1-propynyl group, a 2-propynyl group, a 1-butynyl group, a 2-butynyl group, a 3-butynyl group, a pentynyl group, a hexynyl group, and a phenylethynyl group.

The term "acyl group" is identical in meaning to an alkanoyl group, and means a group represented by R-C(=O)-. An R moiety in the acyl group represented by the structure is, for example, a hydrogen atom, a linear or branched alkyl group that may be substituted, or an aryl group that may be substituted. The acyl group may have, for example, 2 to 7 carbon atoms, and encompasses an acetyl group, a propionyl group, a butyryl group, an isobutyryl group, a valeryl group, an isovaleryl group, a pivaloyl group, a valeryl group, an isovaleryl group, a hexanoyl group, and a heptanoyl group.

The term "aryl group" refers to a monovalent aromatic hydrocarbon group. The group may have, for example, 6 to 10 carbon atoms, and encompasses a phenyl group and a naphthyl group.

The term "heterocyclic group" means a monovalent group containing at least one of one or more kinds of heteroatoms selected from a nitrogen atom, an oxygen atom, and a sulfur atom, and the group is preferably from 5-membered to 14-membered. The heterocyclic group may be a monocyclic heterocyclic group or a fused heterocyclic group. The number of the heteroatoms incorporated into the 5-membered to 14-membered heterocyclic group may be, for example, from 1 to 5, from 1 to 4, from 1 to 3, 1 or 2, 2, or 1. For example, the following various combinations exist: a heterocyclic group containing 1 nitrogen atom; a heterocyclic group containing 2 nitrogen atoms; a heterocyclic group containing 3 nitrogen atoms; a heterocyclic group containing 1 oxygen atom; a heterocyclic group containing 2 oxygen atoms; a heterocyclic group containing 1 oxygen atom and 1 nitrogen atom; and a heterocyclic group containing 1 sulfur atom. The 5-membered to 14-membered heterocyclic group may be aromatic or nonaromatic. The monocyclic heterocyclic group is preferably a 5-membered or 6-membered ring. The fused heterocyclic group is preferably an 8-membered to 10-membered ring. Examples of the 5-membered to 14-membered heterocyclic group may include piperidyl, piperazyl, morpholyl, quinuclidyl, pyrrolidyl, azetidyl, oxetyl, azetidin-2-one-yl, aziridinyl, tropanyl, furyl, tetrahydrofuryl, thienyl, pyrrolyl, pyrrolinyl, pyrrolidinyl, dioxolanyl, oxazolyl, oxazolinyl, isoxazolyl, thiazolyl, thiazolinyl, isothiazolyl, imidazolyl, imidazolinyl, imidazolidinyl, oxazolidinyl, thiazolidinyl, pyrazolyl, pyrazolinyl, pyrazolidinyl, oxadiazolyl, furazanyl, thiadiazolyl, 1,2,3-triazolyl, 1,2,4-triazolyl, tetrazolyl, pyranyl, pyridyl, piperidinyl, pyridazinyl, pyrimidinyl, pyrazinyl, piperazinyl, dioxanyl, oxazinyl, morpholinyl, thiazinyl, triazinyl, benzofuranyl, isobenzofuranyl, dihydrobenzofuranyl, dihydroisobenzofuranyl, benzothienyl, isobenzothienyl, dihydrobenzothienyl, dihydroisobenzothienyl, tetrahydrobenzothienyl, quinolyl, isoquinolyl, quinazolinyl, phthalazinyl, pteridinyl, coumaryl, chromonyl, 1,4-benzodiazepinyl, indolyl, isoindolyl, benzimidazolyl, benzofuryl, purinyl, acridinyl, phenoxazinyl, phenothiazinyl, benzoxazolyl, benzothiazolyl, indazolyl, benzimidazolyl, benzodioxolanyl, benzodioxanylchromenyl, chromanyl, isochromanyl, chromanonyl, cinnolinyl, quinoxalinyl, indolizinyl, quinolidinyl, imidazopyridyl, naphthyridinyl, dihydrobenzoxazinyl, dihydrobenzoxazolynonyl, dihydrobenzoxazinonyl, and benzothioxanyl.

Herein, the term "halogen atom" means a fluorine atom, a chlorine atom, a bromine atom, and an iodine atom, and the halogen atom is preferably a fluorine atom, a chlorine atom, and a bromine atom, more preferably a fluorine atom or a chlorine atom. Herein, the term "carboxyl group" refers to a group represented by -C(=O)-OH.

The amino acid A is preferably Met, Thr, His, Tyr, Phe, Ser, Gln, Arg, Lys, MetO, and Nle. The amino acid B is preferably a hydrophobic amino acid, for example, Leu or Val, more preferably Leu. Preferred examples of the combination of (amino acid B)-(amino acid A) may include Leu-Met, Leu-Nle, Leu-Phe, Leu-Tyr, Leu-Thr, Leu-Arg, Leu-Lys, Leu-His, Leu-MetO, Val-Tyr, Val-Met, and Leu-Ser.

R¹ preferably represents a linear or branched alkyl group. In addition, "p" represents preferably 0 or 1, more preferably 0.

R² preferably represents a hydrogen atom.

R³ preferably represents a hydrogen atom.

R⁴ preferably represents a hydrogen atom.

R⁵ preferably represents a hydrogen atom.

R⁶ preferably represents a hydrogen atom.

R⁷ preferably represents a hydrogen atom.

R⁸ preferably represents a hydrogen atom.

R⁹ preferably represents a hydrogen atom.

R¹⁰ preferably represents a hydrogen atom, an alkyl group, or an alkoxy group, more preferably a hydrogen atom, a methyl group, or a methoxy group.

R¹¹ preferably represents a hydrogen atom, an alkyl group, or an alkoxy group, more preferably a hydrogen atom, a methyl group, or a methoxy group.

Alternatively, R¹⁰ and R¹¹ are bonded to each other to form preferably a cycloalkyl group, more preferably a C3-C5 cycloalkyl group.

R¹² represents preferably a hydrogen atom, or a linear, branched, or cyclic alkyl group that may be substituted, more preferably a hydrogen atom, or a linear, branched, or cyclic alkyl group having 1 to 6 carbon atoms that may be substituted with one or more groups selected from a hydroxyl group, a carboxyl group, a cycloether, an alkoxy group (e.g., a methoxy group or an ethoxy group), a sulfonyl group, a halogen atom, an amino group, a methylamino group, and a dimethylamino group, still more preferably a hydrogen atom, or a linear or branched alkyl group having 1 to 4 carbon atoms (e.g., a methyl group or an ethyl group, a propyl group, an isopropyl group, or a t-butyl group) that may be substituted with one or more groups selected from a hydroxyl group, a carboxyl group, a cycloether, an alkoxy group (e.g., a methoxy group, an ethoxy group), a sulfonyl group, a halogen atom, an amino group, a methylamino group, and a dimethylamino group. Alternatively, R¹² is preferably bonded to R¹⁰ or R¹¹ to form a 3-membered to 8-membered heterocyclic group having 1 or 2 oxygen atoms (e.g., dioxolanyl or dioxanyl).

L preferably represents -(linear or branched alkylene-O-)ₙ- (where "n" represents an integer of from 1 to 10), or a single bond. The linear or branched alkylene group may be, for example, an alkylene group having 1 to 4 carbon atoms, preferably 2 or 3 carbon atoms, more preferably 2 carbon atoms.

For example, in the formula (I), a group represented by -O-L-R¹² may be a group represented by the following formula (II) : 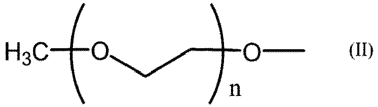 where "n" represents an integer of from 0 to 10.

In addition, for example, in the formula (I), a group represented by -O-L-R¹² may be a hydroxyl group or a group selected from the following.

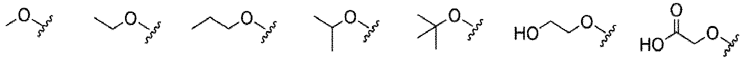

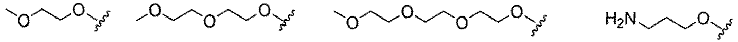

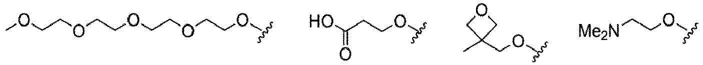

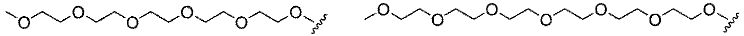

In addition, for example, in the formula (I), a partial structure represented by 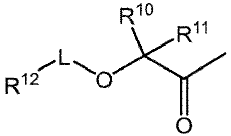 may be a structure represented by the following formula: 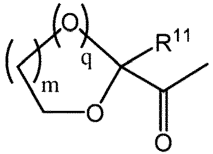 where "m" represents an integer of from 1 to 3, and "q" represents 0 or 1.

The compound of the present invention may be preferably, for example, a compound represented by the formula (III). In addition, a compound obtained by appropriately changing the combination of amino acids in the formula (III) may also be given as a preferred example of the compound of the present invention.

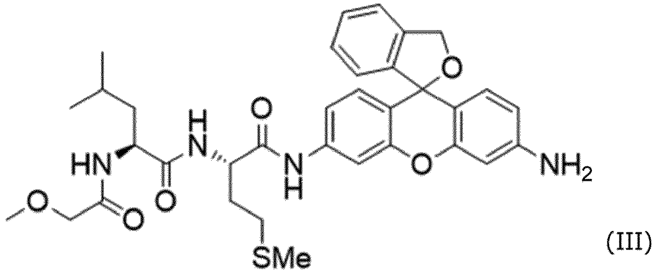

Examples of the salt of the compound (I) may include a base addition salt, an acid addition salt, and an amino acid salt. Examples of the base addition salt may include: metal salts, such as a lithium salt, a sodium salt, a potassium salt, a calcium salt, and a magnesium salt; organic amine salts, such as an ammonium salt, a methylamine salt, a dimethylamine salt, a dicyclohexylamine salt, a tris(hydroxymethyl)aminomethane salt, an N,N-bis(hydroxyethyl)piperazine salt, a 2-amino-2-methyl-1-propanol salt, an ehanolamine salt, an N-methylglucamine salt, an L-glucamine salt, a triethylamine salt, a piperidine salt, and a morpholine salt; and salts with basic amino acids, such as lysine, δ-hydroxylysine, and arginine. Examples of the acid addition salt may include: mineral acid salts, such as a hydrochloric acid salt, a hydrobromic acid salt, a sulfuric acid salt, a nitric acid salt, and a phosphoric acid salt; and organic acid salts, such as a methanesulfonic acid salt, a benzenesulfonic acid salt, a para-toluenesulfonic acid salt, a citric acid salt, an oxalic acid salt, an acetic acid salt, a propionic acid salt, a tartaric acid salt, a fumaric acid salt, a maleic acid salt, a malic acid salt, a succinic acid salt, a benzoic acid salt, a mandelic acid salt, a cinnamic acid salt, a lactic acid salt, a glycolic acid salt, a glucuronic acid salt, an ascorbic acid salt, a nicotinic acid salt, and a salicylic acid salt. The amino acid salt may be, for example, a glycine salt. Of course, the salt of the compound of the present invention is not limited thereto.

The compound (I) may have one or two or more asymmetric carbon atoms in accordance with the kind of a substituent thereof, and hence a stereoisomer, such as an optical isomer or a diastereoisomer, may exist. A stereoisomer in a pure form, any appropriate mixture of stereoisomers, a racemic form, and the like are each included in the scope of the present invention. In addition, although the compound represented by the general formula (I) or the salt thereof may exist as a hydrate or a solvate, these substances are each included in the scope of the present invention. Although the kind of a solvent that forms the solvate is not particularly limited, examples thereof may include solvents, such as ethanol, acetone, and isopropanol. The reference "compound (I)" as used herein encompasses any appropriate mixture of the isomers of the compound (I) or a specific stereoisomer thereof, a pharmacologically acceptable salt, hydrate, and solvate of the compound (I), and a hydrate or solvate of a pharmacologically acceptable salt of the compound (I) even when no such compound is clearly described except for the case where such compound is obviously unsuitable.

In another aspect, the present invention relates to a fluorescent probe including the compound (I). The fluorescent probe of the present invention is a probe that shows a fluorescence response when brought into contact with an enzyme. It is preferred that the fluorescent probe of the present invention emit no fluorescence under a state in which the probe does not react with the enzyme, and the probe emit fluorescence when caused to react with the enzyme. Such enzyme includes a calpain. That is, the fluorescent probe can be used for detecting calpain activity because the probe shows a fluorescence response in the presence of the calpain. In addition, the present invention encompasses a calpain activity-measuring agent including the compound (I) or the salt thereof as an active ingredient.

The calpain is a gene product having a sequence region significantly homologous to the catalytic domain of human calpain-1 (µ-calpain, µCANP, µ80K, calpain I, or CDP I), and includes calpains-1 to 16. For example, calpain-1 is a heterodimer of CAPN1 and CAPNS1, and calpain-2 is a heterodimer of CAPN2 and CAPNS1. The compound (I) can be utilized as a diagnostic agent for a calpain activity-associated disease because it has been known that the calpain is involved in various diseases. Accordingly, the present invention relates to a diagnostic composition for a calpain activity-associated disease, the composition including the compound (I).

The calpain activity-associated disease may be such a disease that the expression of a calpain or the activity thereof contributes to its onset or exacerbation. Examples of the calpain activity-associated disease may include: an eye disease, such as glaucoma including normal-tension glaucoma, autosomal dominant neovascular inflammatory vitreoretinopathy, retinitis pigmentosa, age-related macular degeneration, retinal neuropathy or a retinal vascular occlusive disease associated with diabetes, a retinal disease such as retinal ischemia or retinal neuropathy, or cataract; a muscle disease such as muscular dystrophy; diabetes; an inflammatory disease or an autoimmune disease, such as acute esophagitis, multiple sclerosis, an idiopathic inflammatory muscle disease, or autoimmune encephalitis; a neurological disease, such as spinal cord injury, Parkinson's disease, a neurodegenerative disease, lissencephaly, Alzheimer's disease, or spinocerebellar degeneration; a heart or blood vessel disease, such as cardiac ischemia-reperfusion injury, a chronic heart disease, an abdominal aortic aneurysm, or atherosclerosis; a cancer, such as a melanoma, breast cancer, colon cancer, kidney cancer, stomach cancer, cervical cancer, ovarian cancer, or soft tissue sarcoma; a brain tumor; an aging syndrome; progeria; an infectious disease caused by a parasite or a pathogenic microorganism, such as a malaria infectious disease, trypanosomiasis, African sleeping sickness, schistosomiasis, leishmaniasis, or meniscocytosis; traumatic encephalopathy; Machado-Joseph disease; preeclampsia; and pulmonary fibrosis.

The compound (I) of the present invention may be used in combination with a calpain inhibitor. Specifically, the compound (I) may be used for determining the indication and therapeutic effect of the calpain inhibitor. Accordingly, the present invention relates to a method of treating a calpain activity-associated disease, the method including: identifying or selecting a treatment subject having sensitivity to a calpain inhibitor through use of a fluorescence response caused by an interaction between a calpain in a treatment subject candidate or a sample derived from the candidate and the compound (I) as an indicator; and administering a pharmaceutical composition including the calpain inhibitor as an active ingredient to the treatment subject having sensitivity. The present invention also relates to a method of determining the therapeutic effect of a pharmaceutical composition including a calpain inhibitor as an active ingredient, the method including: administering the pharmaceutical composition to a treatment subject; and determining the therapeutic effect of the pharmaceutical composition through use of a fluorescence response caused by an interaction between a calpain in the treatment subject or a sample derived from the treatment subject and the compound (I) as an indicator. The present invention also relates to a pharmaceutical composition for treating a calpain activity-associated disease, the pharmaceutical composition including a calpain inhibitor as an active ingredient, the pharmaceutical composition being administered to a treatment subject determined to have sensitivity to the calpain inhibitor through use of a fluorescence response caused by an interaction between a calpain in a treatment subject candidate or a sample derived from the candidate and the compound (I) as an indicator. The present invention also relates to a use of a calpain inhibitor in the production of the pharmaceutical composition. The fluorescence response caused by the interaction between the calpain in, for example, the treatment subject candidate or the sample derived from the candidate and the compound (I) may be visualized or turned into an image by using a fluorescence imaging approach. For example, the concentration or amount of the calpain, or calpain activity may be obtained on the basis of the intensity of fluorescence from the compound (I) measured in, for example, the treatment subject candidate or the sample derived from the candidate. The treatment subject having sensitivity to the calpain inhibitor may be identified or selected by using, for example, a method described in the section D to be described later. In addition, the therapeutic effect of the pharmaceutical composition may be determined by using, for example, a method described in the section C to be described later. The target disease of the above-mentioned pharmaceutical composition may be the above-mentioned calpain activity-associated disease, and may be, for example, a glaucoma disease. The present invention also relates to a method of determining the preventive effect of a pharmaceutical composition including a calpain inhibitor as an active ingredient, the method including: administering the pharmaceutical composition to a prevention subject in the prevention of a calpain activity-associated disease; and determining the preventive effect of the pharmaceutical composition through use of a fluorescence response caused by an interaction between a calpain in the prevention subject or a sample derived from the prevention subject and the compound (I) as an indicator. The prevention subject may be identified or selected in the same manner as in the identification or selection of the treatment subject. The treatment subject or the prevention subject is typically a mammal subject.

In one aspect, the fluorescent probe of the present invention (more specifically, the compound (I)) may be used in combination with a pharmaceutical composition for treating or preventing a calpain activity-associated disease, the pharmaceutical composition including a calpain inhibitor as an active ingredient, for determining the degree of the effect of the pharmaceutical composition in the treatment or prevention of the disease with the pharmaceutical composition. In another aspect, the fluorescent probe of the present invention (more specifically, the compound (I)) may be used as a diagnostic agent (so-called companion diagnostic agent) for examining in advance whether or not a treatment subject candidate or a prevention subject candidate corresponds to a subject to be treated or prevented with a pharmaceutical composition for treating or preventing a calpain activity-associated disease, the pharmaceutical composition including a calpain inhibitor as an active ingredient.

One aspect of the present invention may be a cancer diagnostic agent or a pharmaceutical composition for identifying a cancer region to be used in cancer surgical treatment or a cancer test, the cancer diagnostic agent or the pharmaceutical composition including the compound (I) as an active ingredient, and the cancer surgical treatment or the cancer test may be, for example, wound-opening surgery, such as craniotomy, thoracotomy, or laparotomy, scopic surgery, or endoscopy. The cancer diagnostic agent or pharmaceutical composition for identifying a cancer region of the present invention may be used for intraoperative rapid diagnosis.

In one aspect, the fluorescent probe of the present invention (more specifically, the compound (I)) does not emit fluorescence under a state in which the probe does not react with a cathepsin, and the probe emits fluorescence when caused to react with the cathepsin. That is, the fluorescent probe can be used for detecting cathepsin activity because the probe shows a fluorescence response in the presence of the cathepsin.

The term "cathepsin" is a collective term for acid proteases localized in lysosome, and includes cathepsins B, C, F, H, K, L1, L2, O, S, W, and X/Z each having cysteine at its active site, cathepsins A and G each having serine at its active site, and cathepsins D and E each having aspartic acid as its active site. The fluorescent probe of the present invention (more specifically, the compound (I)) that shows a fluorescence response in the presence of the cathepsin can be used in the detection of pancreatic juice because the pancreatic juice contains the cathepsin. Accordingly, one aspect of the present invention may be a kit for pancreatic juice detection including the compound (I). The kit may include a sample-collecting tool or a reagent such as a sample treatment liquid, and may further include, for example, a tool required for detection.

The present invention also provides the following methods.
[A] A method of measuring calpain activity in a sample, the method including: bringing the compound (I) and the sample into contact with each other; and measuring fluorescence from the compound (I) after the contact.
[B1] A method of providing information for the diagnosis of a calpain activity-associated disease in a mammal subject, the method including: bringing the compound (I) and a sample derived from the mammal subject into contact with each other; and measuring fluorescence from the compound (I) after the contact.
[B2] A method of diagnosing a calpain activity-associated disease in a mammal subject, the method including: administering the compound (I) to the mammal subject; measuring fluorescence from the compound (I) after the administration; and determining a possibility that the mammal subject suffers from the calpain activity-associated disease on the basis of the measurement result.
[C] A test method for the determination of a therapeutic effect of a candidate compound on a calpain activity-associated disease, the method including: preparing a sample from a calpain activity-associated disease model to which the candidate compound is not administered and a sample from the calpain activity-associated disease model to which the candidate compound is administered; bringing the compound (I) and each of the samples into contact with each other; and measuring fluorescence from the compound (I) after the contact.
[D1] A method of providing information for the monitoring of a calpain activity-associated disease in a mammal subject, the method including: bringing the compound (I) and a sample derived from the mammal subject into contact with each other; and measuring fluorescence from the compound (I) after the contact.
[D2] A method of monitoring a calpain activity-associated disease in a mammal subject, the method including: administering the compound (I) to the mammal subject; measuring fluorescence from the compound (I) after the administration; and determining the course of the calpain activity-associated disease on the basis of the measurement result.

The present invention also provides a pharmaceutical composition for treating or preventing a calpain activity-associated disease, including a calpain inhibitor as an active ingredient, wherein the pharmaceutical composition is administered to a treatment or prevention subject determined to have sensitivity to the calpain inhibitor through use of a fluorescence response caused by an interaction between a calpain in a treatment or prevention subject candidate, or a sample derived from the candidate and the compound (I) as an indicator.

### Advantageous Effects of Invention

The probe provided by the present invention can detect calpain activity with higher sensitivity because the probe is improved in reactivity with a calpain as compared to the related-art HMRG probe.

### Brief Description of Drawings

FIG. **1(a)** is the absorption spectrum of Compound 19 at each pH, and FIG. **1(b)** is the fluorescence spectrum of Compound 19 at each pH.
FIG. **2(a)** shows a change in absorption spectrum of Compound 19 by the addition of calpain-1 thereto, and FIG. **2(b)** shows a change in fluorescence spectrum of Compound 19 by the addition of calpain-1 thereto.
FIG. **3** shows a change in HPLC chromatogram of Compound 19 by the addition of calpain-1 thereto.
FIG. **4** is a graph for showing a change in fluorescence intensity of each of Compound 16 and Compound 19 with time by the addition of calpain-1 thereto.
FIG. **5(a)** is a graph for showing a change in fluorescence intensity of each of Compound 16 and Compounds 19 to 25 10 minutes after the addition of calpain-1 thereto, FIG. **5(b)** is a graph for showing a change in fluorescence intensity of each of Compounds 16 to 18 and Compounds 26 to 29 10 minutes after the addition of calpain-1 thereto, and FIG. **5(c)** is a graph for showing a change in fluorescence intensity of each of Compound 16 and Compounds 30 to 36 10 minutes after the addition of calpain-1 thereto.
FIG. **6(a)** is a graph for showing a change in fluorescence intensity of Compound 37 with time by the addition of calpain-1 thereto, FIG. **6(b)** is a graph for showing a change in fluorescence intensity of Compound 42 with time by the addition of calpain-1 thereto, and FIG. **6(c)** is a graph for showing a change in fluorescence intensity of Compound 45 with time by the addition of calpain-1 thereto.
FIG. **7(a)** is a graph for showing a change in fluorescence intensity of Compound 48 with time by the addition of calpain-1 thereto, FIG. **7(b)** is a graph for showing a change in fluorescence intensity of Compound 51 with time by the addition of calpain-1 thereto, and FIG. **7(c)** is a graph for showing a change in fluorescence intensity of Compound 53 with time by the addition of calpain-1 thereto.
FIG. **8(a)** is a graph for showing a change in fluorescence intensity of Compound 55 with time by the addition of calpain-1 thereto, FIG. **8(b)** is a graph for showing a change in fluorescence intensity of Compound 57 with time by the addition of calpain-1 thereto, and FIG. **8(c)** is a graph for showing a change in fluorescence intensity of Compound 59 with time by the addition of calpain-1 thereto.
FIG. **9(a)** is a graph for showing a change in fluorescence intensity of each of Compounds 37 to 41 10 minutes after the addition of calpain-1 thereto, FIG. **9(b)** is a graph for showing a change in fluorescence intensity of each of Compounds 42 to 44 10 minutes after the addition of calpain-1 thereto, and FIG. **9(c)** is a graph for showing a change in fluorescence intensity of each of Compounds 45 to 47 10 minutes after the addition of calpain-1 thereto.
FIG. **10(a)** is a graph for showing a change in fluorescence intensity of each of Compounds 48 to 50 10 minutes after the addition of calpain-1 thereto, FIG. **10(b)** is a graph for showing a change in fluorescence intensity of each of Compound 51 and Compound 52 10 minutes after the addition of calpain-1 thereto, and FIG. **10(c)** is a graph for showing a change in fluorescence intensity of each of Compound 53 and Compound 54 10 minutes after the addition of calpain-1 thereto.
FIG. **11(a)** is a graph for showing a change in fluorescence intensity of each of Compound 55 and Compound 56 10 minutes after the addition of calpain-1 thereto, FIG. **11(b)** is a graph for showing a change in fluorescence intensity of each of Compound 57 and Compound 58 10 minutes after the addition of calpain-1 thereto, and FIG. **11(c)** is a graph for showing a change in fluorescence intensity of each of Compounds 59 to 66 10 minutes after the addition of calpain-1 thereto.
FIG. **12(a)** is an image obtained by live cell imaging with Compound 16 and FIG. **12(b)** is an image obtained by live cell imaging with Compound 19.
FIG. **13(a)** is an image obtained by live cell imaging with Compound 20, FIG. **13(b)** is an image obtained by live cell imaging with Compound 21, and FIG. **13(c)** is an image obtained by live cell imaging with Compound 22.
FIG. **14(a)** is an image obtained by live cell imaging with Compound 23, FIG. **14(b)** is an image obtained by live cell imaging with Compound 38, and FIG. **14(c)** is an image obtained by live cell imaging with Compound 56.
FIGS. **15** are each a photographed image (rat) of an ocular fundus having administered thereto Compound 19.
FIG. **16** is a graph for showing the number of HMRG-positive cells (rat) in the ocular fundus having administered thereto Compound 19.
FIGS. **17** are each a photographed image of an ocular fundus (rabbit) having administered thereto Compound 19 in an NMDA-administered group.
FIGS. **18** are each a photographed image of an ocular fundus (rabbit) having administered thereto Compound 19 in a PBS-administered group.
FIGS. **19** are each a photographed image of an ocular fundus (rabbit) having administered thereto Compound 16 (19 µL) in the NMDA-administered group.
FIGS. **20** are each a photographed image of an ocular fundus (rabbit) having administered thereto Compound 16 (38 µL) in the NMDA-administered group.
FIGS. **21** are each a photographed image of an ocular fundus (rabbit) having administered thereto Compound 16 (19 µL) in the PBS-administered group.
FIGS. **22** are each a photographed image of an ocular fundus (rabbit) having administered thereto Compound 16 (38 µL) in the PBS-administered group.
FIG. **23** is a graph for showing the number of HMRG-positive cells (rabbit) in each of the ocular fundi having administered thereto Compound 16 or Compound 19.
FIG. **24** is an image obtained by the fluorescence imaging of retinal ganglion cells with Compound 19.
FIG. **25** is a fluorescence photographed image (glutamic acid final concentration: 0 µM) of an iPS cell-derived three-dimensional stereoscopic retinal tissue with Compound 19.
FIG. **26** is a fluorescence photographed image (glutamic acid final concentration: 300 µM) of the iPS cell-derived three-dimensional stereoscopic retinal tissue with Compound 19.
FIG. **27** is a fluorescence photographed image (glutamic acid final concentration: 1 mM) of the iPS cell-derived three-dimensional stereoscopic retinal tissue with Compound 19.
FIG. **28** is a graph for showing the fluorescence intensity of the iPS cell-derived three-dimensional stereoscopic retinal tissue with Compound 19.
FIG. **29** is a graph for showing the result of comparison between the fluorescence intensities of Compound 19 and Compound 16.
FIGS. **30** are images obtained by live cell imaging with Compound 19 in the presence or absence of a calpain inhibitor.
FIG. **31** is a photographed image of an ocular fundus (rat) having administered thereto Compound 19 in a calpain inhibitor-administered group or a control group.
FIG. **32** is a graph for showing the number of HMRG-positive cells in the photographed images of the ocular fundi (rats) each having administered thereto Compound 19 in the calpain inhibitor-administered group or the control group.
FIG. **33** is a graph for showing the number of HMRG-positive cells in the photographed images of ocular fundi (rats) each having administered thereto Compound 19 in a calpain inhibitor-administered group or a control group.
FIG. **34** is a graph for showing the number of HMRG-positive cells in the photographed images of ocular fundi (rats) each having administered thereto Compound 19 in a calpain inhibitor-administered group or a control group.
FIG. **35** is a graph for showing the number of HMRG-positive cells in the photographed images of ocular fundi each having administered thereto Compound 19 in each calpain inhibitor-administered group.
FIGS. **36** are each a fluorescence photographed image of an iPS cell-derived RGC axon model with Compound 19.
FIG. **37** is a graph for showing a change in fluorescence intensity of Compound 19 with time when cathepsin B is added or not added thereto.

### Description of Embodiments

### (A. Synthesis of Compound (I))

The compound (I) may be synthesized by any appropriate method. For example, the compound (I) may be synthesized in conformity with a method described in Example 2 of the present application. In a synthesis scheme represented in Example 2, in Step "a", an amide bond is formed from a N atom of HMRG and the C-terminal of an amino acid. Subsequently, Compound A1 is carried on a resin in Step "b" by a solid-phase synthesis method, and the deprotection of the protective group of the amino acid in Step "c" and the elongation of the amino acid in Step "d" are sequentially performed. The protective group of the amino acid is deprotected in Step "e", and in Step "f", for example, a carboxy compound R¹²-L-O-C(R¹⁰)(R¹¹)-COOH having a partial structure represented by R¹²-L-O-C(R¹⁰)(R¹¹)in the formula (I), or an acid anhydride or an acid halide derived therefrom is caused to react with the amino acid to bond an amide group having a R¹²-L-O-C(R¹⁰)(R¹¹)- group to the N-terminal of a peptide chain. The compound represented by the formula (I) can be obtained by being cut out of the resin in Step "g". In Step "g", protective groups in the respective substituents R, and the amino acid A and the amino acid B may be simultaneously deprotected as appropriate. A crude product of the compound represented by the formula (I) to be obtained may be further purified. For example, the resultant crude product may be purified by reverse-phase column chromatography.

### (B. Method of measuring or detecting Calpain Activity)

The compound (I) can be used in the measurement or detection of a calpain or calpain activity because the compound reacts with the calpain to show a fluorescence response. Accordingly, in another aspect, the present invention relates to a method of measuring (or detecting) a calpain (or calpain activity) in a sample, the method including: bringing the compound (I) and the sample into contact with each other; and measuring (or detecting) fluorescence from the compound (I) after the contact. A case in which the fluorescence is detected may mean that the calpain or the calpain activity is present in the sample. In addition, the detected fluorescence may represent the calpain concentration of the sample or the degree of the calpain activity thereof. The measurement and detection of the calpain (or the calpain activity) may be performed by using the measured fluorescence intensity as an absolute value. Alternatively, the calpain (or the calpain activity) may be measured based on the measured fluorescence intensity by using the amount of a change in fluorescence intensity with time or the fluorescence intensity of a control for comparison. Such fluorescence intensity of the control may be obtained by subjecting the control to measurement simultaneously with the sample, or a value obtained by using the control in advance may be used. Although the compound (I) and the sample are brought into contact with each other by a method, such as the addition, application, spraying, or injection of a solution or a suspension containing the compound (I), the method may be appropriately selected in accordance with, for example, the form of the sample and a measurement environment. A temperature condition at the time of the contact is not limited as long as the calpain can exhibit its catalytic action. The fluorescence from the compound (I) after the contact may be measured by applying visible light having a wavelength of from about 440 nm to about 510 nm as an excitation wavelength to the sample at any appropriate time point after the contact (e.g., any appropriate time point during a time period from a time point immediately after the contact to a time point after the lapse of several hours therefrom). In addition, the calpain concentration or activity may be calculated by utilizing a standard curve created by subjecting a calpain-containing sample whose concentration or activity has been known in advance to measurement by the same method as that in the measurement of the sample as required. A living organism-derived tissue or liquid, or a treated product thereof; a cell culture, a cell culture supernatant, a cell disrupted product, a cell lysate, or a treated product thereof; an isolated and purified enzyme; or the like may be used as the sample, and the sample may be, for example, a sample derived from a mammal subject to be described later. In addition, the above-mentioned method of measuring or detecting calpain activity may be performed in vivo by applying a living organism as the sample to be subjected to measurement or detection. When the above-mentioned method of measuring or detecting calpain activity is performed in vivo, the compound (I) and the sample are brought into contact with each other by administering the compound (I) to the living organism to be subjected to measurement or detection. Any appropriate route and method may be selected for the administration of the compound (I) to the living organism in accordance with a site to be subjected to the measurement or detection of the calpain activity. Examples of the administration method include an eye drop, a nasal drop, an ear drop, injection, and a drip. The living organism to be subjected to measurement or detection is, for example, a mammal subject to be described later.

Calpain measurement in the following method may be performed in conformity with the above-mentioned measurement method.

### (C. Method of diagnosing Calpain Activity-associated Disease)

As described above, it has been known that a calpain is involved in various diseases. Accordingly, the measurement of calpain activity through utilization of the compound (I) enables the diagnosis of a calpain activity-associated disease, the determination of the pathology thereof, or the monitoring of the pathological condition thereof. Accordingly, the present invention relates to a method of providing information for the diagnosis of a calpain activity-associated disease in a mammal subject, the method including: bringing the compound (I) and a sample derived from the mammal subject into contact with each other; and measuring fluorescence from the compound (I) after the contact. The calpain activity-associated disease in the mammal subject to be subjected to the diagnosis described above can be diagnosed on the basis of the information provided by the method of the present invention. The provided information is, for example, information reflecting a fluorescence response, such as a fluorescence intensity, the number of cells each emitting fluorescence (e.g., the number of cells each emitting fluorescence having an intensity equal to or more than a predetermined value per unit area), or the area of a range emitting fluorescence. The calpain activity-associated disease may be diagnosed by, for example, comparing the above-mentioned provided information to a predetermined reference value. Such reference value is, for example, a measured value in a sample derived from a mammal subject that does not suffer from the calpain activity-associated disease (hereinafter referred to as "negative control") or a measured value in a sample derived from a mammal subject suffering from the calpain activity-associated disease (hereinafter referred to as "positive control"). The above-mentioned reference value may be a value measured simultaneously with the measurement of the sample, may be a value measured in advance, or may be a value statistically calculated from the value measured in advance. In one embodiment, when information reflecting a fluorescence response more than a reference value based on the negative control is provided and/or when information reflecting a fluorescence response equal to or more than a reference value based on the positive control is provided, it can be diagnosed that the mammal subject from which the sample is derived may suffer from the calpain activity-associated disease. In another embodiment, when information reflecting a fluorescence response equal to or less than the reference value based on the negative control is provided and/or when information reflecting a fluorescence response less than the reference value based on the positive control is provided, it can be diagnosed that the mammal subject from which the sample is derived may not suffer from the calpain activity-associated disease. In addition, the present invention relates to a method of providing information for the monitoring of a calpain activity-associated disease in a mammal subject, the method including: bringing the compound (I) and a sample derived from the mammal subject into contact with each other; and measuring fluorescence from the compound (I) after the contact. The course of the calpain activity-associated disease in the above-mentioned mammal subject can be determined on the basis of the information provided by the method of the present invention. The provided information includes the same information reflecting a fluorescence response as that described above. The course of the calpain activity-associated disease may be determined by, for example, comparing the above-mentioned provided information to information obtained from the mammal subject before then. In one embodiment, when information reflecting a fluorescence response less than a fluorescence response obtained before is provided, it can be determined that the calpain activity-associated disease may be cured or alleviated, or may be prevented. In another embodiment, when information reflecting a fluorescence response equal to or more than the fluorescence response obtained before is provided, it can be determined that the calpain activity-associated disease may not be cured or alleviated, or may not be prevented. In still another embodiment, when information reflecting a fluorescence response comparable to the fluorescence response obtained before is provided, it can be determined that the progress of the calpain activity-associated disease may be suppressed, or the onset thereof may be prevented.

In another aspect, the present invention relates to a method of diagnosing a calpain activity-associated disease in a mammal subject, the method including: administering the compound (I) to the mammal subject; measuring fluorescence from the compound (I) after the administration; and determining a possibility that the mammal subject suffers from the calpain activity-associated disease on the basis of the measurement result. As in the foregoing, the above-mentioned measurement result may be, for example, information reflecting a fluorescence response, such as a fluorescence intensity, the number of cells each emitting fluorescence (e.g., the number of cells each emitting fluorescence having an intensity equal to or more than a predetermined value per unit area), or the area of a range emitting fluorescence. The possibility that the above-mentioned mammal subject suffers from the calpain activity-associated disease may be determined by, for example, comparing the above-mentioned measurement result to a predetermined reference value. Such reference value is, for example, a measured value in a negative control or a measured value in a positive control. The above-mentioned reference value may be a value measured simultaneously with the measurement of the sample, or may be a value measured in advance or a value statistically calculated from the value measured in advance. In one embodiment, when the measurement result is more than a reference value based on the negative control and/or when the measurement result is equal to or more than a reference value based on the positive control, it can be determined that the above-mentioned mammal subject may suffer from the calpain activity-associated disease. In another embodiment, when the measurement result is equal to or less than the reference value based on the negative control and/or when the measurement result is less than the reference value based on the positive control, it can be determined that the above-mentioned mammal subject may not suffer from the calpain activity-associated disease. The present invention also relates to a method of monitoring a calpain activity-associated disease in a mammal subject, the method including: administering the compound (I) to the mammal subject; measuring fluorescence from the compound (I) after the administration; and determining the course of the calpain activity-associated disease on the basis of the measurement result. The above-mentioned measurement result may be, for example, the above-mentioned information reflecting a fluorescence response. The course of the calpain activity-associated disease in the above-mentioned mammal subject may be determined by, for example, comparing the above-mentioned measurement result to a measurement result measured in the mammal subject before then. In one embodiment, when a measurement result showing a fluorescence response equal to or more than the previous measurement result is obtained, it can be determined that the calpain activity-associated disease may not be cured or alleviated, or may not be prevented. In another embodiment, when a measurement result showing a fluorescence response less than the previous measurement result is obtained, it can be determined that the calpain activity-associated disease may be cured or alleviated, or may be prevented. In still another embodiment, when a measurement result showing a fluorescence response comparable to the previous measurement result is obtained, it can be determined that the progress of the calpain activity-associated disease may be suppressed, or the onset thereof may be prevented.

The "monitoring" may include determining the therapeutic effect of a candidate therapeutic agent or a treatment method serving as a candidate after the administration of the agent to the above-mentioned mammal subject or after the application of the method to the above-mentioned mammal subject. Accordingly, the following is permitted: the above-mentioned monitoring method includes, before the monitoring step, administering the candidate therapeutic agent to the above-mentioned mammal subject or applying the treatment method serving as a candidate to the above-mentioned mammal subject; when a disease is not cured or alleviated, the therapeutic agent or the treatment method is determined to have no effect; and when the disease is cured or alleviated, or the progress of the disease is suppressed, the therapeutic agent or the treatment method is determined to have an effect.

In, for example, an ophthalmologic region, when the compound (I) is administered to a mammal subject, fluorescence observation can be performed by using fluorescein angiography equipment that has already been put into general use in the ophthalmologic region.

The term "sample derived from a mammal subject" as used herein means a specimen collected from the mammal subject, and the specimen is, for example, a tissue or a body fluid. The term "body fluid" means a liquid derived from a subject, and examples thereof include: blood, such as whole blood, plasma, serum, or a hemolysate of whole blood or a blood cell; saliva; urine; a lacrimal fluid; an exudate; lymph; a serous fluid; a spinal fluid; a cerebrospinal fluid; a synovial fluid; an aqueous humor; a vitreous humor, or a fractionated product or treated product thereof; and diluted liquids or concentrated liquids thereof.

The control sample that does not suffer from the calpain activity-associated disease may be a sample derived from a mammal subject that has been known to be free from suffering from the calpain activity-associated disease, or a sample derived from a mammal subject that is obviously free from suffering from the calpain activity-associated disease. The control sample suffering from the calpain activity-associated disease may be a sample derived from a mammal subject that has been known to suffer from the calpain activity-associated disease, or a sample derived from a mammal subject that obviously suffers from the calpain activity-associated disease.

The sample derived from the above-mentioned mammal subject and the compound (I) may be brought into contact with each other in vivo, in vitro, or ex vivo.

Examples of the "mammal subject" include mammals, such as humans, cattle, horses, dogs, cats, pigs, sheep, rabbits, and rats. Of those, humans are preferred.

In the entirety of the description, the term "determination" or "possibility" means the prediction of the condition of the mammal subject (e.g., a patient) by this, and does not mean that the condition can be determined with an accuracy of 100%.

In the entirety of this description, "fluorescence intensities" to be compared to each other may each be a value converted into a value (e.g., a concentration, an amount, or an activity value) calculated from the fluorescence intensity as required. For example, increases in fluorescence intensity (concentration increases) of samples may be compared to each other by using a calpain concentration calculated through utilization of a standard curve created by subjecting a calpain-containing sample whose concentration has been known in advance to measurement by the same method as that in the measurement of the sample.

### (D. Methods of selecting and treating Mammal Subject treatable with Calpain Inhibitor)

A calpain inhibitor has been known as a therapeutic agent for a calpain activity-associated disease. In particular, when a calpain concentration is high or calpain activity is strong in the disease, the calpain inhibitor may easily exhibit its effect (in other words, a treatment subject may have sensitivity to the calpain inhibitor). Accordingly, the present invention relates to a method of selecting a mammal subject treatable with a calpain inhibitor, the method including: bringing the compound (I) and a sample derived from a test mammal subject into contact with each other; measuring fluorescence from the compound (I) after the contact; and selecting the mammal subject treatable with the calpain inhibitor on the basis of the measurement result. Alternatively, the present invention relates to a method of selecting a mammal subject treatable with a calpain inhibitor, the method including: administering the compound (I) to a test mammal subject; measuring fluorescence from the compound (I) after the administration; and selecting the mammal subject treatable with the calpain inhibitor on the basis of the measurement result. The above-mentioned measurement result may be, for example, the information reflecting a fluorescence response described in the section C. The mammal subject treatable with the calpain inhibitor may be selected by, for example, comparing the above-mentioned measurement result to a predetermined reference value. Such reference value is, for example, a measured value in a negative control or a measured value in a positive control. The above-mentioned reference value may be a value measured simultaneously with the measurement of the sample, or may be a value measured in advance or a value statistically calculated from the value measured in advance. In one embodiment, when the measurement result is more than a reference value based on the negative control and/or when the measurement result is equal to or more than a reference value based on the positive control, the above-mentioned test mammal subject can be selected as the mammal subject treatable with the calpain inhibitor.

A method of treating the mammal subject treatable with the calpain inhibitor described above may further include administering the calpain inhibitor to the mammal subject selected as the mammal subject treatable with the calpain inhibitor.

Examples of the calpain inhibitor may include ALLNal, PD150606, calpeptin, MDL28170, Calpain inhibitor XI (AK295), calpastatin peptide (CAST), SNJ-1945, SJA6017, ABT-957, BDA-410, Macrocyclic aldehyde (CAT811), NH2-GRKKRRQRRRPP QPDALKSRTLR-COOH (Tat-pCL), Dipeptidyl α,β-unsaturated ester, Hypervalent organotellurium compound (RF19), and Sm-p80 (recombinant protein).

### (E. Method of screening Therapeutic Agent for Calpain Activity-associated Disease)

Further, the compound (I) can examine a therapeutic effect targeted at a calpain because the compound can be used in the measurement of whether or not a specific candidate compound or treatment method affects a calpain level or calpain activity. Accordingly, the compound (I) can be used in a method of screening a therapeutic agent for a calpain activity-associated disease. As an example, the present invention relates to a method for the determination of the therapeutic effect of a candidate compound or a candidate treatment method on a calpain activity-associated disease, the method including: administering the candidate compound, or applying the candidate treatment method, to a calpain activity-associated disease model; bringing the compound (I) and a sample derived from the disease model into contact with each other or administering the compound (I) to the disease model; measuring fluorescence from the compound (I) after the contact or the administration; and determining the therapeutic effect of the candidate compound or the candidate treatment method on the calpain activity-associated disease on the basis of the measurement result. The above-mentioned measurement result may be, for example, the information reflecting a fluorescence response described in the section C. The therapeutic effect may be determined by, for example, comparing the above-mentioned measurement result to a predetermined reference value. A measurement result in a disease model to which the candidate compound is not administered or the candidate treatment method is not applied may be used as such reference value. In one embodiment, when the measurement result shows a fluorescence response equal to or more than the above-mentioned predetermined reference value, the above-mentioned compound or treatment method can be determined to have no therapeutic effect on the calpain activity-associated disease. In another embodiment, when the measurement result shows a fluorescence response less than the above-mentioned predetermined reference value, the above-mentioned compound or treatment method can be determined to have a therapeutic effect on the calpain activity-associated disease. The present invention also relates to a test method for the determination of the therapeutic effect of a candidate compound on a calpain activity-associated disease, the method including: preparing a sample from a calpain activity-associated disease model to which the candidate compound is not administered and a sample from the calpain activity-associated disease model to which the candidate compound is administered; bringing the compound (I) and each of the samples into contact with each other; and measuring fluorescence from the compound (I) after the contact. The therapeutic effect of the candidate compound can be determined on the basis of the measurement results in the same manner as that described above. Examples of the calpain activity-associated disease model include a retinal disease model derived by NMDA and a retinal ganglion cell injury model derived by glutamic acid.

In the entirety of this description, the term "level" means an indicator concerning an abundance converted into a numerical value. The term encompasses, for example, a concentration or an amount, or an indicator that may be used instead thereof. Accordingly, the level may be the measured value itself of a fluorescence intensity or the like, or may be a value converted into a concentration. In addition, the level may be an absolute numerical value (e.g., an abundance or an abundance per unit area), or may be a relative numerical value compared to a control set as required.

In the entirety of this description, when the calpain activity-related disease is an ophthalmologic disease, the compound (I) may be administered by intravitreal injection.

### (F. Method of measuring or detecting Cathepsin Activity)

The compound (I) can be used in the measurement or detection of a cathepsin or cathepsin activity because the compound reacts with the cathepsin to show a fluorescence response. Accordingly, in another aspect, the present invention relates to a method of measuring (or detecting) a cathepsin (or cathepsin activity) in a sample, the method including: bringing the compound (I) and the sample into contact with each other; and measuring (or detecting) fluorescence from the compound (I) after the contact. A case in which the fluorescence is detected may mean that the cathepsin or the cathepsin activity is present in the sample. In addition, the detected fluorescence may represent the cathepsin concentration of the sample or the degree of the cathepsin activity thereof. The measurement and detection of the cathepsin (or the cathepsin activity) may be performed by using the measured fluorescence intensity as an absolute value. Alternatively, the cathepsin (or the cathepsin activity) may be measured based on the measured fluorescence intensity by using the amount of a change in fluorescence intensity with time or the fluorescence intensity of a control for comparison. Such fluorescence intensity of the control may be obtained by subjecting the control to measurement simultaneously with the sample, or a value obtained by using the control in advance may be used. The sample, the contact between the compound (I) and the sample, and the measurement of the fluorescence from the compound (I) after the contact are as described for the method of measuring or detecting calpain activity.

The present invention is described in more detail below by way of Examples. However, Examples do not limit the scope of the present invention. The entirety of literatures cited throughout the description of the present application is incorporated herein by reference.

### Examples

### (Example 1) Synthesis of Compound 1-5

### (1) Synthesis of Compound 1

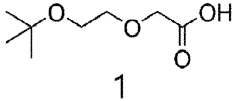

Ethylene glycol monotertbutyl ether (1.4 mL, 11.0 mmol) and bromoacetic acid (1.39 g, 10.0 mmol) were added to a solution (30 mL) of NaH (1.20 g, 30.0 mmol) in THF at 0°C, and the mixture was stirred at room temperature for 14 hours. Water was added to the reaction solution, and the mixture was extracted with diethyl ether. 2 M hydrochloric acid was added to the aqueous phase to neutralize the solution, and the neutralized solution was extracted with ethyl acetate. The organic phase was dried with sodium sulfate, and then the solvent was evaporated under reduced pressure. Thus, a target compound was obtained (1.22 g, 69%).
¹H NMR (400 MHz, CDCl₃) δ1.26 (9H, s), 3.55-3.59 (2H, m), 3.72-3.77 (2H, m), 4.14 (2H, s)
HRMS (ESI⁺) : calcd for [M+Na]⁺, 199.0946; found, 199.0941 (-0.5 mmu)

### (2) Synthesis of Compound 2

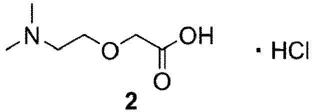

N,N-Dimethyl amino ethanol (1.1 mL, 11.0 mmol) and bromoacetic acid (1.39 g, 10.0 mmol) were added to a solution (30 mL) of NaH (1.20 g, 30.0 mmol) in THF at 0°C, and the mixture was stirred at room temperature for 14 hours. 2 M hydrochloric acid was added to the reaction solution, and the mixture was extracted with diethyl ether. The aqueous phase was collected, and the solvent was evaporated under reduced pressure. Thus, a target compound was obtained (1.80 g, 98%).
¹H NMR (400 MHz, D₂O) δ2.95 (6H, s), 3.39 (2H, t, J=4.8 Hz), 3.89 (2H, t, J=4.8 Hz), 4.19 (2H, s)
HRMS (ESI⁺): calcd for [M+H]⁺, 148.0968; found, 148.0967 (-0.1 mmu)

### (3) Synthesis of Compound 3

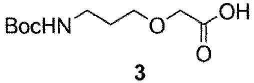

3-(tert-Butoxycarbonylamino)-1-propanol (1.0 mL, 5.88 mmol) and bromoacetic acid (742 mg, 5.34 mmol) were added to a solution of NaH (0.40 g, 10.0 mmol) in THF at 0°C, and the mixture was stirred at room temperature for 19.5 hours. Distilled water was added to the reaction solution, and the mixture was extracted with dichloromethane. 2 M hydrochloric acid was added to the aqueous phase to neutralize the solution, and the neutralized solution was extracted with dichloromethane. The organic phase was dried with sodium sulfate, and then the solvent was evaporated under reduced pressure. The resultant residue was purified by silica gel column chromatography (developing solvent: hexane/ethyl acetate=85/15→35/65) to provide a target compound (0.578 g, 46%).
¹H NMR (400 MHz, CDCl₃) δ1.44 (9H, s), 1.78 (2H, q, J=6.0 Hz), 3.32 (2H, br-s), 3.60 (2H, t, J=6.0 Hz), 4.09 (2H, s)
HRMS (ESI⁺) : calcd for [M+Na]⁺, 256.1161; found, 256.1151 (-1.0 mmu)

### (4) Synthesis of Compound 4

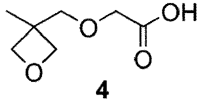

3-Methyl-3-oxethanemethanol (2.4 mL, 30.0 mmol) and bromoacetic acid (1.39 g, 10.0 mmol) were added to a solution of NaH (2.00 g, 50.0 mmol) in THF at 0°C, and the mixture was stirred at room temperature for 2 hours. Water was added to the reaction solution, and the mixture was extracted with diethyl ether. 2 M hydrochloric acid was added to the aqueous phase to neutralize the solution, and the neutralized solution was extracted with ethyl acetate. The organic phase was dried with sodium sulfate, and then the solvent was evaporated under reduced pressure. Thus, a target compound was obtained (1.7 g, 79%).
¹H NMR (400 MHz, CDCl₃) δ1.34 (3H, s), 3.66 (2H, s), 4.19 (2H, s), 4.42 (2H, d, J=6.0 Hz), 4.60 (2H, d, J=6.0 Hz)
HRMS (ESI⁺) : calcd for [M+Na]⁺, 183.0633; found, 183.0629 (-0.4 mmu)

### (5) Synthesis of Compound 5

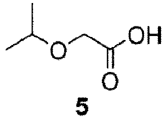

2-Propanol (0.77 mL, 10.0 mmol) and bromoacetic acid (0.695 g, 5.0 mmol) were added to a solution of NaH (0.60 g, 15.0 mmol) in THF at 0°C, and the mixture was stirred at room temperature for 21 hours. Water was added to the reaction solution, and the mixture was extracted with dichloromethane. 2 M hydrochloric acid was added to the aqueous phase to neutralize the solution, and the neutralized solution was extracted with dichloromethane. The organic phase was dried with sodium sulfate, and then the solvent was evaporated under reduced pressure. Thus, a target compound was obtained (0.219 g, 37%).
¹H NMR (400 MHz, CDCl₃) δ1.24 (6H, d, J=6.0 Hz), 3.76 (1H, qn, J=6.0 Hz), 4.08 (2H, s)
HRMS (ESI⁺): calcd for [M+H]⁺, 119.0703; found, 119.0704 (0.1 mmu)

### (Example 2) Synthesis of Compound A7 and Compound A7'

Compound A7 and Compound A7' having various peptide moieties were synthesized through steps represented in the following scheme.

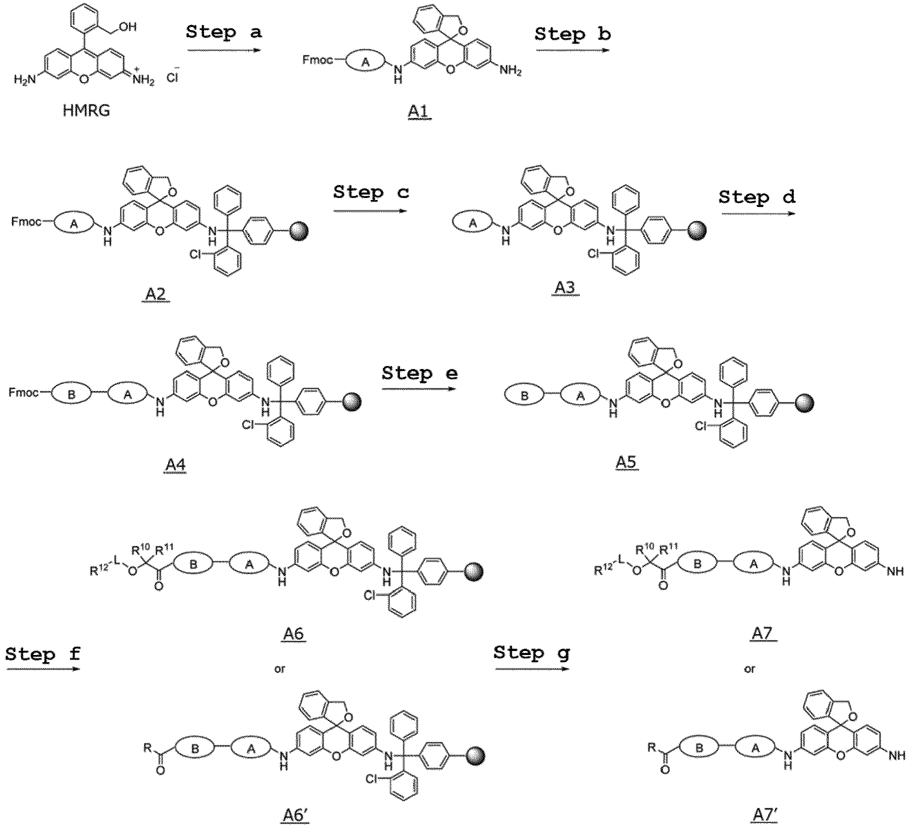

### (1) Compound 6 to Compound 15 were each synthesized as Compound A1.

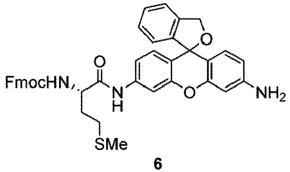

HRMS (ESI⁺): calcd for [M+H]⁺, 670.2370; found, 670.2311 (-5.9 mmu)

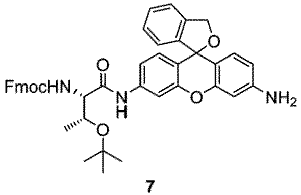

HRMS (ESI⁺): calcd for [M+H]⁺, 696.3068; found, 696.3093 (2.5 mmu)

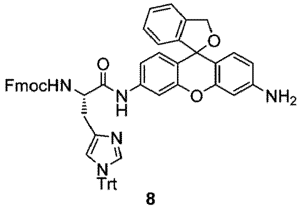

HRMS (ESI⁺): calcd for [M+H]⁺, 918.3650; found, 918.3627 (-2.3 mmu) 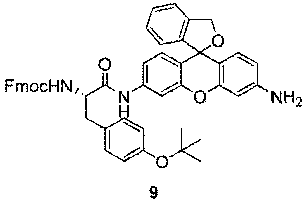 HRMS (ESI⁺): calcd for [M+H]⁺, 758.3225; found, 758.3242 (1.7 mmu) 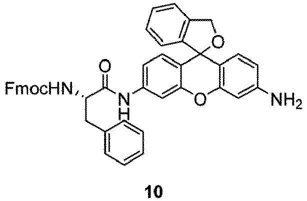 HRMS (ESI⁺): calcd for [M+H]⁺, 686.2649; found, 686.2654 (0.5 mmu)

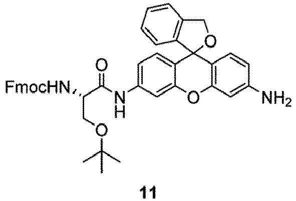

HRMS (ESI⁺): calcd for [M+H]⁺, 682.2912; found, 682.2914 (0.2 mmu)

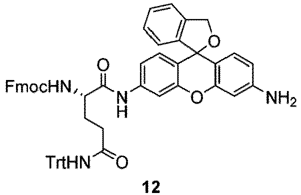

HRMS (ESI⁺): calcd for [M+H]⁺, 909.3647; found, 909.3628 (-1.9 mmu)

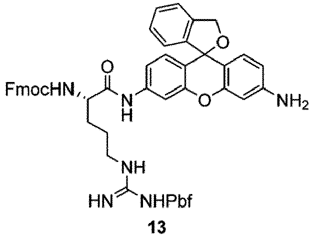

HRMS (ESI⁺): calcd for [M+H]⁺, 947.3797; found, 947.3770 (-2.7 mmu)

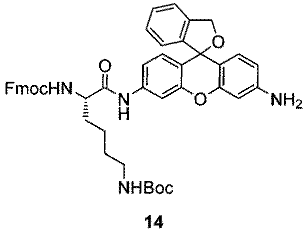

HRMS (ESI⁺) : calcd for [M+H]⁺, 767.3439; found, 767.3486 (4.7 mmu)

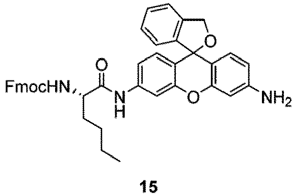

HRMS (ESI⁺): calcd for [M+H]⁺, 652.2806; found, 652.2797 (-0.9 mmu)

### (2) Compound 16 to Compound 60 were synthesized as Compound A7 and Compound A7'.

### (2-1) Synthesis of Compound 16

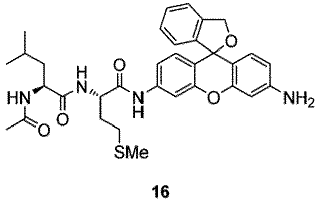

DIPEA (2.1 mL, 12.0 mmol), Fmoc-Met-OH (1.49 g, 4.00 mmol), and HATU (1.52 g, 4.00 mmol) were added to a solution (13 mL) of HMRG hydrochloride (Goryo Chemical, Inc., 1.41 g, 2.83 mmol) in DMF at room temperature, and the mixture was stirred for 15 hours. The solvent was evaporated under reduced pressure, and the residue was roughly purified by silica gel chromatography. Compound 6 thus obtained was used in the next step without being further purified.

DIPEA (1.0 mL, 5.73 mmol) and Cl-Trt(2-Cl)-resin (818 mg, 1.33 mmol) were added to a solution (10 mL) of Compound 6 in dichloromethane at room temperature, and the mixture was shaken for 15 hours. The reaction solution was filtered, and the resin was washed with DMF. Thus, Compound A2 (A=Met) was obtained.

A 20% solution (10 mL) of piperidine in DMF was added to Compound A2 (A=Met), and the mixture was shaken at room temperature for 1 hour. The reaction solution was filtered, and the resin was washed with DMF and CH₂Cl₂ in the stated order. Thus, Compound A3 (A=Met) was obtained.

DMF (20 mL), DIPEA (2.1 mL, 12.0 mmol), Fmoc-Leu-OH (1.41 g, 3.99 mmol), and HATU (1.52 g, 3.99 mmol) were sequentially added to Compound A3 (A=Met), and the mixture was shaken at room temperature for 13.5 hours. The resin was washed with DMF. Thus, Compound A4 (A=Met, B=Leu) was obtained.

A 20% solution (10 mL) of piperidine in DMF was added to Compound A4 (A=Met, B=Leu), and the mixture was shaken at room temperature for 1 hour. The reaction solution was filtered, and the resin was washed with DMF and CH₂Cl₂ in the stated order. The resin was dried under vacuum to provide Compound A5 (A=Met, B=Leu).

1 Milliliter of a 20% solution of Ac₂O in CH₂Cl₂ was added to Compound A5 (A=Met, B=Leu) whose amount corresponded to 0.05 mmol, and the mixture was shaken at room temperature for 1 hour. The reaction solution was filtered, and the resin was washed with CH₂Cl₂. Thus, Compound A6' (A=Met, B=Leu, R=Me) was obtained.

A solution (1 mL) containing TFA, TES, and H₂O at a ratio of 15:1:1 was added to Compound A6' (A=Met, B=Leu, R=Me), and the mixture was shaken at room temperature under light shielding for 1 hour. The resin was filtered out, and the filtrate was evaporated under reduced pressure. The residue was purified by reverse-phase column chromatography (H₂O (0.1% TFA)/CH₃CN (0.1% TFA)=80:20→65:35) to provide a target compound (7.6 mg) .
HRMS (ESI⁺): calcd for [M+H]⁺, 603.2636; found, 603.2643 (0.7 mmu)

### (2-2) Synthesis of Compound 17

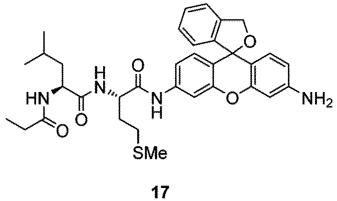

DMF (4 mL), DIPEA (0.09 mL, 0.50 mmol), propionic acid (0.014 mL, 0.15 mmol), and HATU (57.0 mg, 0.15 mmol) were sequentially added to Compound A5 (A=Met, B=Leu) whose amount corresponded to 0.05 mmol, and the mixture was shaken at room temperature for 12 hours. The reaction solution was filtered, and the resin was sequentially washed with DMF and CH₂Cl₂. A solution (1 mL) containing TFA, TES, and H₂O at a ratio of 15:1:1 was added to the washed resin, and the mixture was shaken at room temperature under light shielding for 1 hour. The resin was filtered out, and the filtrate was evaporated under reduced pressure. The residue was purified by reverse-phase column chromatography (H₂O (0.1% TFA)/CH₃CN (0.1% TFA)=80:20→65:35) to provide a TFA salt of a target compound (7.5 mg).
HRMS (ESI⁺): calcd for [M+H]⁺, 617.2792; found, 617.2802 (1.0 mmu)

### (2-3) Synthesis of Compound 18

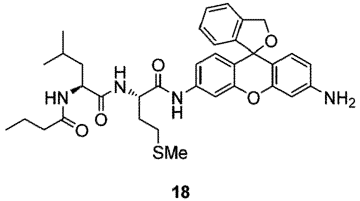

Compound 18 was synthesized by the same approach as that in the section (2-2). A TFA salt of a target compound (7.3 mg) was obtained by using butyric acid as a carboxylic acid.
HRMS (ESI⁺): calcd for [M+H]⁺, 631.2949; found, 631.2962 (1.3 mmu)

### (2-4) Synthesis of Compound 19

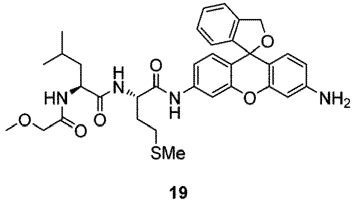

Compound 19 was synthesized by the same approach as that in the section (2-2). A TFA salt of a target compound (12.8 mg) was obtained by using methoxyacetic acid as a carboxylic acid.
¹H NMR (400 MHz, CD₃OD+Na₂CO₃)
δ 0.94 (3H, d, J=6.0 Hz), 0.97 (3H, d, J=6.0 Hz), 1.59-1.73 (3H, m), 1.97-2.08 (1H, m), 2.09 (3H, s), 2.11-2.21 (1H, m), 2.49-2.65 (2H, m), 3.43 (3H, s), 3.94 (2H, s), 4.51 (1H, t, J=7.2 Hz), 4.59 (1H, dd, J=5.2, 9.2 Hz), 5.25 (2H, s), 6.41 (1H, d, J=8.4 Hz), 6.50 (1H, s), 6.65 (1H, d, J=8.4 Hz), 6.81 (1H, d, J=7.6 Hz), 6.84 (1H, d, J=8.8 Hz), 7.13 (1H, d, J=8.8 Hz), 7.27 (1H, t, J=7.2 Hz), 7.38 (1H, t, J=7.6 Hz), 7.41 (1H, t, J=7.6 Hz), 7.62 (1H, s)
HRMS (ESI⁺): calcd for [M+H]⁺, 633.2741; found, 633.2723 (-1.8 mmu)

### (2-5) Synthesis of Compound 20

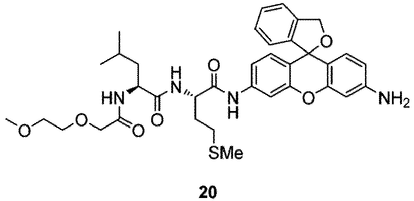

Compound 20 was synthesized by the same approach as that in the section (2-2). A TFA salt of a target compound (16.3 mg) was obtained by using 2-(2-methoxyethoxy)acetic acid as a carboxylic acid.
HRMS (ESI⁺): calcd for [M+H]⁺, 677.3003; found, 677.2997 (-0.6 mmu)

### (2-6) Synthesis of Compound 21

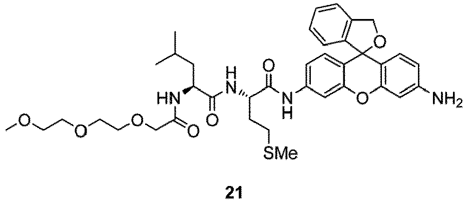

Compound 21 was synthesized by the same approach as that in the section (2-2). A TFA salt of a target compound (19.7 mg) was obtained by using [2-(2-methoxyethoxy)ethoxy]acetic acid as a carboxylic acid.
HRMS (ESI⁺): calcd for [M+H]⁺, 721.3266; found, 721.3246 (-2.0 mmu)

### (2-7) Synthesis of Compound 22

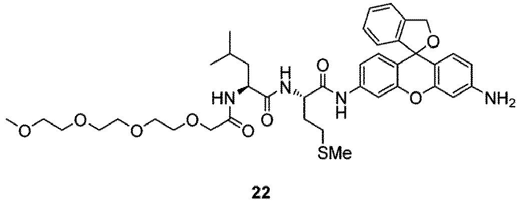

Compound 22 was synthesized by the same approach as that in the section (2-2). A TFA salt of a target compound (5.9 mg) was obtained by using 2-[2-[2-(2-methoxyethoxy)ethoxy]ethoxy] acetic acid as a carboxylic acid.
HRMS (ESI⁺): calcd for [M+H]⁺, 765.3528; found, 765.3520 (-0.8 mmu)

### (2-8) Synthesis of Compound 23

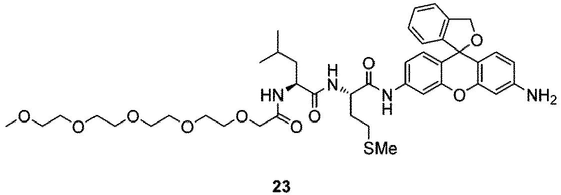

Compound 23 was synthesized by the same approach as that in the section (2-2). A TFA salt of a target compound (10.8 mg) was obtained by using [2-[2-[2-(2-methoxyethoxy)ethoxy]ethoxy]ethoxy]acetic acid as a carboxylic acid.
HRMS (ESI⁺): calcd for [M+H]⁺, 809.3790; found, 809.3821 (3.1 mmu)

### (2-9) Synthesis of Compound 24

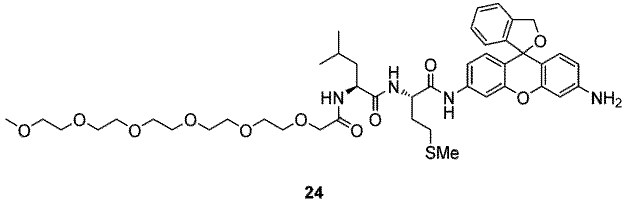

Compound 24 was synthesized by the same approach as that in the section (2-2). A TFA salt of a target compound (10.8 mg) was obtained by using [2-[2-[2-[2-(2-methoxyethoxy)ethoxy]ethoxy]ethoxy]ethoxy]acetic acid as a carboxylic acid.
HRMS (ESI⁺): calcd for [M+H]⁺, 853.4052; found, 853.4048 (-0.4 mmu)

### (2-10) Synthesis of Compound 25

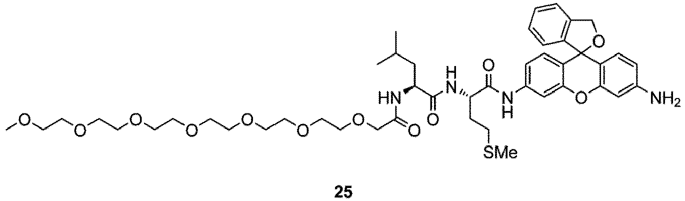

Compound 25 was synthesized by the same approach as that in the section (2-2). A TFA salt of a target compound (10.8 mg) was obtained by using [2-[2-[2-[2-[2-(2-methoxyethoxy)ethoxy]ethoxy]ethoxy]ethoxy]ethoxy]acetic acid as a carboxylic acid.
HRMS (ESI⁺): calcd for [M+H]⁺, 897.4314; found, 897.4312 (-0.2 mmu)

### (2-11) Synthesis of Compound 26

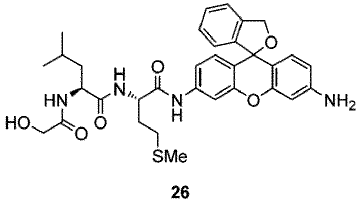

Compound 26 was synthesized by the same approach as that in the section (2-2). A TFA salt of a target compound (7.1 mg) was obtained by using hydroxyacetic acid as a carboxylic acid.
HRMS (ESI⁺): calcd for [M+H]⁺, 619.2585; found, 619.2595 (1.0 mmu)

### (2-12) Synthesis of Compound 27

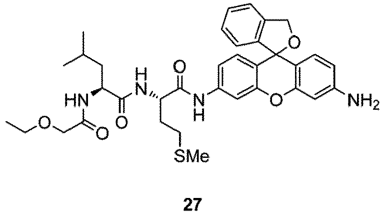

Compound 27 was synthesized by the same approach as that in the section (2-2). A TFA salt of a target compound (8.2 mg) was obtained by using ethoxyacetic acid as a carboxylic acid.
HRMS (ESI⁺): calcd for [M+H]⁺, 647.2898; found, 647.2898 (0.0 mmu)

### (2-13) Synthesis of Compound 28

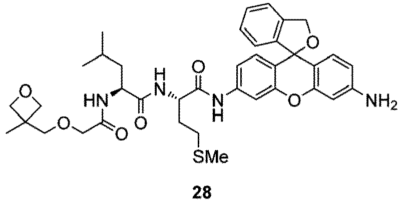

Compound 28 was synthesized by the same approach as that in the section (2-2). A TFA salt of a target compound (11.8 mg) was obtained by using Compound 4 as a carboxylic acid.
HRMS (ESI⁺): calcd for [M+H]⁺, 703.3160; found, 703.3160 (0.0 mmu)

### (2-14) Synthesis of Compound 29

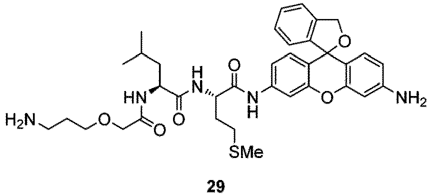

Compound 29 was synthesized by the same approach as that in the section (2-2). A TFA salt of a target compound (11.8 mg) was obtained by using Compound 3 as a carboxylic acid.
HRMS (ESI⁺): calcd for [M+H]⁺, 676.3163; found, 676.3137 (-2.6 mmu)

### (2-15) Synthesis of Compound 30

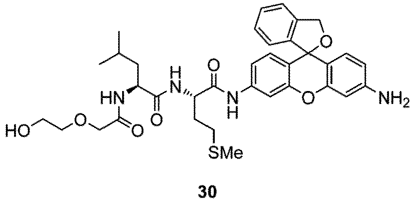

Compound 30 was synthesized by the same approach as that in the section (2-2). A TFA salt of a target compound (13.9 mg) was obtained by using Compound 1 as a carboxylic acid.
HRMS (ESI⁺): calcd for [M+H]⁺, 663.2847; found, 663.2837 (-1.0 mmu)

### (2-16) Synthesis of Compound 31

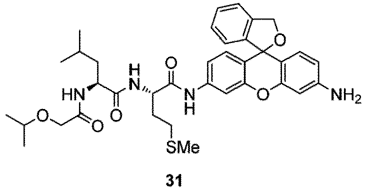

Compound 31 was synthesized by the same approach as that in the section (2-2). A TFA salt of a target compound (28.3 mg) was obtained by using Compound 5 as a carboxylic acid.
HRMS (ESI⁺): calcd for [M+H]⁺, 661.3054; found, 661.3044 (-1.0 mmu)

### (2-17) Synthesis of Compound 32

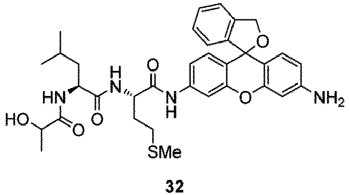

Compound 32 was synthesized by the same approach as that in the section (2-2). A TFA salt of a target compound (26.2 mg) was obtained by using DL-lactic acid as a carboxylic acid.
HRMS (ESI⁺): calcd for [M+H]⁺, 633.2741; found, 633.2731 (-1.0 mmu)

### (2-18) Synthesis of Compound 33

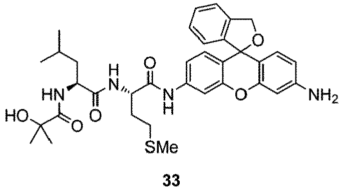

Compound 33 was synthesized by the same approach as that in the section (2-2). A TFA salt of a target compound (19.9 mg) was obtained by using 2-hydroxyisobutyric acid as a carboxylic acid.
HRMS (ESI⁺): calcd for [M+H]⁺, 647.2898; found, 647.2890 (-0.8 mmu)

### (2-19) Synthesis of Compound 34

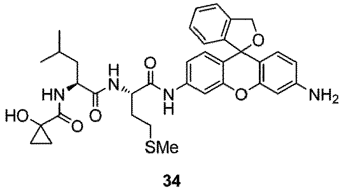

Compound 34 was synthesized by the same approach as that in the section (2-2). A TFA salt of a target compound (18.4 mg) was obtained by using 1-hydroxycyclopropanecarboxylic acid as a carboxylic acid.
HRMS (ESI⁺): calcd for [M+H]⁺, 645.2741; found, 645.2717 (-2.4 mmu)

### (2-20) Synthesis of Compound 35

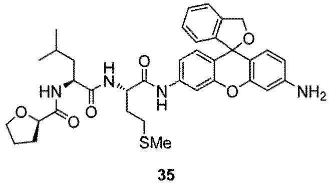

Compound 35 was synthesized by the same approach as that in the section (2-2). A TFA salt of a target compound (6.4 mg) was obtained by using (R)-(+)-tetrahydrofuran-2-carboxylic acid as a carboxylic acid.
HRMS (ESI⁺): calcd for [M+H]⁺, 659.2898; found, 659.2885 (-1.3 mmu)

### (2-21) Synthesis of Compound 36

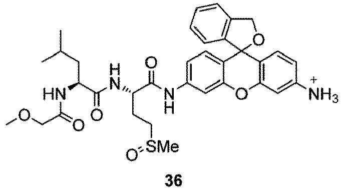

Hydrogen peroxide water (0.5 mL) was added to a solution (2 mL) of Compound 19 (0.0410 g, 0.0647 mmol) in THF, and the mixture was stirred at room temperature for 2 hours. A saturated aqueous solution of sodium hydrogen carbonate and a saturated aqueous solution of sodium thiosulfate were added to the reaction solution, and the mixture was extracted with dichloromethane. The organic phase was dried with sodium sulfate, and then the solvent was evaporated under reduced pressure. Thus, a target compound was obtained (0.0417 g, 99%).
HRMS (ESI⁺): calcd for [M+H]⁺, 649.2690; found, 649.2660 (-3.0 mmu)

### (2-22) Synthesis of Compound 37

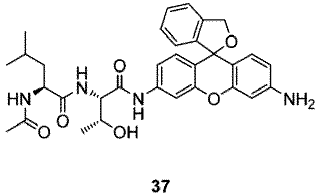

Compound 37 was synthesized by the same approach as that in the section (2-1).

Compound 7 was synthesized by changing the amino acid in Step (a) from Fmoc-Met-OH to Fmoc-Thr(tBu)-OH. Compound A5 (A=Thr(tBu), B=Leu) was synthesized from Compound 7 through the same steps as those in the section (2-1). A TFA salt of a target compound (5.5 mg) was obtained by using Compound A5 (A=Thr(tBu), B=Leu) in an amount corresponding to 0.05 mmol.
HRMS (ESI⁺): calcd for [M+H]⁺, 573.2708; found, 573.2715 (0.7 mmu)

### (2-23) Synthesis of Compound 38

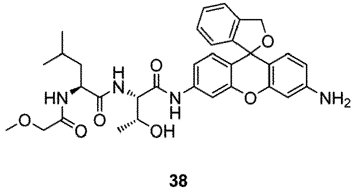

Compound 38 was synthesized through the same steps as those in the section (2-4). A TFA salt of a target compound (10.7 mg) was obtained by using Compound A5 (A=Thr(tBu), B=Leu) whose amount corresponded to 0.05 mmol.
HRMS (ESI⁺): calcd for [M+H]⁺, 603.2813; found, 603.2818 (0.5 mmu)

### (2-24) Synthesis of Compound 39

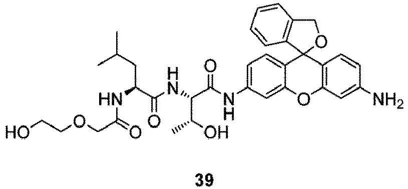

Compound 39 was synthesized through the same steps as those in the section (2-15). A TFA salt of a target compound (3.6 mg) was obtained by using Compound A5 (A=Thr(tBu), B=Leu) whose amount corresponded to 0.05 mmol.
HRMS (ESI⁺): calcd for [M+H]⁺, 633.2919; found, 633.2876 (-4.3 mmu)

### (2-25) Synthesis of Compound 40

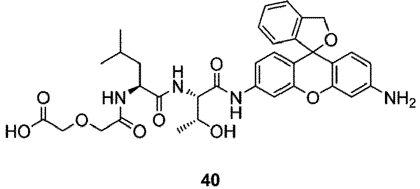

DIPEA (0.043 mL, 0.25 mmol) and diglycolic anhydride (29.0 mg, 0.25 mmol) were added to a solution (1 mL) of Compound A5 (A=Thr(tBu), B=Leu) whose amount corresponded to 0.05 mmol in CH₂Cl₂, and the mixture was shaken at room temperature for 24 hours. The reaction solution was filtered, and the resin was washed with CH₂Cl₂. A solution (1 mL) containing TFA, TES, and H₂O at a ratio of 15:1:1 was added to the washed resin, and the mixture was shaken at room temperature under light shielding for 1 hour. The resin was filtered out, and the filtrate was evaporated under reduced pressure. The residue was purified by reverse-phase column chromatography to provide a target compound (18.6 mg).
HRMS (ESI⁺): calcd for [M+H]⁺, 647.2712; found, 647.2716 (0.4 mmu)

### (2-26) Synthesis of Compound 41

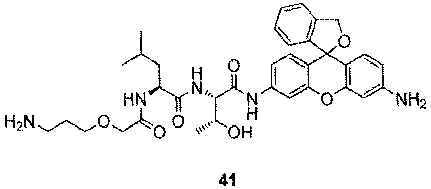

Compound 41 was synthesized by the same approach as that in the section (2-14). A TFA salt of a target compound (16.5 mg) was obtained by using Compound A5 (A=Thr(tBu), B=Leu) whose amount corresponded to 0.05 mmol.
HRMS (ESI⁺): calcd for [M+H]⁺, 646.3235; found, 646.3206 (-2.9 mmu)

### (2-27) Synthesis of Compound 42

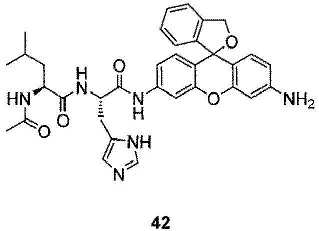

Compound 42 was synthesized by the same approach as that in the section (2-1).

Compound 8 was synthesized by changing the amino acid in Step (a) from Fmoc-Met-OH to Fmoc-His(Trt)-OH. Compound A5 (A=His(Trt), B=Leu) was synthesized from Compound 8 through the same steps as those in the section (2-1). A TFA salt of a target compound (7.9 mg) was obtained by using Compound A5 (A=His(Trt), B=Leu) in an amount corresponding to 0.05 mmol.
HRMS (ESI⁺): calcd for [M+H]⁺, 609.2820; found, 609.2820 (0 mmu)

### (2-28) Synthesis of Compound 43

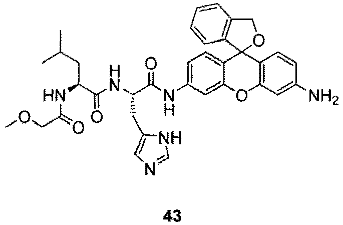

Compound 43 was synthesized through the same steps as those in the section (2-4). A TFA salt of a target compound (15.9 mg) was obtained by using Compound A5 (A=His(Trt), B=Leu) whose amount corresponded to 0.10 mmol.
HRMS (ESI⁺): calcd for [M+H]⁺, 639.2926; found, 639.2949 (2.3 mmu)

### (2-29) Synthesis of Compound 44

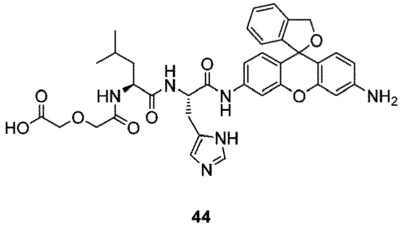

Compound 44 was synthesized through the same steps as those in the section (2-25). A TFA salt of a target compound (11.2 mg) was obtained by using Compound A5 (A=His(Trt), B=Leu) whose amount corresponded to 0.050 mmol.
HRMS (ESI⁺): calcd for [M+H]⁺, 683.2824; found, 683.2820 (-0.4 mmu)

### (2-30) Synthesis of Compound 45

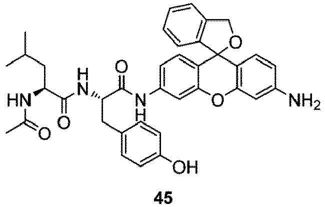

Compound 45 was synthesized by the same approach as that in the section (2-1).

Compound 9 was synthesized by changing the amino acid in Step (a) from Fmoc-Met-OH to Fmoc-Tyr(tBu)-OH. Compound A5 (A=Tyr(tBu), B=Leu) was synthesized from Compound 9 through the same steps as those in the section (2-1). A TFA salt of a target compound (3.0 mg) was obtained by using Compound A5 (A=Tyr(tBu), B=Leu) in an amount corresponding to 0.025 mmol.
HRMS (ESI⁺): calcd for [M+H]⁺, 635.2864; found, 635.2867 (0.3 mmu)

### (2-31) Synthesis of Compound 46

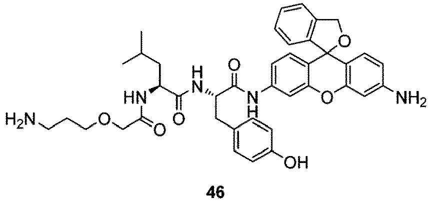

Compound 46 was synthesized through the same steps as those in the section (2-14). A TFA salt of a target compound (23.1 mg) was obtained by using Compound A5 (A=Tyr(tBu), B=Leu) whose amount corresponded to 0.050 mmol.
HRMS(ESI⁺): calcd for [M+H]⁺, 708.3392; found, 708.3358 (-3.4 mmu)

### (2-32) Synthesis of Compound 47

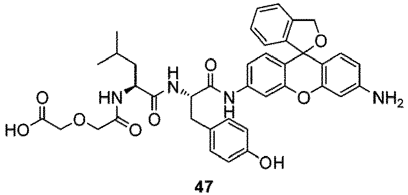

Compound 47 was synthesized through the same steps as those in the section (2-25). A TFA salt of a target compound (22.1 mg) was obtained by using Compound A5 (A=Tyr(tBu), B=Leu) whose amount corresponded to 0.050 mmol.
HRMS (ESI⁺): calcd for [M+H]⁺, 709.2868; found, 709.2868 (0.0 mmu)

### (2-33) Synthesis of Compound 48

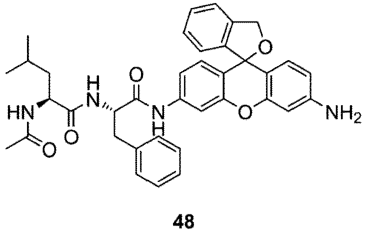

Compound 48 was synthesized by the same approach as that in the section (2-1).

Compound 10 was synthesized by changing the amino acid in Step (a) from Fmoc-Met-OH to Fmoc-Phe-OH. Compound A5 (A=Phe, B=Leu) was synthesized from Compound 10 through the same steps as those in the section (2-1). A TFA salt of a target compound (3.0 mg) was obtained by using Compound A5 (A=Phe, B=Leu) in an amount corresponding to 0.025 mmol.
HRMS (ESI⁺): calcd for [M+H]⁺, 619.2915; found, 619.2910 (-0.5 mmu)

### (2-34) Synthesis of Compound 49

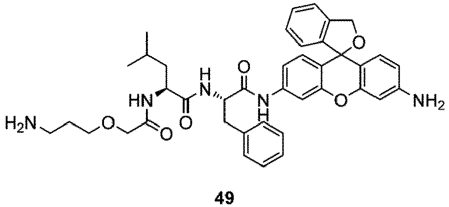

Compound 49 was synthesized through the same steps as those in the section (2-14). A TFA salt of a target compound (15.4 mg) was obtained by using Compound A5 (A=Phe, B=Leu) whose amount corresponded to 0.050 mmol.
HRMS (ESI⁺): calcd for [M+H]⁺, 692.3443; found, 692.3412 (-3.1 mmu)

### (2-35) Synthesis of Compound 50

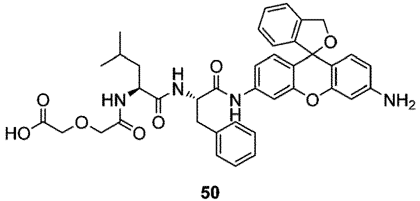

Compound 50 was synthesized through the same steps as those in the section (2-25). A TFA salt of a target compound (7.3 mg) was obtained by using Compound A5 (A=Phe, B=Leu) whose amount corresponded to 0.050 mmol.
HRMS (ESI⁺): calcd for [M+H]⁺, 693.2919; found, 693.2922 (0.2 mmu)

### (2-36) Synthesis of Compound 51

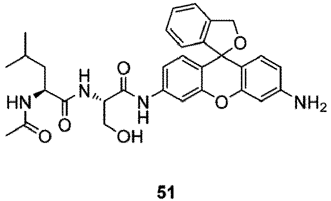

Compound 51 was synthesized by the same approach as that in the section (2-1).

Compound 11 was synthesized by changing the amino acid in Step (a) from Fmoc-Met-OH to Fmoc-Ser(tBu)-OH. Compound A5 (A=Ser(tBu), B=Leu) was synthesized from Compound 11 through the same steps as those in the section (2-1). A TFA salt of a target compound (6.4 mg) was obtained by using Compound A5 (A=Ser(tBu), B=Leu) in an amount corresponding to 0.025 mmol.
HRMS (ESI⁺): calcd for [M+H]⁺, 559.2551; found, 559.2555 (0.4 mmu)

### (2-37) Synthesis of Compound 52

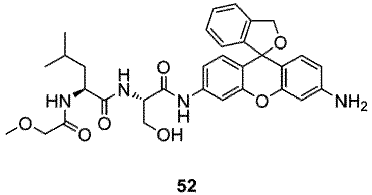

Compound 52 was synthesized through the same steps as those in the section (2-4). A TFA salt of a target compound (7.2 mg) was obtained by using Compound A5 (A=Ser(tBu), B=Leu) whose amount corresponded to 0.025 mmol.
HRMS (ESI⁺): calcd for [M+H]⁺, 589.2657; found, 589.2692 (3.5 mmu)

### (2-38) Synthesis of Compound 53

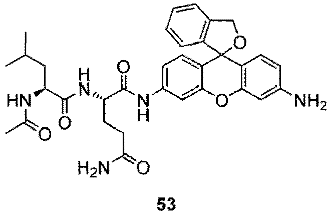

Compound 53 was synthesized by the same approach as that in the section (2-1).

Compound 12 was synthesized by changing the amino acid in Step (a) from Fmoc-Met-OH to Fmoc-Gln(Trt)-OH. Compound A5 (A=Gln(Trt), B=Leu) was synthesized from Compound 12 through the same steps as those in the section (2-1). A TFA salt of a target compound (2.9 mg) was obtained by using Compound A5 (A=Gln(Trt), B=Leu) in an amount corresponding to 0.025 mmol.
HRMS (ESI⁺): calcd for [M+H]⁺, 600.2817; found, 600.2821 (0.4 mmu)

### (2-39) Synthesis of Compound 54

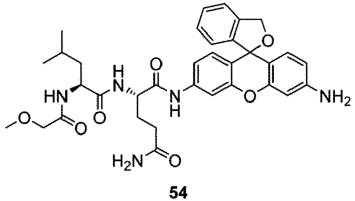

Compound 54 was synthesized through the same steps as those in the section (2-4). A TFA salt of a target compound (3.4 mg) was obtained by using Compound A5 (A=Gln(Trt), B=Leu) whose amount corresponded to 0.025 mmol.
HRMS (ESI⁺): calcd for [M+H]⁺, 630.2922; found, 630.2916 (-0.6 mmu)

### (2-40) Synthesis of Compound 55

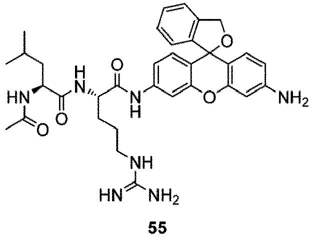

Compound 55 was synthesized by the same approach as that in the section (2-1).

Compound 13 was synthesized by changing the amino acid in Step (a) from Fmoc-Met-OH to Fmoc-Arg(Pbf)-OH. Compound A5 (A=Arg(Pbf), B=Leu) was synthesized from Compound 13 through the same steps as those in the section (2-1). A TFA salt of a target compound (1.5 mg) was obtained by using Compound A5 (A=Arg(Pbf), B=Leu) in an amount corresponding to 0.025 mmol.
HRMS (ESI⁺): calcd for [M+H]⁺, 628.3242; found, 628.3237 (-0.5 mmu)

### (2-41) Synthesis of Compound 56

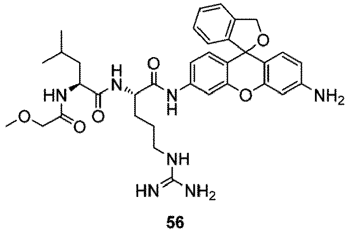

Compound 56 was synthesized through the same steps as those in the section (2-4). A TFA salt of a target compound (3.3 mg) was obtained by using Compound A5 (A=Arg(Pbf), B=Leu) whose amount corresponded to 0.025 mmol.
HRMS (ESI⁺): calcd for [M+H]⁺, 658.3348; found, 658.3353 (0.5 mmu)

### (2-42) Synthesis of Compound 57

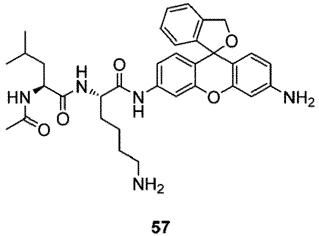

Compound 57 was synthesized by the same approach as that in the section (2-1).

Compound 14 was synthesized by changing the amino acid in Step (a) from Fmoc-Met-OH to Fmoc-Lys (Boc)-OH. Compound A5 (A=Lys(Boc), B=Leu) was synthesized from Compound 14 through the same steps as those in the section (2-1). A TFA salt of a target compound (11.1 mg) was obtained by using Compound A5 (A=Lys(Boc), B=Leu) in an amount corresponding to 0.050 mmol.
HRMS (ESI⁺): calcd for [M+H]⁺, 600.3180; found, 600.3176 (-0.4 mmu)

### (2-43) Synthesis of Compound 58

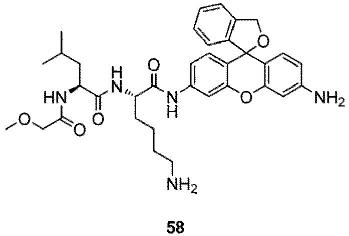

Compound 58 was synthesized through the same steps as those in the section (2-4). A TFA salt of a target compound (12.1 mg) was obtained by using Compound A5 (A=Lys(Boc), B=Leu) whose amount corresponded to 0.10 mmol.
HRMS (ESI⁺): calcd for [M+H]⁺, 630.3286; found, 630.3287 (0.1 mmu)

### (2-44) Synthesis of Compound 59

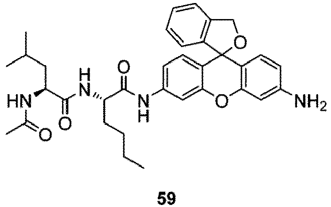

Compound 59 was synthesized by the same approach as that in the section (2-1).

Compound 15 was synthesized by changing the amino acid in Step (a) from Fmoc-Met-OH to Fmoc-Nle-OH. Compound A5 (A=Nle, B=Leu) was synthesized from Compound 15 through the same steps as those in the section (2-1). A TFA salt of a target compound (6.7 mg) was obtained by using Compound A5 (A=Nle, B=Leu) in an amount corresponding to 0.050 mmol.
HRMS (ESI⁺): calcd for [M+H]⁺, 585.3071; found, 585.3077 (0.6 mmu)

### (2-45) Synthesis of Compound 60

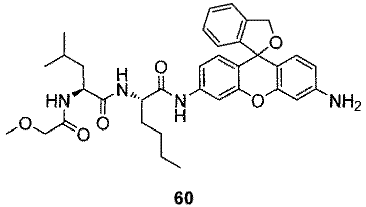

Compound 60 was synthesized by the same approach as that in the section (2-4). A TFA salt of a target compound (8.2 mg) was obtained by using Compound A5 (A=Nle, B=Leu) whose amount corresponded to 0.050 mmol.
HRMS (ESI⁺): calcd for [M+H]⁺, 615.3177; found, 615.3184 (0.7 mmu)

### (2-46) Synthesis of Compound 61

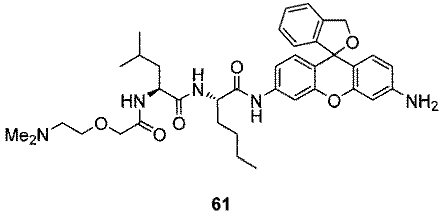

DMF (4 mL), DIPEA (0.09 mL, 0.50 mmol), Compound 2 (29.6 mg, 0.20 mmol), and HATU (57.0 mg, 0.15 mmol) were sequentially added to Compound A5 (A=Nle, B=Leu) whose amount corresponded to 0.05 mmol, and the mixture was shaken at room temperature for 24 hours. The reaction solution was filtered, and the resin was sequentially washed with DMF and CH₂Cl₂. A solution (1 mL) containing TFA, TES, and H₂O at a ratio of 15:1:1 was added to the washed resin, and the mixture was shaken at room temperature under light shielding for 1 hour. The resin was filtered out, and the filtrate was evaporated under reduced pressure. The residue was purified by reverse-phase column chromatography (H₂O (0.1% TFA)/CH₃CN (0.1% TFA)=80:20→65:35) to provide a TFA salt of a target compound (4.7 mg).
HRMS (ESI⁺): calcd for [M+H]⁺, 672.3756; found, 672.3762 (0.6 mmu)

### (2-47) Synthesis of Compound 62

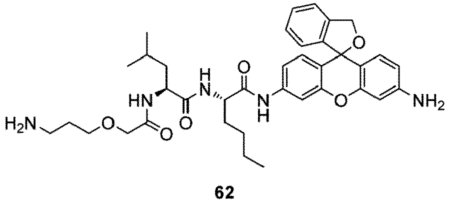

Compound 62 was synthesized by the same approach as that in the section (2-14). A TFA salt of a target compound (12.1 mg) was obtained by using Compound A5 (A=Nle, B=Leu) whose amount corresponded to 0.050 mmol.
HRMS (ESI⁺): calcd for [M+H]⁺, 658.3599; found, 658.3608 (0.9 mmu)

### (2-48) Synthesis of Compound 63

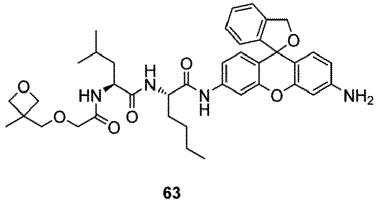

Compound 63 was synthesized by the same approach as that in the section (2-13). A TFA salt of a target compound (9.1 mg) was obtained by using Compound A5 (A=Nle, B=Leu) whose amount corresponded to 0.050 mmol.
HRMS (ESI⁺): calcd for [M+H]⁺, 685.3596; found, 685.3607 (1.1 mmu)

### (2-49) Synthesis of Compound 64

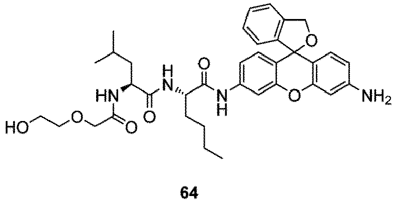

Compound 64 was synthesized by the same approach as that in the section (2-15). A TFA salt of a target compound (4.6 mg) was obtained by using Compound A5 (A=Nle, B=Leu) whose amount corresponded to 0.050 mmol.
HRMS (ESI⁺): calcd for [M+H]⁺, 645.3283; found, 645.3256 (-2.7 mmu)

### (2-50) Synthesis of Compound 65

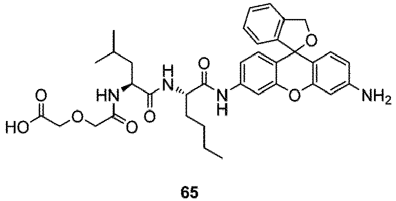

Compound 65 was synthesized by the same approach as that in the section (2-25). A TFA salt of a target compound (8.0 mg) was obtained by using Compound A5 (A=Nle, B=Leu) whose amount corresponded to 0.050 mmol.
HRMS (ESI⁺): calcd for [M+H]⁺, 659.3075; found, 659.3073 (-0.2 mmu)

### (2-51) Synthesis of Compound 66

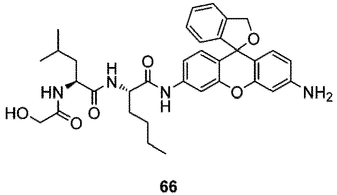

Compound 66 was synthesized by the same approach as that in the section (2-11). A TFA salt of a target compound (5.5 mg) was obtained by using Compound A5 (A=Nle, B=Leu) whose amount corresponded to 0.050 mmol.
HRMS (ESI⁺): calcd for [M+H]⁺, 601.3021; found, 601.3046 (2.5 mmu)

### (Example 3) Optical Characteristics of Fluorescent Probe

The absorption spectrum and fluorescence spectrum of Compound 19 at each pH were each measured. Specifically, a measurement solution was prepared by adding a solution (0.1 mM) of Compound 19 in DMSO to a 10 mM phosphate buffer at each pH in the pH range of from 2 to 11 so that the final probe concentration became 1 µM, and the final DMSO concentration became 1%. The fluorescence spectrum of the measurement solution was measured at an excitation wavelength of 490 nm.

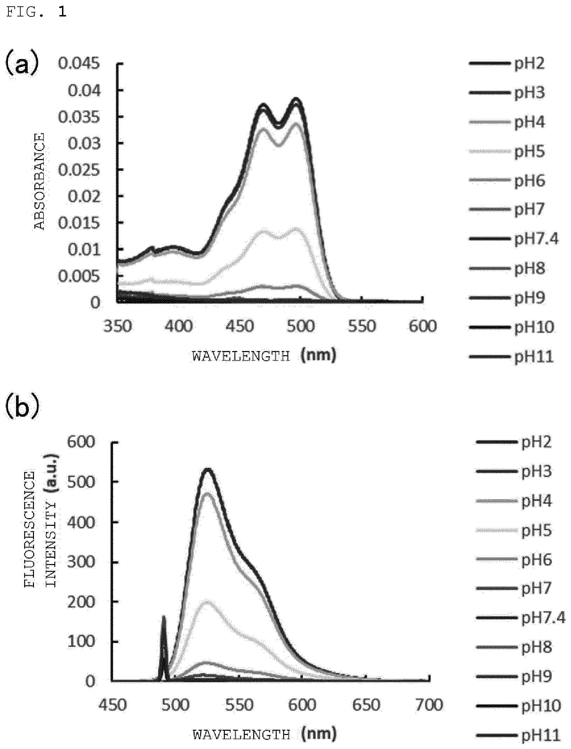

The spectra at the respective pHs are shown in FIG. **1(a)** and FIG. 1(b). It was shown that Compound 19 serving as the fluorescent probe of the present invention became mainly colorless and nonfluorescent under a neutral condition.

### (Example 4) Reactivity of Fluorescent Probe (Compound 19) with Calpain

Calpain-1 was caused to act on Compound 19, and changes in absorption spectrum and fluorescence spectrum thereof were measured. Specifically, a solution (0.5 mM) of Compound 19 in DMSO and calpain-1 (100 units) (calpain-1 from human erythrocytes, Cat. No. 208713, Merck Millipore, the same holds true for the following) were added to a 50 mM TRIS buffer (pH: 7.4, 5 mM CaCl₂, 100 mM NaCl, 1 mM EGTA) to produce 2 mL of a reaction solution (final probe concentration: 5 µM). An enzyme reaction was performed at 37°C. The fluorescence spectrum of the reaction solution was measured at an excitation wavelength of 490 nm. In addition, the reaction solution was analyzed by HPLC chromatography. Compound 19 to which calpain-1 was not added (final concentration: 5 µM) was used as a control.

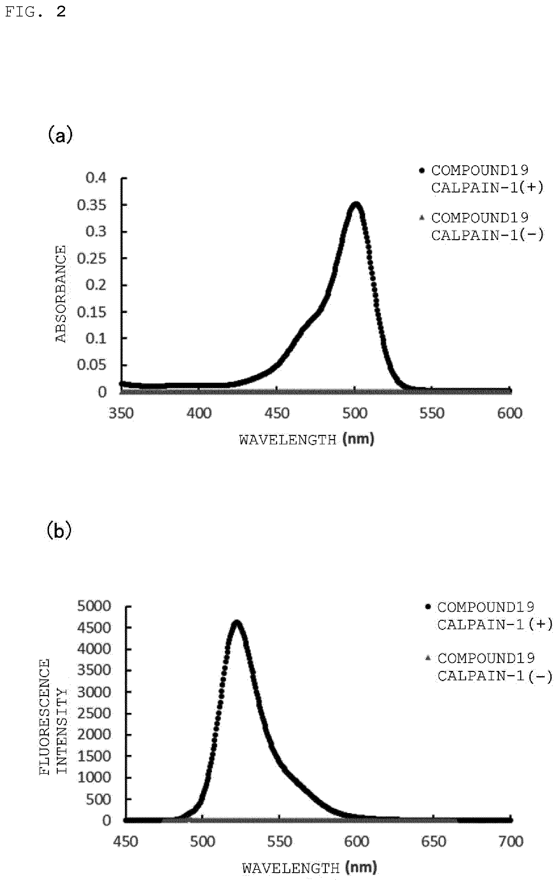

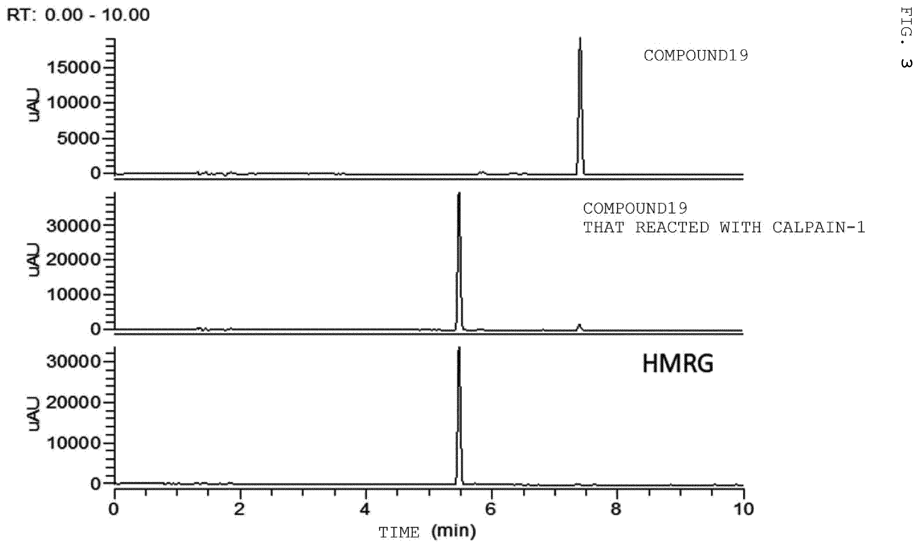

The results of the measurement of the absorption and fluorescence spectra are shown in FIG. **2(a)** and FIG. **2(b)**. Along with the addition of calpain-1, increases in absorbance and fluorescence intensity of the probe solution were observed. In addition, the results of the HPLC are shown in FIG. **3**. The peak position of Compound 19 was made identical to that of HMRG by its reaction with calpain-1. Accordingly, it was shown that the hydrolysis of the peptide moiety of Compound 19 by calpain-1 produced HMRG serving as a fluorescent substance.

### (Example 5) Change in Fluorescence Intensity with Time by Calpain-1

The fluorescence assays of Compound 19 and Compound 16 with time were performed. Specifically, 17.4 U/mL of calpain-1 and 5 mM CaCl₂ were added to a 50 mM TRIS buffer (pH: 7.4, 2 mM EGTA, 2 mM DTE) containing 10 µM of each of the synthesized fluorescent probes to perform an enzyme reaction at 37°C. Each of the compounds to which calpain-1 and CaCl₂ were not added was similarly subjected as a negative control to the measurement.

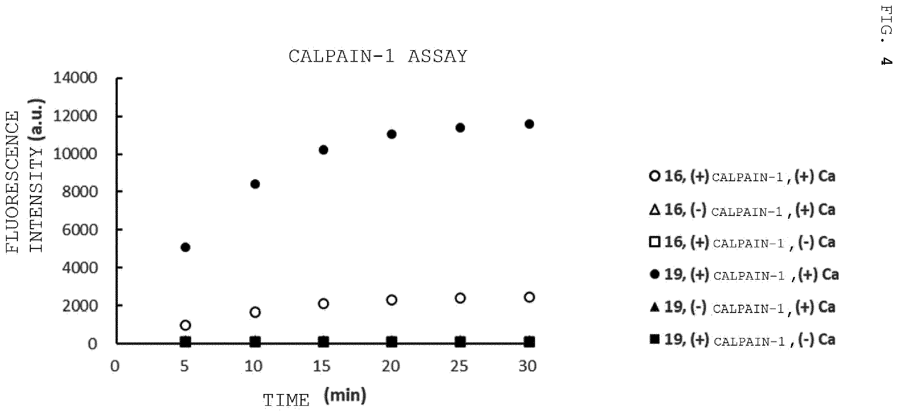

The results of the measurement of changes in fluorescence intensity with time by calpain-1 are shown in FIG. **4** (excitation wavelength: 475 nm, fluorescence wavelength: from 500 nm to 550 nm) . Each of Compound 16 and Compound 19 functioned as a fluorescent probe for calpain-1. In particular, it was shown that Compound 19 was further improved in reactivity with calpain-1. In addition, it was confirmed that no fluorescence increase occurred in a solution free of Ca²⁺ because the activation of a calpain was caused by Ca²⁺.

### (Example 6) Reactivity of each of Fluorescent Probes (Compounds 16 to 36) with Calpain

The fluorescence assays of Compounds 16 to 36 were similarly performed. 50 Units per milliliter (Compounds 16 and 19 to 25) or 17.4 U/mL (Compounds 16 to 18 and 26 to 36) of calpain-1 and 5 mM CaCl₂ were added to a 50 mM TRIS buffer (pH: 7.4, 2 mM EGTA, 2 mM DTE) containing 10 µM of each of the synthesized fluorescent probes to perform an enzyme reaction at 37°C.

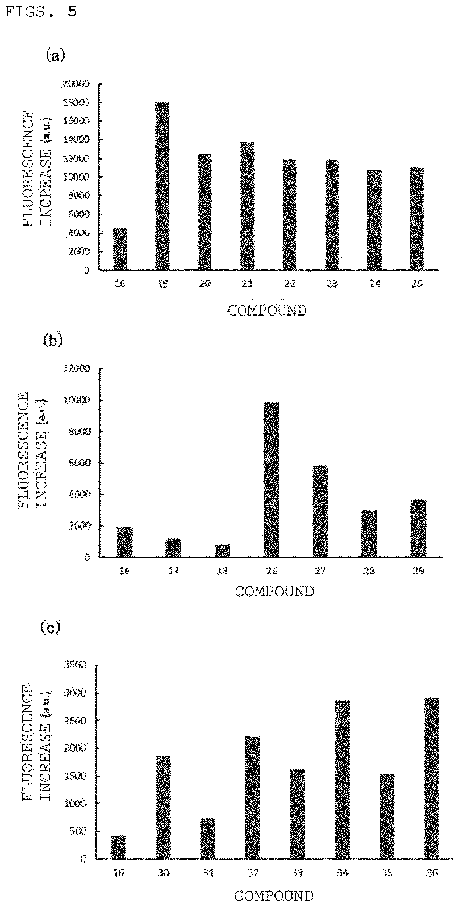

Increases in fluorescence intensity 10 minutes after the addition of calpain-1 are shown in FIG. **5(a)** and FIG. **5(b)** (excitation wavelength: 475 nm, fluorescence wavelength: from 500 nm to 550 nm). It was shown that each of the compounds functioned as a fluorescent probe for calpain-1. In addition, the fluorescence intensities of Compounds 19 to 25 were larger than that of Compound 16, and the fluorescence intensities of Compounds 26 to 29 were larger than those of Compounds 16 to 18. Increases in fluorescence intensity 30 minutes after the addition of calpain-1 are shown in FIG. **5(c)** (excitation wavelength: 460 nm, fluorescence wavelength: 530 nm). It was shown that each of the compounds functioned as a fluorescent probe for calpain-1. In addition, the fluorescence intensities of Compounds 30 to 36 were larger than that of Compound 16.

It was confirmed from the foregoing that Compounds 19 to 36 each serving as the compound of the present invention were each improved in enzymatic reactivity with a calpain as compared to Compound 16, or Compound 17 or 18 serving as a comparative compound. In addition, it was shown that a compound having an alkyl group in R¹⁰ or R¹¹, a compound in which R¹⁰ and R¹¹ formed a ring, and a compound in which R¹²-L- formed a ring with R¹⁰ in the formula (I), the compounds each serving as the compound of the present invention, each had satisfactory enzymatic reactivity with a calpain. Meanwhile, Compounds 17 and 18 serving as comparative compounds were each reduced in fluorescence intensity as compared to Compound 16. That is, it was shown that the bonding of an amide group having a R¹²-L-O-C(R¹⁰)(R¹¹)- group to the N-terminal of a peptide chain in the formula (I) was important for an improvement in sensitivity with which the enzyme activity of calpain-1 was detected. It was suggested that a R¹²-L-O-C(R¹⁰)(R¹¹)- group was not limited to a chemical structure described in this Example, and even any appropriate chemical structure similarly exhibited an improving effect on the sensitivity with which calpain activity was detected because various R¹²-L-O-C(R¹⁰)(R¹¹)- groups similarly showed sensitivity-improving effects.

### (Example 7) Changes of Fluorescent Probes (Compounds 37, 42, 45, 48, 51, 53, 55, 57, and 59) with Time by Calpain

Next, similar fluorescence assays of Compounds 37, 42, 45, 48, 51, 53, 55, 57, and 59 with calpain-1 were performed. Specifically, 17.4 U/mL of calpain-1 and 5 mM CaCl₂ were added to a 50 mM TRIS buffer (pH: 7.4, 2 mM EGTA, 2 mM DTE) containing 10 µM of each of the synthesized fluorescent probes to perform an enzyme reaction at 37°C. Each of the compounds to which calpain-1 was not added was used as a control.

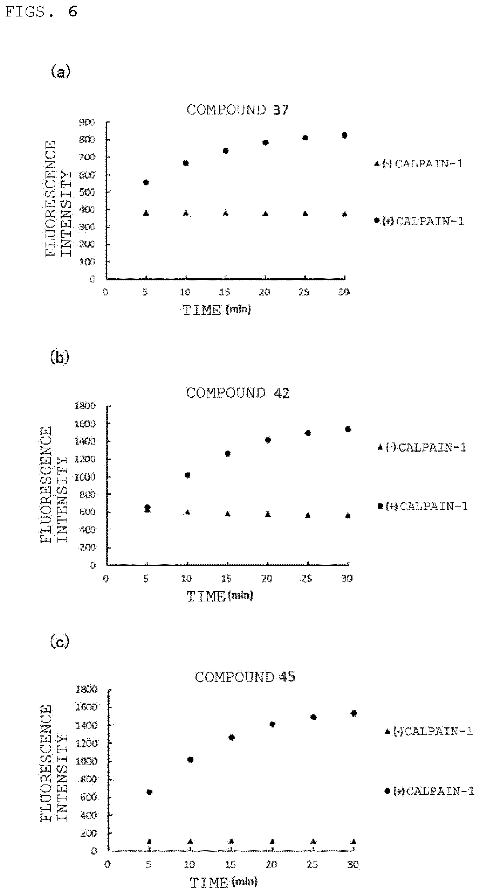

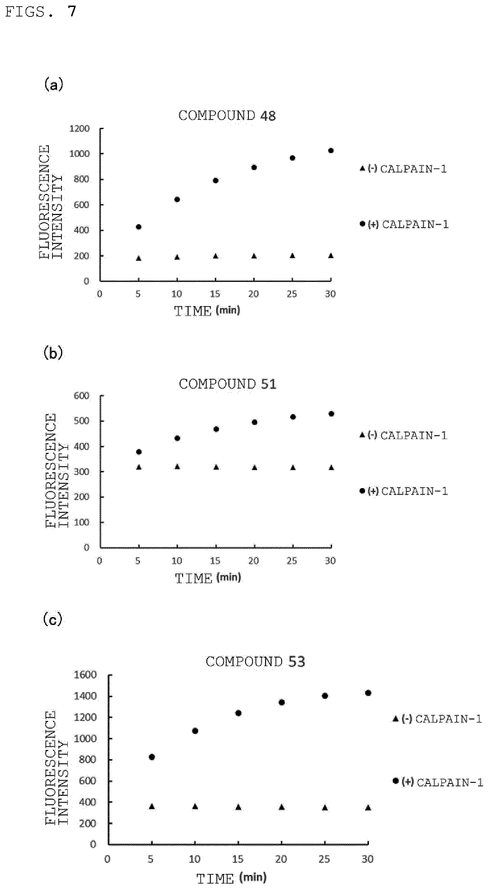

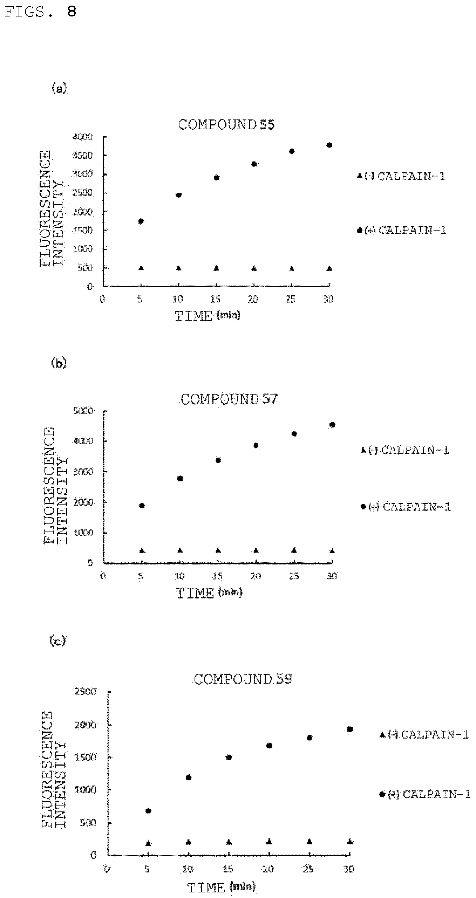

Results obtained by plotting the time dependence of fluorescence intensities are shown in FIGS. **6** to FIGS. **8** (excitation wavelength: 475 nm, fluorescence wavelength: from 500 nm to 550 nm). It was shown that each of the probes showed an increase in fluorescence intensity through its action with calpain-1, and hence served as a calpain-1 enzyme substrate.

### (Example 8) Reactivity of each of Fluorescent Probes (Compounds 37 to 66) with Calpain

To evaluate an influence of a R¹²-L-O- group on the reactivity of each of compounds having the same amino acids with calpain-1, the fluorescence assays of Compounds 37 to 66 with calpain-1 were performed in accordance with the above-mentioned method. Specifically, 17.4 U/mL of calpain-1 and 5 mM CaCl₂ were added to a 50 mM TRIS buffer (pH: 7.4, 2 mM EGTA, 2 mM DTE) containing 10 µM of each of the synthesized fluorescent probes to perform an enzyme reaction at 37°C, followed by the measurement of an increase in fluorescence intensity of each of the compounds 10 minutes after the addition of calpain-1.

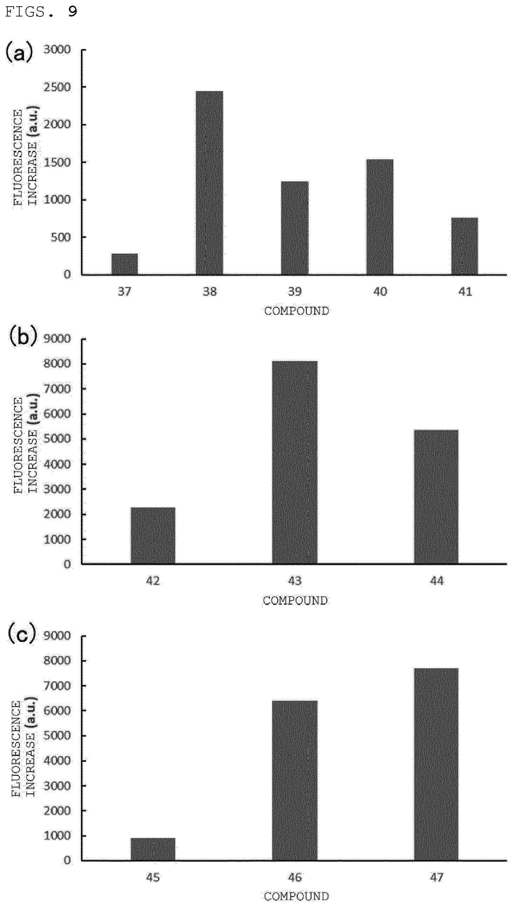

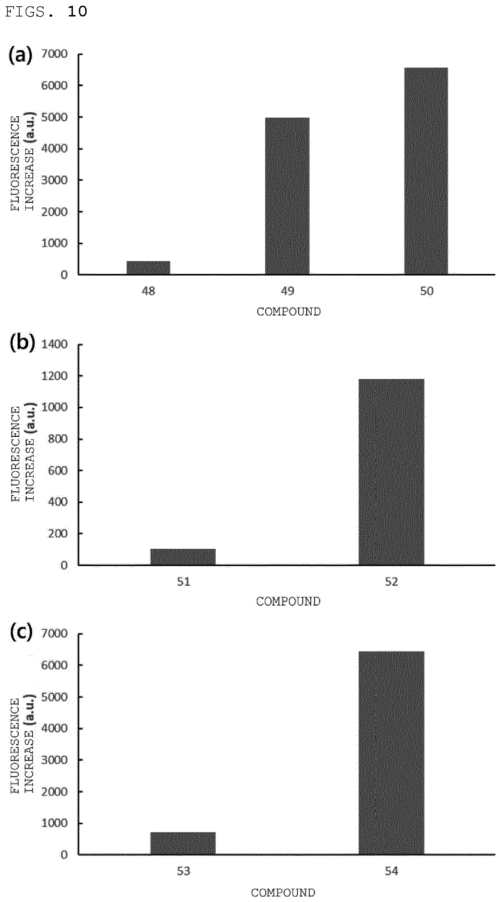

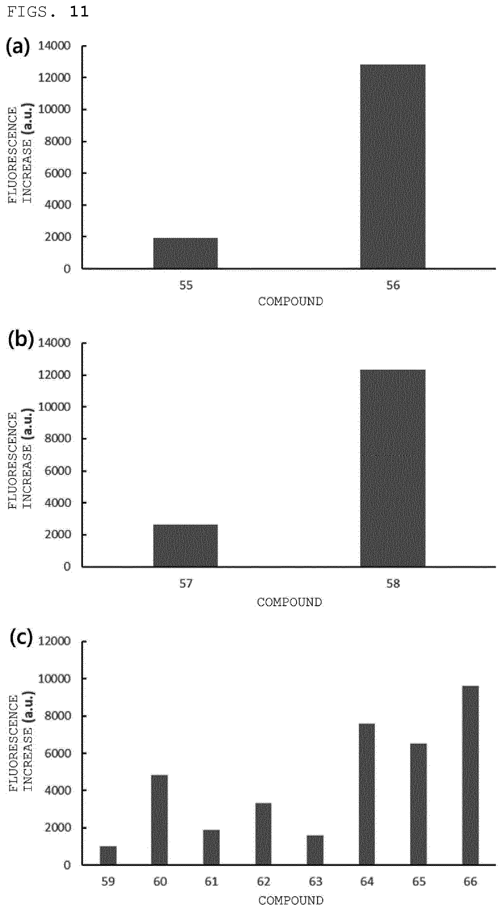

The obtained results are shown in FIGS. **9** to FIGS. **11****.** It was confirmed that in each of the compounds having a R¹²-L-O- group was more increased in fluorescence intensity than a compound free of any R¹²-L-O- group was. It was shown that a compound having a R¹²-L-O- group was able to effectively detect calpain activity in each of various combinations of its amino acids A and B.

The reactivity of each of Compounds 16 to 66 with calpain-1 depended on a R¹²-L-O-C(R¹⁰) (R¹¹)- group in the formula (I), and an improvement in reactivity thereof with calpain-1 was observed when a R¹²-L-O-C(R¹⁰)(R¹¹)- group was present. Further, the presence of a R¹²-L-O-C(R¹⁰) (R¹¹)- group showed an improving effect on reactivity with a calpain in each of the various combinations of the amino acids A and B.

### (Example 9) Live Cell Imaging with each of Fluorescent Probes (Compound 16 and Compound 19)

Whether or not the improvement in enzymatic reactivity between the compound of the present invention and a calpain confirmed by the above-mentioned experiment remained the same even in a cell was verified. Calpain activity imaging in SH-SY5Y cells was performed on each of Compounds 16 and 19. Specifically, the SH-SY5Y cells (American Type Culture Collection, VA, USA) were cultured in a mixed medium of Dulbecco's modified eagle medium and Ham's F-12 medium (DMEM/F-12; Thermo Fisher Scientific, Inc.) (containing 10% fetal bovine serum (FBS; Thermo Fisher Scientific, Inc., MA, USA) and 100 U/mL penicillin-100 ug/mL streptomycin (PS; Thermo Fisher Scientific, Inc.)) under the conditions of 37°C and 5% CO₂-95% air. 6×10⁴ to 8×10⁴ Cells/well of the cells were seeded in a multiwell plate (µ-Plate 96 Well; ibidi, Grafelfing, Germany) coated with poly-D-lysine (Thermo Fisher Scientific, Inc.) and laminin (BD Biosciences, CA, USA), and were preincubated in a culture solution. Immediately after the removal of the culture solution in the wells, 200 µL of an assay solution (a culture solution containing a 5 µM probe) was added thereto, and the cells were incubated under the conditions of 37°C and 5% CO₂-95% air. 10 Minutes after that, a bright field image and a fluorescence image were taken with a confocal microscope (LSM 710, Carl Zeiss AG, Oberkochen, Germany).

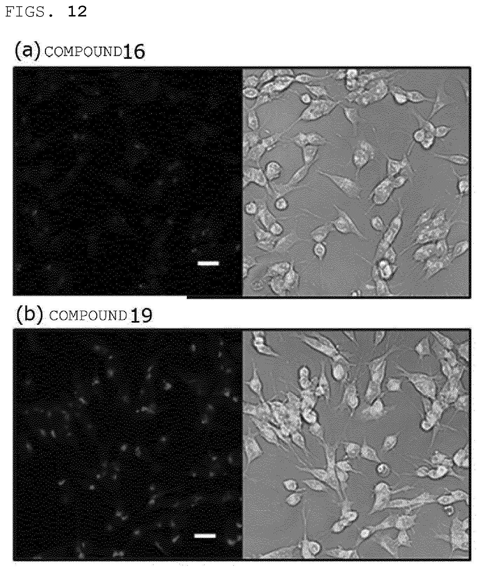

As shown in FIG. **12(a)** and FIG. **12(b)**, in each case, the addition of the compound enabled the observation of calpain activity in the SH-SY5Y cells. Clear imaging was able to be performed in Compound 19 as compared to Compound 16, and hence it was shown that an improvement in enzymatic reactivity by a R¹²-L-O-C(R¹⁰)(R¹¹)- group in the formula (I) was also effective in live cell imaging.

### (Example 10) Live Cell Imaging with Fluorescent Probe

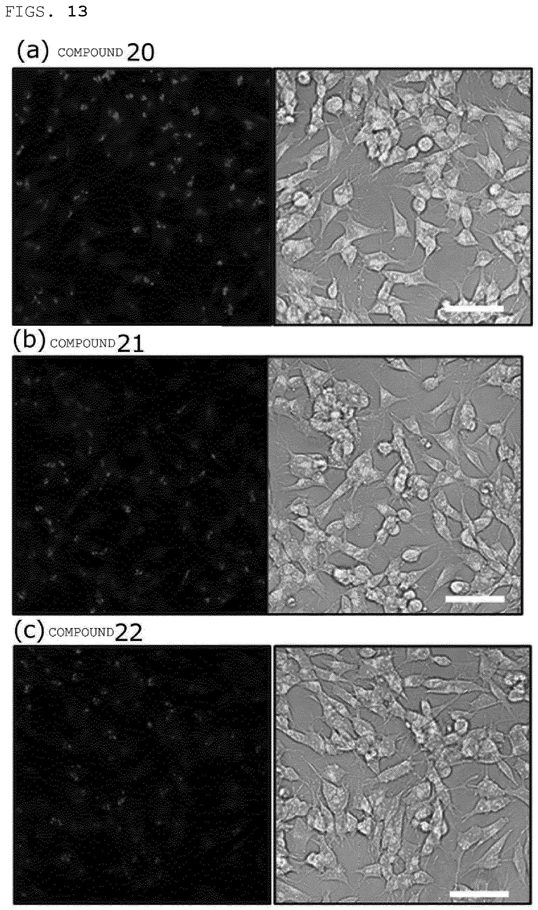

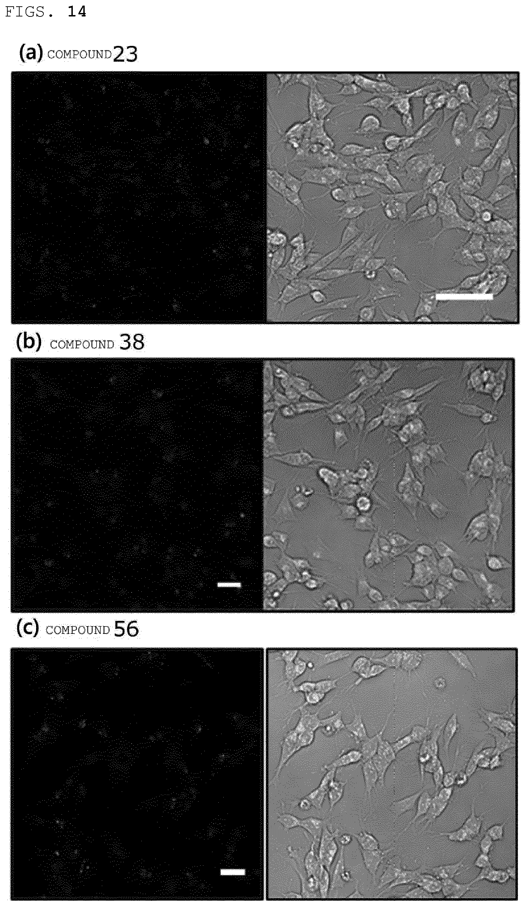

Calpain activity imaging in SH-SY5Y cells was performed on each of Compounds 20 to 23, Compound 38, and Compound 56 in the same manner as in Example 9. In the case of each of Compounds 20 to 23 and Compound 38, a bright field image and a fluorescence image were taken after 30 minutes of incubation of the cells, and in the case of Compound 56, such images were taken after 20 minutes of incubation thereof. As shown in FIGS. **13** and FIGS. **14**, in each of the compounds, calpain activity in the SH-SY5Y cells was able to be observed.

As demonstrated in Examples described above, the present invention can provide a novel fluorescent probe targeted at a calpain. Specifically, the inventors have found that the presence of a R¹²-L-O-C(R¹⁰)(R¹¹)- group in the formula (I) enables the creation of a probe improved in enzymatic reactivity with a calpain. Such improvement was confirmed in a wide variety of groups each serving as a R¹²-L-O-C(R¹⁰)(R¹¹)- group. For example, even when the group had a substituent, such as a hydroxyl group, a carboxyl group, an alkoxy group, and/or an amino group, the probe showed satisfactory enzymatic reactivity. In addition, even when R¹² and R¹⁰ or R¹¹ were bonded to form a ring, when R¹⁰ and R¹¹ each represented an alkyl group, or when R¹⁰ and R¹¹ formed a ring, the enzymatic reactivity with a calpain was satisfactory. Further, even when the amino acids A and B were various amino acids, the enzymatic reactivity with a calpain provided a satisfactory result. Further, even in the fluorescence imaging of cells, fluorescence intensity enhancement depending on a R¹²-L-O-C(R¹⁰)(R¹¹)- group was observed. The foregoing results showed that the presence of a R¹²-L-O-C(R¹⁰)(R¹¹)- group in the formula (I) enabled a compound of the formula (I) having any appropriate amino acids A and B to effectively detect calpain activity. That is, it is demonstrated that the fluorescent probe of the present invention is a compound that can effectively detect the enzyme activity of a calpain.

### (Example 11) Evaluation with Rat NMDA Injury Model

Black Brown-Norway rats were each subjected to general anesthesia with isoflurane. PBS or N-methyl-D-aspartate (NMDA) (10 mM, 2 µL) was administered into an eye of the rat. 6 Hours after that, the rat was anesthetized with a mixed liquid of ketamine and xylazine again, and Compound 19 (18.3 µM, 3 µL) was administered into the eye. Images of an ocular fundus were taken with F-10 (a confocal biomicroscope manufactured by Nidek Co., Ltd.) before the administration of Compound 19 and 20 minutes after the administration.

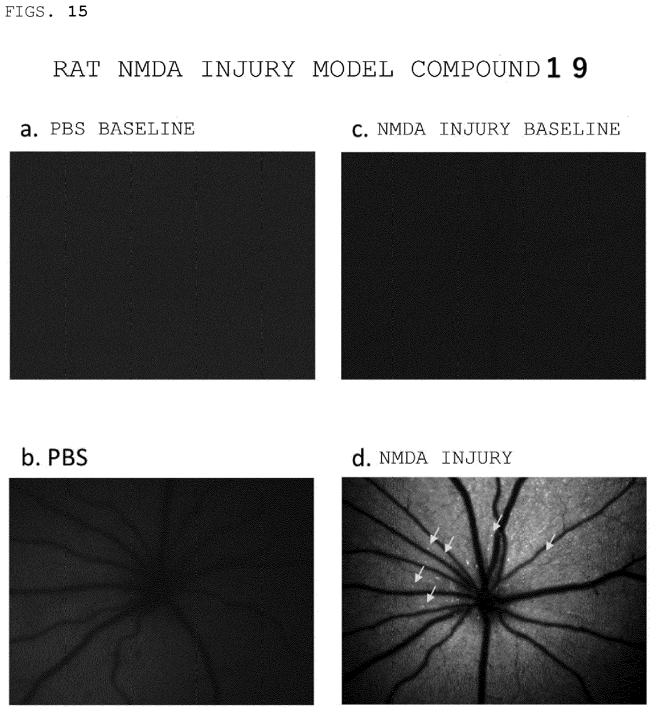

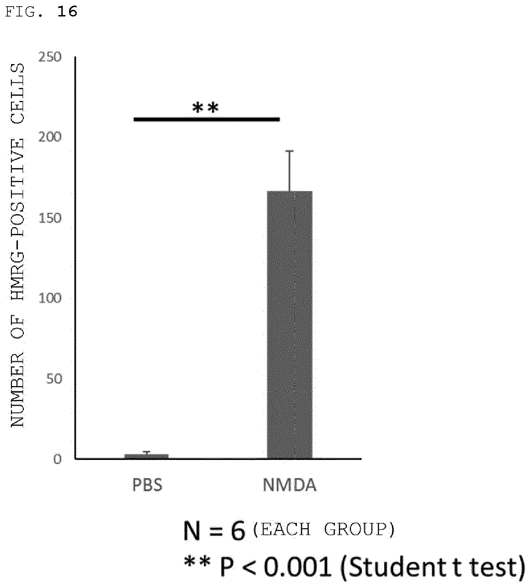

The results are shown in FIGS. **15**. Sites indicated by the arrows represent examples of nerve cells stained with HMRG. In each of the rats each having administered thereto the PBS, no signal is observed before the administration of Compound 19 (FIG. **15a****)**, and substantially no nerve cells are stained 20 minutes thereafter (FIG. **15b****)**. In each of the rats each having administered thereto NMDA, as shown in FIG. **15c**, no clear signal is observed before the administration of Compound 19. However, as shown in FIG. 15d, many of the nerve cells were clearly stained 20 minutes after the administration of Compound 19. In addition, results obtained by counting the numbers of HMRG-positive nerve cells in the photographed images of the ocular fundi of the rats shown in FIGS. **15** are shown in FIG. **16**. The number of the HMRG-positive cells in the rats each having administered thereto the PBS was extremely small, and the number of the HMRG-positive cells in the rats each having administered thereto NMDA showed a significant increase.

The fluorescent probe of the present invention may be useful in the diagnosis of a retinal disease because a rat NMDA retinal injury model is considered to be a retinal disease model. In particular, the probe can rapidly detect the activation of a calpain in an ocular fundus, and is hence expected to be useful in clinical diagnosis or treatment selection. Further, the fluorescent probe of the present invention is also expected to be useful in the evaluation of a therapeutic effect exhibited by a calpain inhibitor. Specifically, for example, the probe can determine the therapeutic effect of treatment performed on a mammal subject (e.g., a patient), in which a calpain activity increase has been observed, with the calpain inhibitor. Thus, the utility value and economic effect of the fluorescent probe of the present invention in medical and industrial uses are extremely large.

### (Example 12) Evaluation of Compound 19 with Rabbit NMDA Injury Model

Black Dutch rabbits were each subjected to general anesthesia with a mixed liquid of ketamine and xylazine. N-Methyl-D-aspartate (NMDA) (12 mM, 50 µL) or PBS was administered into an eye of the rabbit. 9 Hours after that, the rabbit was anesthetized with a mixed liquid of ketamine and xylazine again, and Compound 19 (18.3 µM, 19 µL), Compound 16 (20 µM, 19 µl), or Compound 16 (20 µM, 38 ul) was administered into the eye. Images of an ocular fundus were taken with Mirante (a confocal scanning diode laser ophthalmoscope manufactured by Nidek Co., Ltd.) before the administration of each of the compounds and after the lapse of a predetermined time from the administration.

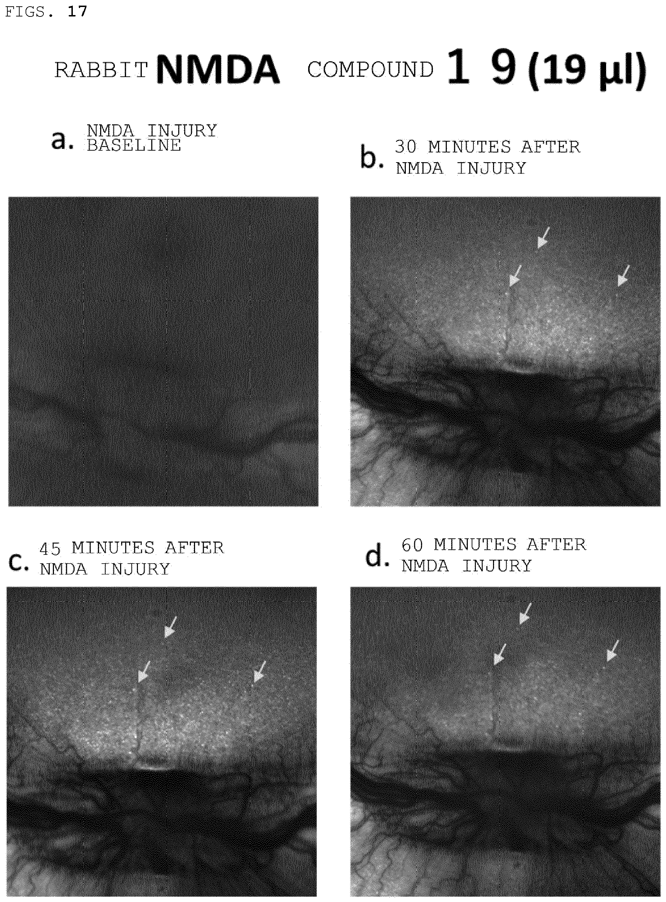

The results are shown in FIGS. **17** to FIGS. **22**. Sites indicated by the arrows represent examples of nerve cells stained with HMRG. As shown in FIG. **17a**, no clear signal is observed before the administration of Compound 19. However, as shown in FIG. **17b** to FIG. **17d**, in each of the rabbits each having administered thereto NMDA, many nerve cells were clearly stained with HMRG 30 minutes, 45 minutes, and 60 minutes after the administration of Compound 19.

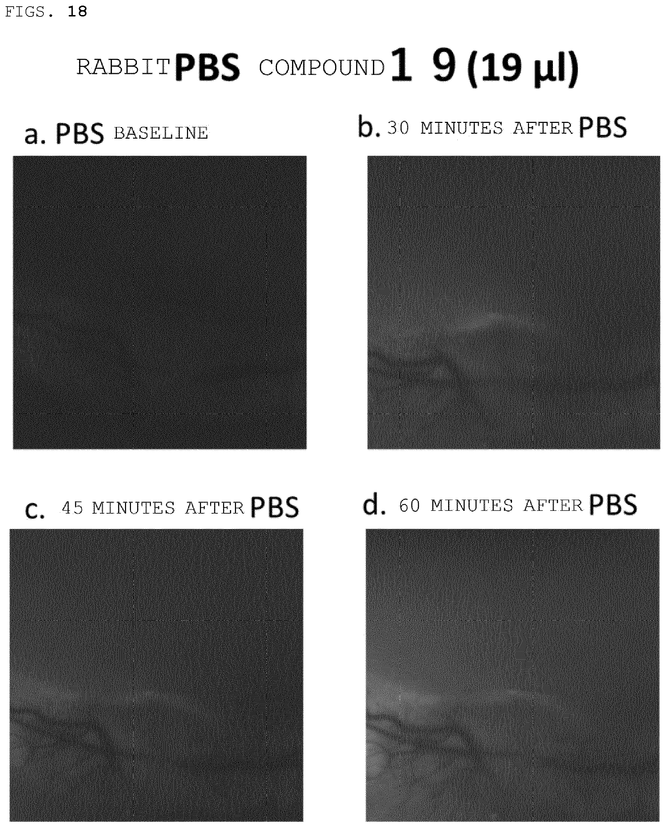

The results of the PBS-administered eye are shown in FIGS. **18**. As shown in FIG. **18a**, no clear signal is observed before the administration of Compound 19. In addition, as shown in FIG. **18b** to FIG. **18d**, substantially no staining of the nerve cells with HMRG is observed even 30 minutes, 45 minutes, and 60 minutes after the administration of Compound 19.

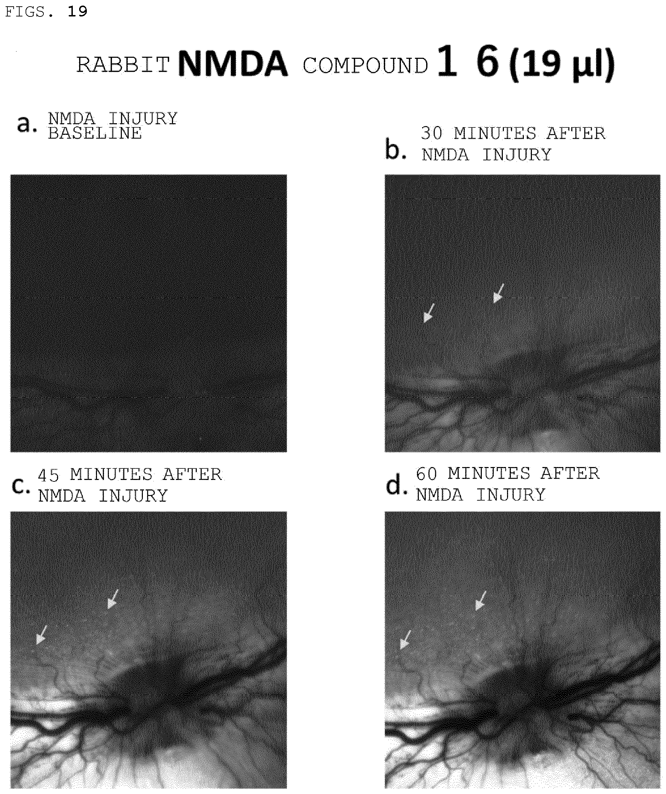

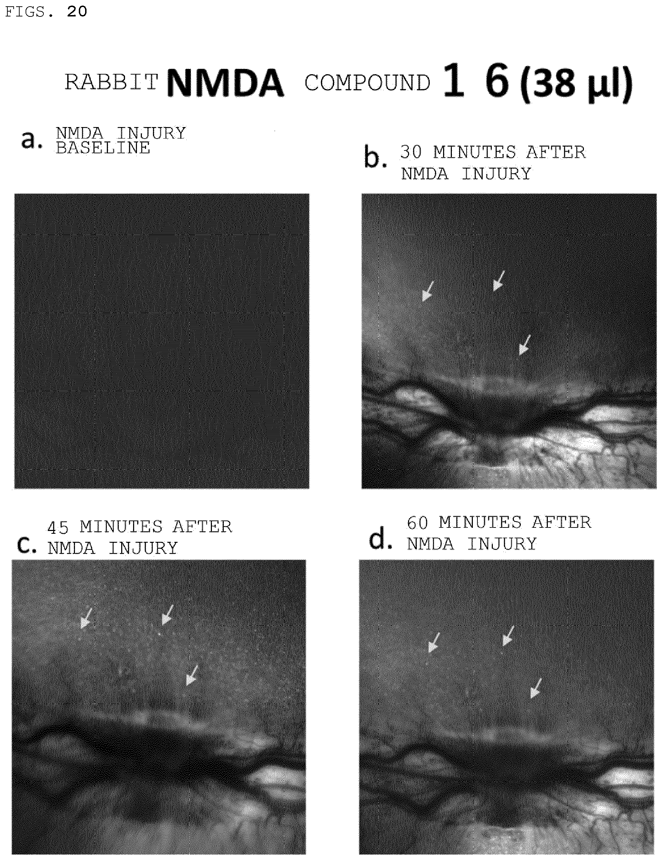

As shown in each of FIG. **19a** and FIG. **20a**, no clear signal is observed before the administration of Compound 16 (19 µl or 38 µl). However, as shown in FIG. **19b** to FIG. **19d** or FIG. **20b** to FIG. **20d**, in each of the rabbits each having administered thereto NMDA, many nerve cells were clearly stained with HMRG 30 minutes, 45 minutes, and 60 minutes after the administration of Compound 16 (19 µl or 38 µl).

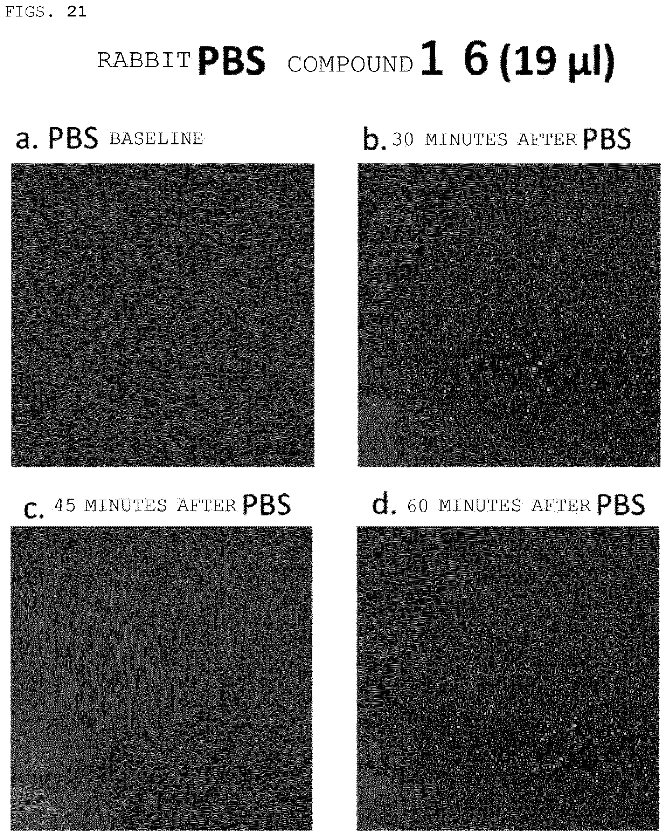

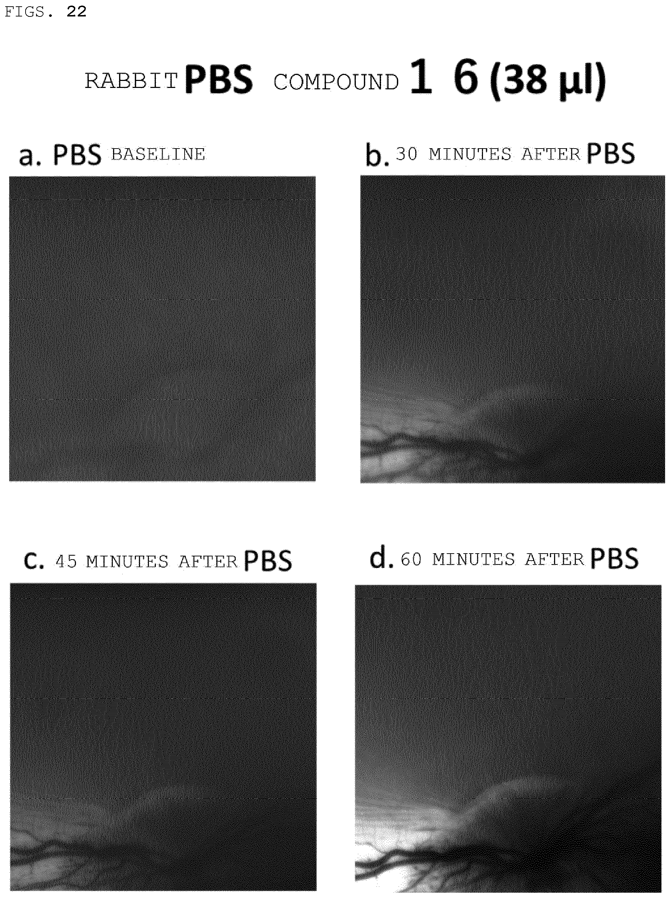

Meanwhile, in each of the rabbits each having administered thereto the PBS, as shown in FIG. **21a** or FIG. **22a**, no signal is observed in each group before the administration of Compound 16 (19 µl or 38 µl). In addition, as shown in FIG. **21b** to FIG. **21d** or FIG. **22b** to FIG. **22d**, substantially no staining of the nerve cells with HMRG is observed even 30 minutes, 45 minutes, and 60 minutes thereafter.

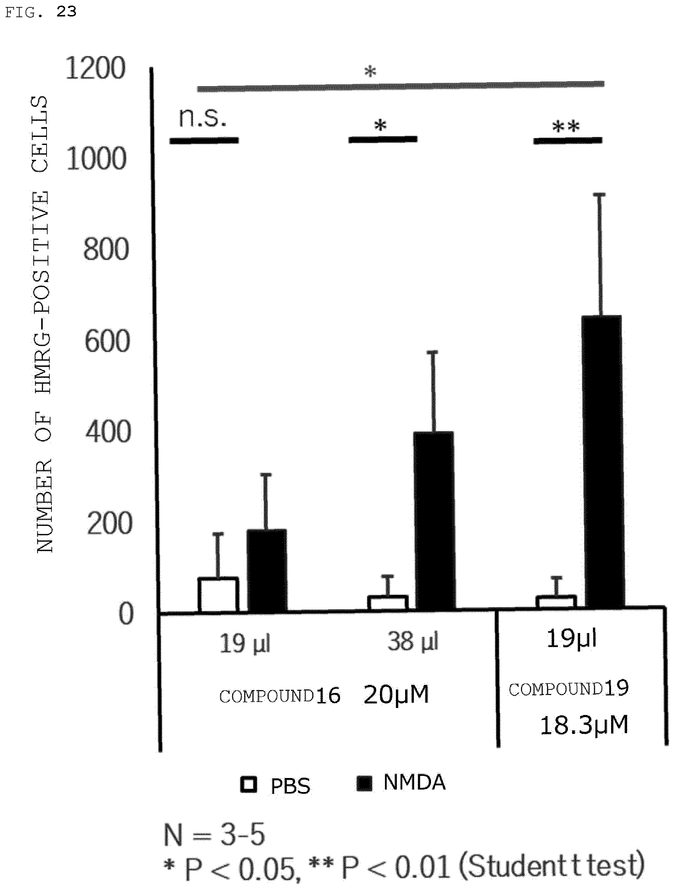

Results obtained by counting the numbers of HMRG-positive nerve cells in certain regions (each measuring 819 pixels by 819 pixels) superior optic papillae in the photographed images of the ocular fundi having administered thereto Compound 19 and Compound 16 (19 µl and 38 ul) shown in FIGS. **17** to FIGS. **22** are shown in FIG. **23**. In the rabbits each having administered thereto NMDA, the number of the HMRG-positive cells in the rabbits each having administered thereto Compound 19 significantly increased as compared to that in the rabbits each having administered thereto Compound 16 (19 µl), and tended to increase as compared to that in the rabbits each having administered thereto Compound 16 (38 µl). In the rabbits each having administered thereto the PBS, the number of the HMRG-positive cells was extremely small, and the numbers of the HMRG-positive cells in the rabbits having administered thereto Compound 19 and Compound 16 (38 ul) significantly reduced as compared to the numbers of the HMRG positive cells in the rabbits each having administered thereto NMDA.

The fluorescent probe of the present invention may be useful in the diagnosis of a retinal disease because a rabbit NMDA injury model is considered to be a retinal disease model. The eyeball size of a rabbit that is a medium-sized animal is more similar to the eyeball size of a human than to the eyeball size of a rat that is a small animal, and hence the conditions under which the probe is administered to the rabbit may be similar to the conditions under which the probe is administered to the human. Further, Compound 19 provides satisfactory reactivity even when used in a dose smaller than that of Compound 16. Accordingly, the fluorescent probe of the present invention may be useful particularly in administration into an eye of a human.

The foregoing results demonstrate that the use of the compound of the present invention can detect calpain activity in an ocular fundus, and hence the compound is useful as a diagnostic agent for calpain activity-derived glaucoma.

### (Example 13) Evaluation of Calpain Probe with Human iPS Cell-derived Stereoscopic Retinal Tissue

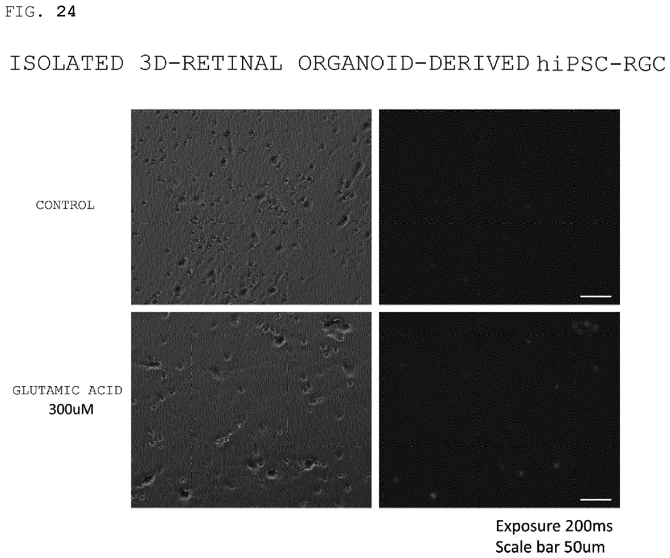

Human iPS cells (201B7 line, purchased from CiRA) were artificially induced to differentiate to a three-dimensional stereoscopic retinal tissue by a previously reported method with minor modifications, and the three-dimensional stereoscopic retinal tissue was maintained and cultured in a 5% CO₂ incubator at 37°C for 60 days. A glutamic acid injury model that activated a calpain to induce a retinal ganglion cell injury was produced from the iPS cell-derived three-dimensional stereoscopic retinal tissue, and calpain activity with Compound 19 was evaluated by using the model. First, to identify a retinal ganglion cell injury caused by a glutamic acid injury, retinal ganglion cells were isolated from the iPS cell-derived three-dimensional stereoscopic retinal tissue with auto MACS pro (trademark) and CD90 MicroBeads. Glutamic acid (final concentration: 300 µM) was added to the cells to be caused to react therewith for 1 hour. Compound 19 (final concentration: 5 µM) was added thereto 1 hour after the addition of glutamic acid, and images of the state and fluorescence of the cells were taken (RGB and B/W modes) with a fluorescence microscope (ZEISS Axio Vert.A1) 1 hour thereafter. As a result, the attenuation of a neural spine and the enhancement of the fluorescence were observed, and hence it was confirmed that the retinal ganglion cell injury was induced by the calpain activation (FIG. **24****)**.

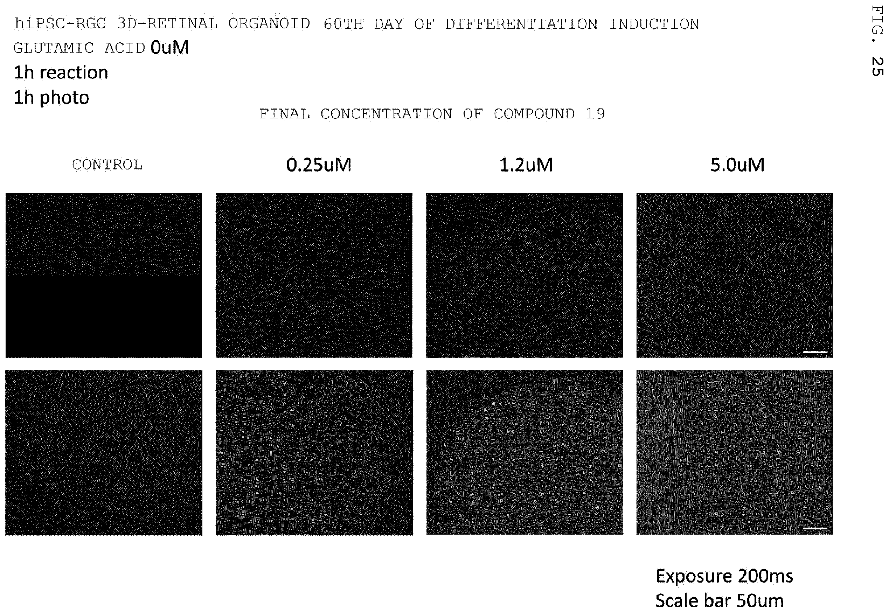

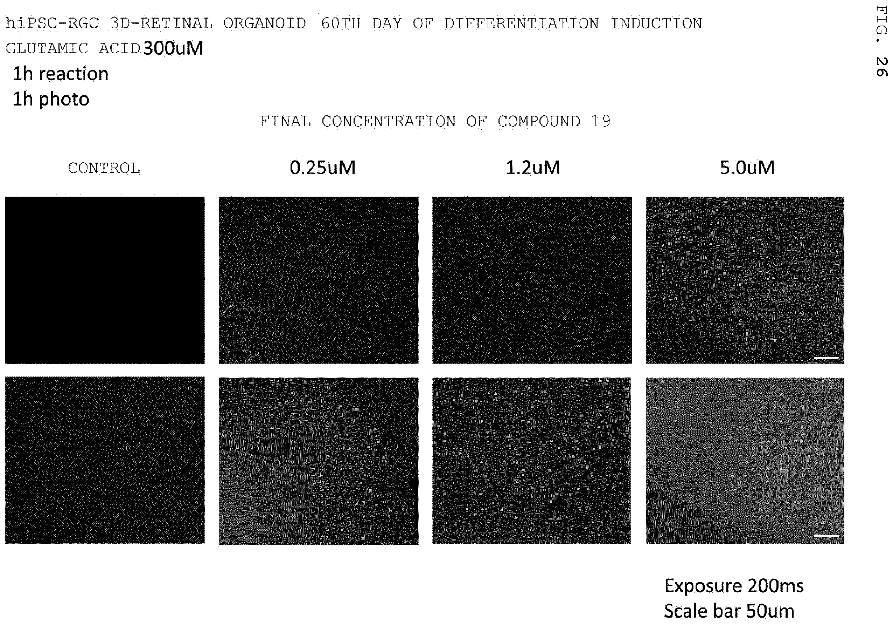

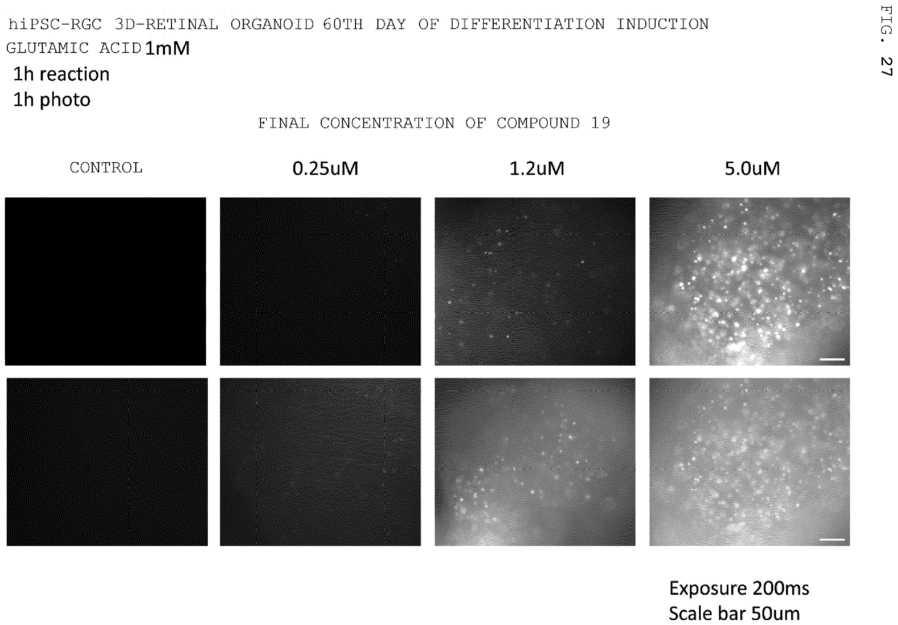

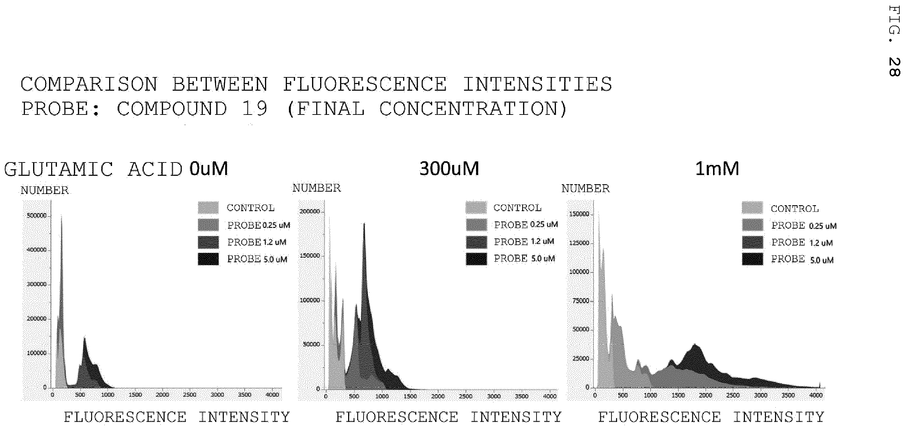

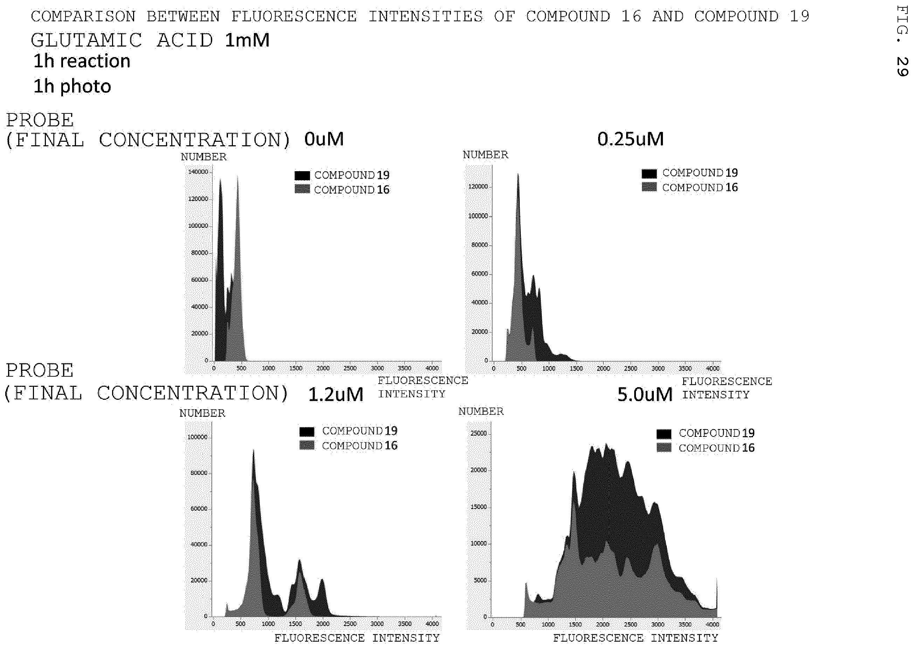

Next, to confirm a reaction under conditions closer to those of a living organism, the iPS cell-derived three-dimensional stereoscopic retinal tissue on the 60th day of the differentiation induction was left at rest in a 24-well plate containing 1 mL of a reaction medium (obtained by adding CaCl₂ (final concentration: 2.4 mM) and HEPES (final concentration: 20 mM) to a HBSS solution) in each well (n=5). The tissue was washed with 1 mL of the reaction medium twice, and glutamic acid was added thereto so that its final concentration became 0 µM, 300 µM, or 1 mM, followed by a reaction for 1 hour. Compound 19 was added to the resultant 1 hour after the addition of glutamic acid so that its final concentration became 0 µM, 0.25 µM, 1.2 µM, or 5.0 µM, and an image of the fluorescence of the mixture was taken with a fluorescence microscope (ZEISS Axio Vert.A1) (a RGB mode (lower stage) and a B/W mode (upper stage)) 1 hour thereafter. In all image-taking operations, an exposure time was fixed to 200 ms. The results of groups in which the final concentrations of glutamic acid are 0 µM, 300 µM, and 1 mM are shown in FIG. **25**, FIG. **26**, and FIG. **27**, respectively. In the group in which the final concentration of glutamic acid was 300 µM or 1 mM, the enhancement of fluorescence expression was observed in proportion to the concentration of Compound 19. In addition, a graphical representation of an average calculated from fluorescence intensities in the images taken in the B/W mode (n=3) is shown in FIG. **28**. It was confirmed from the results that the fluorescence was strongly observed in a manner dependent on the concentration of Compound 19 under such a condition that the glutamic acid concentrations were the same, and the fluorescence was strongly expressed in accordance with the concentration of glutamic acid. In addition, results obtained by comparing and examining the fluorescence intensities of Compound 19 and Compound 16 are shown in FIG. **29**. When each of the compounds was added under such a condition that the final concentration of glutamic acid was 1 mM, Compound 19 was observed to express stronger fluorescence than that of Compound 16 at a final concentration of 0.25 µM, 1.2 µM, or 5.0 µM.

It is conceivable from the foregoing that Compound 19 is useful in evaluating a human iPS cell-derived retinal ganglion cell injury caused by the activation of calpain. Compound 19 is expected to be useful in the evaluation of retinal ganglion cells injury by the activation of a calpain in the retinae of a human living organism because it was confirmed that Compound 19 showed reactivity in retinal ganglion cells and a stereoscopic retinal tissue derived from human iPS cells.

As described above, the fluorescent probe serving as one aspect of the present invention may be used in combination with a known or novel calpain inhibitor. For example, combined use in the following mode is permitted: a mammal subject treatable or preventable with the calpain inhibitor is selected with the fluorescent probe, and then the calpain inhibitor is administered to the mammal subject. Alternatively, for example, combined use in the following mode is permitted: the fluorescent probe is administered to a mammal subject having administered thereto the calpain inhibitor to determine the degree of a therapeutic or preventive effect exhibited by the calpain inhibitor. The fluorescent probe serving as one aspect of the present invention may be administered to a mammal subject before the administration of the calpain inhibitor to the mammal subject, after the administration, before or after the administration, or simultaneously with the administration to enable the evaluation of, for example, its calpain activity (in other words, the effect of the calpain inhibitor). In the subsequent group of Examples, an action exhibited by the combined use of the fluorescent probe and the calpain inhibitor is specifically described.

### (Example 14) Combined Use with Calpain Inhibitor in Live Cell Imaging

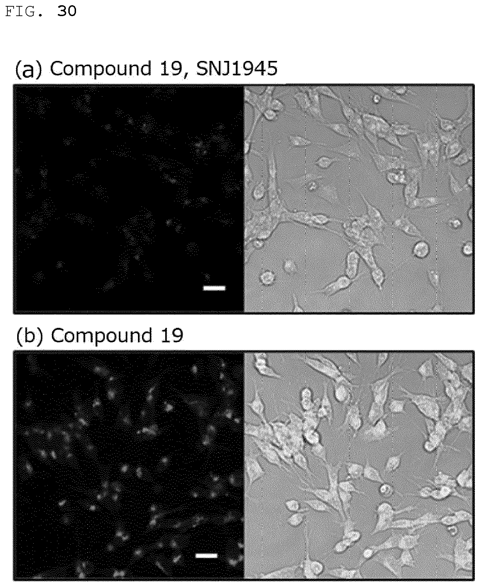

The results of live cell imaging performed in the same manner as in Example 9 except the following are shown in FIGS. **30****:** Compound 19 was used as a fluorescent probe; and the assay solution further contained SNJ-1945 serving as a known calpain inhibitor at a concentration of 10 µM. In FIGS. **30**, FIG. **30(a)** is an image obtained by imaging with the inhibitor-containing assay solution, and FIG. **30(b)** is an image obtained by imaging with the inhibitor-free assay solution. As shown in FIGS. **30**, in the case where the inhibitor-containing assay solution is used (FIG. **30(a)**), an intracellular fluorescence intensity attenuates as compared to that in the case where the inhibitor-free assay solution is used (FIG. **30(b)**). As described above, the fluorescent probe according to the present invention can be used in combination with the calpain inhibitor for determining the pharmacological action of the calpain inhibitor on a live cell.

### (Example 15) Combined Use 1 with Calpain Inhibitor in Mammal Subject

Carboxymethylcellulose sodium was dissolved in distilled water to prepare a 0.5% (w/v) CMC solution. An inhibitor-containing CMC solution was prepared by suspending SNJ-1945 in the 0.5% (w/v) CMC solution so that its concentration became 2.5% (w/v). Rats were each subjected to general anesthesia with isoflurane, and NMDA (10 mM, 2 µL) was administered into an eye of the rat. 2 Hours after the administration of NMDA, the inhibitor-containing CMC solution was intraperitoneally administered to the rat so that the dose of SNJ-1945 became 100 mg/kg body weight. 4 Hours after the administration of the inhibitor (6 hours after the administration of NMDA), the rat was anesthetized with isoflurane again, and Compound 19 (15 µM, 4 µL) was administered into the eye. Images of an ocular fundus were taken with F-10 20 minutes to 30 minutes after the administration of Compound 19. In a control group (N=8), the same operations as those in an SNJ-1945-administered group (N=8) except the following were performed: the 0.5% (w/v) CMC solution was administered instead of the inhibitor-containing CMC solution.

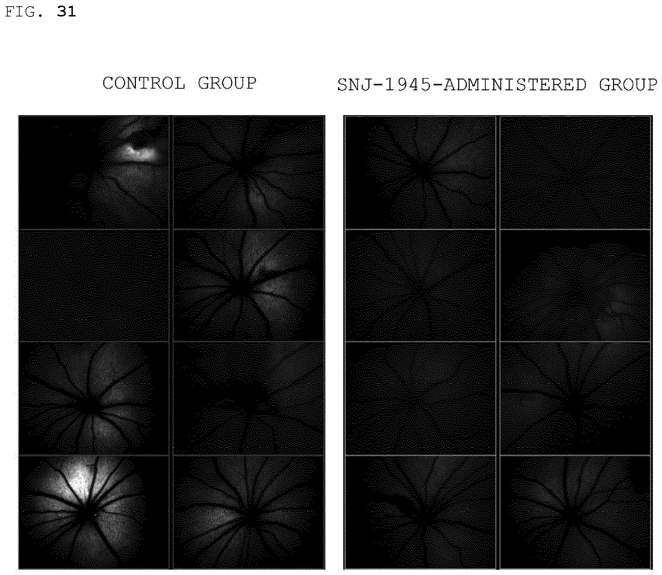

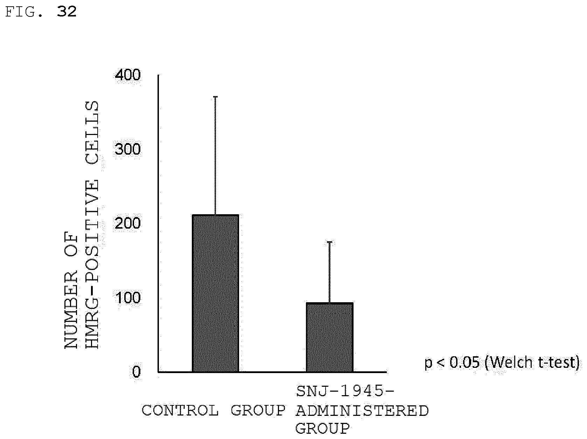

The results are shown in FIG. **31**. As shown in FIG. **31**, in the control group, a larger number of nerve cells than those in the SNJ-1945-administered group are stained. Results obtained by counting the numbers of HMRG-positive nerve cells in the photographed images of the ocular fundi of the rats shown in FIG. **31** are shown in FIG. **32**. As shown in FIG. **32**, in the SNJ-1945-administered group, the number of the HMRG-positive nerve cells significantly reduced as compared to that in the control group.

### (Example 16) Combined Use 2 with Calpain Inhibitor in Mammal Subject

Carboxymethylcellulose sodium was dissolved in distilled water to prepare a 0.5% (w/v) CMC solution. An inhibitor-containing CMC solution was prepared by suspending SNJ-1945 in the 0.5% (w/v) CMC solution so that its concentration became 2.5% (w/v). Rats were each subjected to general anesthesia with isoflurane, and NMDA (10 mM, 2 µL) was administered into an eye of the rat. 2 Hours after the administration of NMDA, the inhibitor-containing CMC solution was orally administered to the rat so that the dose of SNJ-1945 became 100 mg/kg body weight. 4 Hours after the administration of the inhibitor (6 hours after the administration of NMDA), the rat was anesthetized with isoflurane again, and Compound 19 (15 µM, 4 µL) was administered into the eye. Images of an ocular fundus were taken with F-10 20 minutes to 30 minutes after the administration of Compound 19. In a control group (N=7), the same operations as those in an SNJ-1945-administered group (N=8) except the following were performed: the 0.5% (w/v) CMC solution was administered instead of the inhibitor-containing CMC solution.

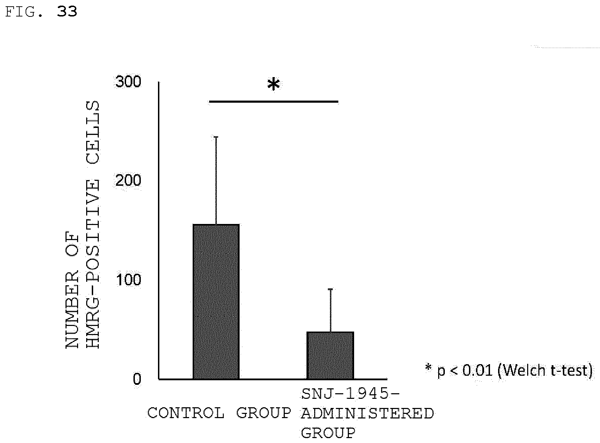

Results obtained by counting the numbers of HMRG-positive nerve cells in the photographed images of the ocular fundi are shown in FIG. **33**. As shown in FIG. **33**, in the SNJ-1945-administered group, the number of the HMRG-positive nerve cells significantly reduced as compared to that in the control group.

### (Example 17) Combined Use 3 with Calpain Inhibitor in Mammal Subject

Carboxymethylcellulose sodium was dissolved in distilled water to prepare a 0.5% (w/v) CMC solution. An inhibitor-containing CMC solution was prepared by suspending SNJ-1945 in the 0.5% (w/v) CMC solution so that its concentration became 2.5% (w/v). The inhibitor-containing CMC solution was intraperitoneally administered to each of rats so that the dose of SNJ-1945 became 100 mg/kg body weight. 2 Hours after the intraperitoneal administration, the rat was subjected to general anesthesia with isoflurane, and NMDA (10 mM, 2 µL) was administered into an eye thereof. 6 Hours after the administration of NMDA, the rat was anesthetized with isoflurane again, and Compound 19 (15 µM, 4 µL) was administered into the eye. Images of an ocular fundus were taken with F-10 20 minutes to 30 minutes after the administration of Compound 19. In a control group (N=7), the same operations as those in an SNJ-1945-administered group (N=8) except the following were performed: the 0.5% (w/v) CMC solution was administered instead of the inhibitor-containing CMC solution.

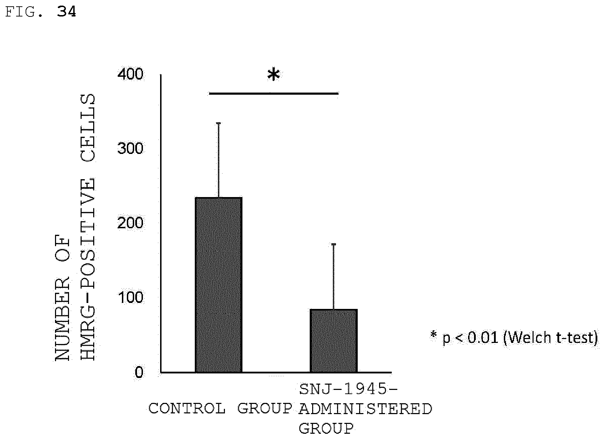

Results obtained by counting the numbers of HMRG-positive nerve cells in the photographed images of the ocular fundi are shown in FIG. **34**. As shown in FIG. **34**, in the SNJ-1945-administered group, the number of the HMRG-positive nerve cells significantly reduced as compared to that in the control group.

As described in Examples 15 to 17, the fluorescent probe according to the present invention can be used in combination with a calpain inhibitor for determining the degree of the therapeutic or preventive effect of the calpain inhibitor in a mammal subject. Specifically, Examples 15 and 16 each correspond to the following aspect: the calpain inhibitor is administered to a mammal subject in which a calpain is activated (consequently, which may have sensitivity to a calpain inhibitor), and the degree of its effect is evaluated with the fluorescent probe according to the present invention. In addition, Example 17 corresponds to the following aspect: the degree of the effect of the calpain inhibitor when the activation of a calpain is induced in a mammal subject to which the calpain inhibitor has been preventively administered is evaluated with the fluorescent probe according to the present invention.

### (Example 18) Combined Use 4 with Calpain Inhibitor in Mammal Subject

Results obtained by the imaging of nerve cells when SNJ-1945 (CAS 854402-59-8), C2I (CAS 677275-28-4), and MDL 28,170 (CAS 88191-84-8) serving as covalent inhibitors, and PD-151746 (CAS 179461-52-0) serving as a noncovalent inhibitor were used in combination as calpain inhibitors were compared to each other. Specifically, the imaging of the nerve cells of rats was performed in the same manner as in Example 16 except that an inhibitor-containing CMC solution prepared as follows was used: each calpain inhibitor was suspended in the 0.5% (w/v) CMC solution so that its concentration became 2.5% (w/v).

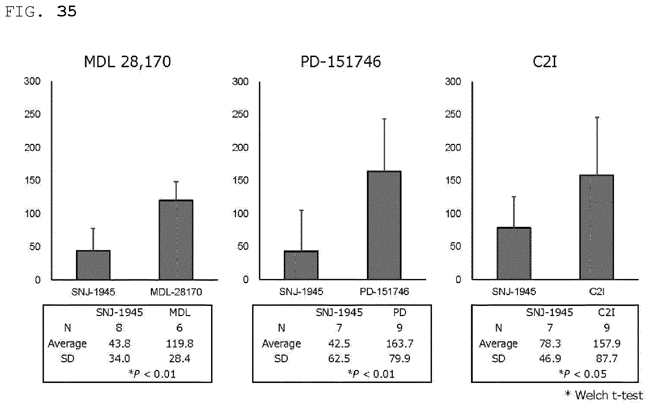

Results, which are obtained by counting the numbers of HMRG-positive nerve cells in the photographed images of the ocular fundi of the rats and comparing the numbers of 2 groups, that is, an SNJ-1945-administered group and a group having administered thereto any other calpain inhibitor, are shown in FIG. **35**. As shown in FIG. **35**, in the SNJ-1945-administered group, the number of the HMRG-positive nerve cells significantly reduced as compared to that in the group having administered thereto any other calpain inhibitor. The results show that the fluorescent probe of the present invention can detect the inhibition abilities of various calpain inhibitors, and that the fluorescent probe of the present invention can detect the calpain inhibition ability of, in particular, SNJ-1945 out of the calpain inhibitors with higher accuracy.

### (Example 19) Detection of Calpain Activity in In Vitro RGC Axon model

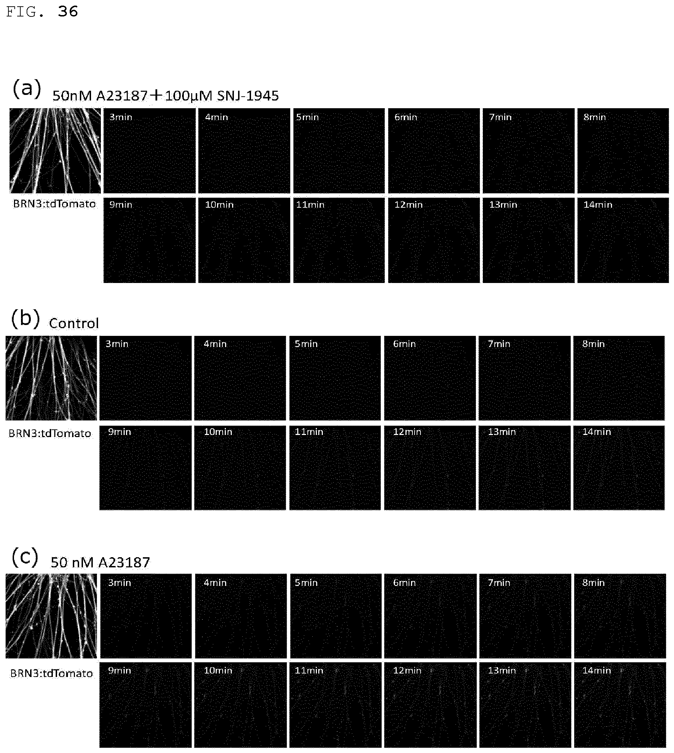

Human iPS cells (201B7 line, purchased from CiRA) were subjected to genome editing, in which the sequence of tdTomato fluorescent protein was inserted downstream the promoter of BRN3B expressed in abundance in retinal ganglion cells, with reference to a previously reported method (STEM CELLS TRANSL MED (2017) 6: 1972-1986). Next, a retinal organoid was induced to differentiate from the iPS cells by a previously reported method (METHODS CELL BIOL (2020) 159: 279-302) with minor modifications. Under the conditions of 37°C and 5% CO₂-95% air, the organoid was subjected to suspension culture for 41 days, and was then subjected to adherent culture for 7 days to induce the elongation of retinal ganglion cell-derived axons. Subsequently, in an evaluation system in which an intracellular calcium concentration was increased by incubating calcium ionophore A23187 at 50 nM, 1 µM of Compound 19 was added simultaneously with the A23187, and cells each having produced therein HMRG were subjected to fluorescence imaging with time through use of a confocal microscope LSM 710. Further, to prove that the production of HMRG resulted from calpain activation, whether or not HMRG was produced from Compound 19 was also confirmed in a retinal organoid in which SNJ-1945 had been incubated at 100 µM 1 hour before the addition of A23187. The results are shown in FIG. **36(a)**. In FIGS. **36**, FIG. **36(b)** is a series of photographed images of the axons to each of which only Compound 19 is added, and none of SNJ-1945 and A23187 is added, and FIG. **36(c)** is a series of photographed images of the axons to each of which only Compound 19 and A23187 are added, and SNJ-1945 is not added. In addition, images at the left ends of the upper rows of FIG. **36(a)** to FIG. **36(c)** are fluorescence photographed images for detecting the fluorescence of tdTomato in those axons, and tdTomato expressed in the axons of the retinal ganglion cells can be observed in each of the images.

It is understood from FIG. **36(a)** to FIG. **36(c)** that calpain activity is enhanced (consequently, a fluorescent probe reaction is enhanced) by the calcium ionophore, and that the calpain activity enhancement disappeared owing to the calpain inhibitor. When an intraocular pressure increases in relation to the onset or progress of glaucoma, ischemia occurs, and hence calcium influx into nerve cells occurs. In addition, various calcium influx mechanisms each including a glutamic acid receptor are conceivable, and calpain activity enhancement may occur as a result of such calcium influx. In Example 19, the reactivity of the fluorescent probe in RGC axons in a nonselective calcium influx model based on calcium ionophore stimulation was confirmed, and it is understood that the fluorescent probe according to an embodiment of the present invention is also effective in defecting calpain activity in axons.

### (Example 20) Change in Fluorescence Intensity with Time by Cathepsin B

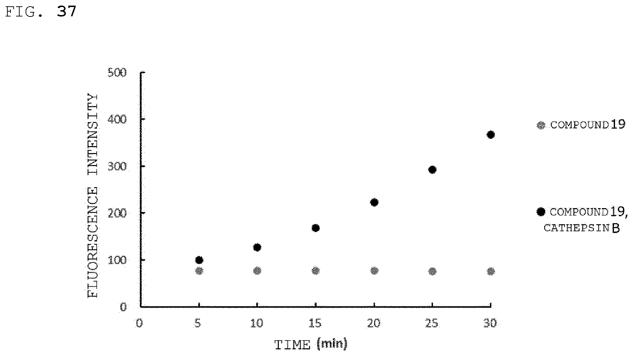

The fluorescence assay of Compound 19 with time was performed. Specifically, 880 ng/mL of recombinant human cathepsin B (953-CY; R&D Systems, MN, USA) was added to a 50 mM TRIS buffer (pH: 7.4, 2 mM EGTA, 2 mM DTE) containing 10 µM of the synthesized fluorescent probe to perform an enzyme reaction at 37°C. The same operations as those described above except that cathepsin B was not added were performed as a negative control. The results of the measurement of a change in fluorescence intensity with time by cathepsin B are shown in FIG. **37** (excitation wavelength: 475 nm, fluorescence wavelength: from 500 nm to 550 nm).

As shown in FIG. **37**, Compound 19 functioned as a fluorescent probe for cathepsin B.

## Claims

1. A compound represented by the following general formula (I) or a salt thereof: 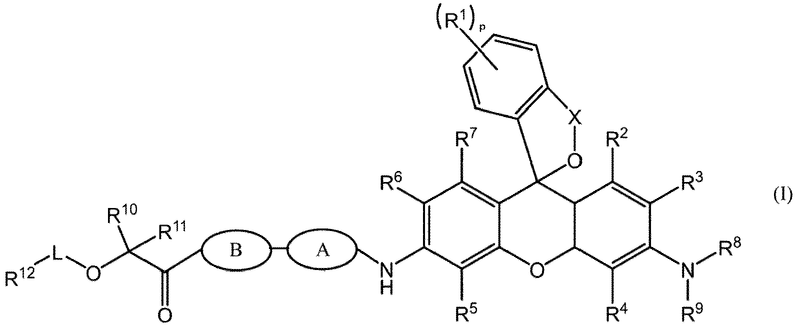 where:
A and B each independently represent an amino acid residue, and the amino acid residues may be identical to or different from each other,
where a bond between A and NH bonded to A is an amide bond between a C-terminal of A and the NH, A and B are bonded through an amide bond, and a bond between B and a carbonyl group (C=O) bonded to B is an amide bond between an N-terminal of B and the carbonyl group (C=O);
R¹s may be identical to or different from each other, and each represent a substituent bonded to a benzene ring;
"p" represents an integer of from 0 to 4;
R², R³, R⁴, R⁵, R⁶, and R⁷ each independently represent a hydrogen atom, a hydroxyl group (OH group), a halogen atom, or a linear, branched, or cyclic alkyl group that may be substituted;
R⁸ and R⁹ each independently represent a hydrogen atom, or a linear, branched, or cyclic alkyl group that may be substituted, or R⁸ and R⁹ may be bonded to form a ring, or R³ and R⁸ may be bonded to form a ring, and/or R⁴ and R⁹ may be bonded to form a ring;
X represents a linear or branched C1 to C3 alkylene group;
R¹⁰ and R¹¹ each independently represent a hydrogen atom, a hydroxyl group (OH), an alkoxy group, or a linear, branched, or cyclic alkyl group that may be substituted, or R¹⁰ and R¹¹ may be bonded to form a ring;
R¹² represents a hydrogen atom, a linear, branched, or cyclic alkyl group that may be substituted, a linear or branched alkenyl group that may be substituted, a linear or branched alkynyl group that may be substituted, an aryl group that may be substituted, or a heterocyclic group that may be substituted, or R¹² may be bonded to R¹⁰ or R¹¹ to form a ring; and
L represents an amide bond, an ester bond, or -(linear or branched alkylene-O-)ₙ- where "n" represents an integer of from 1 to 10, or L represents a single bond.

2. The compound or the salt thereof according to claim 1, wherein in the formula (I), a group represented by -O-L-R¹² is a group represented by the following formula (II): 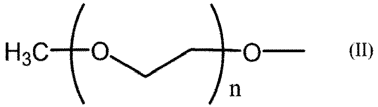 where "n" represents an integer of from 0 to 10.

3. The compound or the salt thereof according to claim 1, wherein in the formula (I), a group represented by -O-L-R¹² is a hydroxyl group or a group selected from the following.

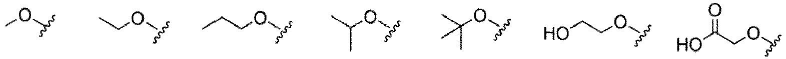

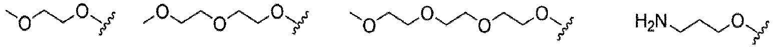

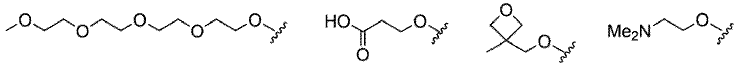

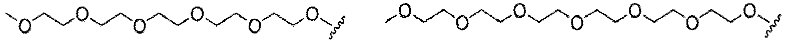

4. The compound or the salt thereof according to claim 1, wherein in the formula (I), a partial structure represented by 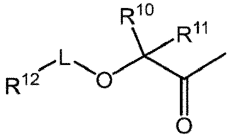 is a structure represented by the following formula: 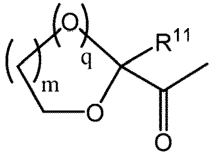 where "m" represents an integer of from 1 to 3, and "q" represents 0 or 1.

5. The compound or the salt thereof according to any one of claims 1 to 4, wherein R¹⁰ and R¹¹ each represent an alkyl group, or are bonded to each other to form a cycloalkyl group.

6. The compound or the salt thereof according to any one of claims 1 to 5, wherein in the formula (I), the amino acid B is Leu.

7. The compound or the salt thereof according to any one of claims 1 to 6, wherein in the formula (I), the amino acid A is Met, Thr, His, Tyr, Phe, Ser, Gln, Arg, Lys, Nle, or MetO.

8. The compound or the salt thereof according to claim 1, wherein the compound is represented by the following general formula (III).

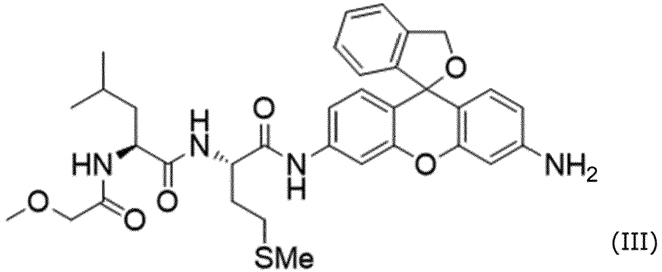

9. A fluorescent probe, comprising the compound or the salt thereof of any one of claims 1 to 8.

10. The fluorescent probe according to claim 9, wherein the fluorescent probe is used for detecting calpain activity.

11. A method of measuring calpain activity in a sample, comprising:
bringing the compound or the salt thereof of any one of claims 1 to 8 and the sample into contact with each other; and
measuring fluorescence from the compound of any one of claims 1 to 8 after the contact.

12. A diagnostic composition for a calpain activity-associated disease, comprising the compound or the salt thereof of any one of claims 1 to 8.

13. The diagnostic composition according to claim 12, wherein the calpain activity-associated disease is an eye disease, a muscle disease, diabetes, an inflammatory disease, an autoimmune disease, a neurological disease, a heart or blood vessel disease, a cancer, a brain tumor, an aging syndrome, progeria, an infectious disease, traumatic encephalopathy, Machado-Joseph disease, preeclampsia, or pulmonary fibrosis.

14. The diagnostic composition according to claim 13, wherein the eye disease is glaucoma, autosomal dominant neovascular inflammatory vitreoretinopathy, retinitis pigmentosa, age-related macular degeneration, retinal neuropathy or a retinal vascular occlusive disease associated with diabetes, retinal ischemia, or cataract.

15. The diagnostic composition according to claim 14, wherein the glaucoma is normal-tension glaucoma.

16. The diagnostic composition according to claim 13, wherein the cancer is a melanoma, breast cancer, colon cancer, kidney cancer, stomach cancer, cervical cancer, ovarian cancer, or soft tissue sarcoma.

17. A method of providing information for diagnosis of a calpain activity-associated disease in a mammal subject, the method comprising:
bringing the compound or the salt thereof of any one of claims 1 to 8 and a sample derived from the mammal subject into contact with each other; and
measuring fluorescence from the compound or the salt thereof of any one of claims 1 to 8 after the contact.

18. A test method for determination of a therapeutic effect of a candidate compound on a calpain activity-associated disease, the method comprising:
preparing a sample from a calpain activity-associated disease model to which the candidate compound is not administered and a sample from the calpain activity-associated disease model to which the candidate compound is administered;
bringing the compound or the salt thereof of any one of claims 1 to 8 and each of the samples into contact with each other; and
measuring fluorescence from the compound or the salt thereof of any one of claims 1 to 8 after the contact.

19. A method of providing information for monitoring of a calpain activity-associated disease in a mammal subject, the method comprising:
bringing the compound or the salt thereof of any one of claims 1 to 8 and a sample derived from the mammal subject into contact with each other; and
measuring fluorescence from the compound or the salt thereof of any one of claims 1 to 8 after the contact.

20. The fluorescent probe according to claim 9, wherein the fluorescent probe is used in combination with a pharmaceutical composition for treating or preventing a calpain activity-associated disease, the pharmaceutical composition including a calpain inhibitor as an active ingredient, for determining a degree of an effect of the pharmaceutical composition in treatment or prevention of the disease with the pharmaceutical composition.

21. The fluorescent probe according to claim 9, wherein the fluorescent probe is used for examining in advance whether or not a treatment subject candidate or a prevention subject candidate corresponds to a subject to be treated or prevented with a pharmaceutical composition for treating or preventing a calpain activity-associated disease, the pharmaceutical composition including a calpain inhibitor as an active ingredient.

22. The fluorescent probe according to claim 9, wherein the fluorescent probe is used for detecting cathepsin activity.

23. The fluorescent probe according to claim 9, wherein
the fluorescent probe is used for detecting pancreatic juice.

24. A kit for pancreatic juice detection, comprising the compound or the salt thereof of any one of claims 1 to 8.

25. A pharmaceutical composition for treating or preventing a calpain activity-associated disease, comprising a calpain inhibitor as an active ingredient, wherein the pharmaceutical composition is administered to a treatment or prevention subject determined to have sensitivity to the calpain inhibitor through use of a fluorescence response caused by an interaction between a calpain in a treatment or prevention subject candidate, or a sample derived from the candidate and the compound or the salt thereof of any one of claims 1 to 8 as an indicator.
